(19) Europäisches Patentamt
European Patent Office
Office européen des brevets

(11) EP 2 985 346 A1

(12) EUROPEAN PATENT APPLICATION
published in accordance with Art. 153(4) EPC

(43) Date of publication:
**17.02.2016 Bulletin 2016/07**

(21) Application number: **14783107.7**

(22) Date of filing: **08.04.2014**

(51) Int Cl.:
***C12N 15/09*** *(2006.01)* ***C12Q 1/68*** *(2006.01)*
***G01N 33/15*** *(2006.01)* ***G01N 33/50*** *(2006.01)*
***G01N 33/53*** *(2006.01)* ***G01N 37/00*** *(2006.01)*

(86) International application number:
**PCT/JP2014/060223**

(87) International publication number:
**WO 2014/168154 (16.10.2014 Gazette 2014/42)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **08.04.2013 JP 2013080395**

(71) Applicant: **Mitsubishi Rayon Co., Ltd.**
**Tokyo 100-8253 (JP)**

(72) Inventors:
- **IKUTA Kenjiro**
**Yokohama-shi**
**Kanagawa 230-0053 (JP)**
- **ISHIDA Susumu**
**Sapporo-shi**
**Hokkaido 060-0808 (JP)**
- **KANDA Atsuhiro**
**Sapporo-shi**
**Hokkaido 060-0808 (JP)**

(74) Representative: **Hoffmann Eitle**
**Patent- und Rechtsanwälte PartmbB**
**Arabellastraße 30**
**81925 München (DE)**

# (54) MICROARRAY FOR EVALUATING EYE DISEASE, AND EVALUATION METHOD OF EYE DISEASE

(57) The purpose of the present invention is to provide a method for objectively evaluating the state of eye disease in a test organism. Provided is a microarray for evaluating the state of eye disease. Further, this method for evaluating the state of eye disease in a test organism is characterized by detecting, from a sample taken from the test organism, at least one gene from a prescribed gene group and comparing the obtained detection result with a control.

EP 2 985 346 A1

SEQUENCE LISTING

<110> MITSUBISHI RAYON CO.,LTD.

<120> A method and a microarray for evaluation of diseases of the eye

<130> G1075WO

<150> JP2013-080395
<151> 2013-04-08

<160> 255

<170> PatentIn version 3.5

<210> 1
<211> 65
<212> DNA
<213> Artificial

<220>
<223> Synthetic DNA

<400> 1
gtagctggag aagggtgtgt ctacctccag acatccttga aatatagtgt tctcccgagg 60

gagaa 65

<210> 2
<211> 65
<212> DNA
<213> Artificial

<220>
<223> Synthetic DNA

<400> 2
tccagaaccc ctgactggtg gctttggcct tagcgatact ttcattctga gtggatctgc 60

ttttg 65

<210> 3
<211> 65
<212> DNA
<213> Artificial

<220>
<223> Synthetic DNA

<400> 3
tgaattactt caagccactg acagaatggc tcgtcaccga gaacaggaga catggagaga 60

cactg 65

<210> 4
<211> 65
<212> DNA
<213> Artificial

<220>

<223> Synthetic DNA

<400> 4
ctctatggta cgaggtgttt agtataccca agcagatagg tgtcgatcga acaggagcag 60

ggaga 65

<210> 5
<211> 65
<212> DNA
<213> Artificial

<220>
<223> Synthetic DNA

<400> 5
ccccttaccc ccgtccttac tttgtaacct ggctgataac gggccatcca tttttgtagc 60

acact 65

<210> 6
<211> 65
<212> DNA
<213> Artificial

<220>
<223> Synthetic DNA

<400> 6
tttctgggca gagtgaataa cccccagagt gtggtgtgag gccttgtgcc tagccatgga 60

gacaa 65

<210> 7
<211> 65
<212> DNA
<213> Artificial

<220>
<223> Synthetic DNA

<400> 7
gctgaagaga ttaattagag aggtgtccag tggctccgag gagacgttca gctactctga 60

ctttc 65

<210> 8
<211> 65
<212> DNA
<213> Artificial

<220>
<223> Synthetic DNA

<400> 8
cgtctgtact gtctacatca cggttccctt agcttgctcc tggtagtgca ttacaggcaa 60

gcatg 65

<210> 9
<211> 65
<212> DNA
<213> Artificial

<220>
<223> Synthetic DNA

<400> 9
gcctgacctg atagagcagg gtgattctgc tggagctatg ggtcaagata tggagacctg 60

gaagg 65

<210> 10
<211> 65
<212> DNA
<213> Artificial

<220>
<223> Synthetic DNA

<400> 10
gcctgacctg atagagcagg gtgattctgc tggagctatg ggtcaagata tggagacctg 60

gaagg 65

<210> 11
<211> 65
<212> DNA
<213> Artificial

<220>
<223> Synthetic DNA

<400> 11
gcctgacctg atagagcagg gtgattctgc tggagctatg ggtcaagata tggagacctg 60

gaagg 65

<210> 12
<211> 65
<212> DNA
<213> Artificial

<220>
<223> Synthetic DNA

<400> 12
ccaagaggtc attgatctgg gtggtgaggc aattaaaggt agtgagtact ttggaaatgg 60

ccgtg 65

<210> 13
<211> 65
<212> DNA
<213> Artificial

<220>

<223> Synthetic DNA

<400> 13
attccttcac agaagtcatt acactggact gatgacatga ggggccagaa gcaaagccag 60

ccttg 65

<210> 14
<211> 65
<212> DNA
<213> Artificial

<220>
<223> Synthetic DNA

<400> 14
gagttaagag gatccgatag cctatctctg ataattctgt gtggaagccc ccactgcaac 60

actct 65

<210> 15
<211> 65
<212> DNA
<213> Artificial

<220>
<223> Synthetic DNA

<400> 15
ggtgtgccct actcagtctc cagaacagcc ctgtaggtcc agccctctct gcaaccagag 60

aacct 65

<210> 16
<211> 65
<212> DNA
<213> Artificial

<220>
<223> Synthetic DNA

<400> 16
ctgcctgtcc catattatac caggtcacct aagaccttcc tggattgatg ctgacctatg 60

aggta 65

<210> 17
<211> 65
<212> DNA
<213> Artificial

<220>
<223> Synthetic DNA

<400> 17
ggaaatacgc actgagagaa ctgtaaacca cttagtcatg ttactcgcgc tggagaacgc 60

actgg 65

<210> 18
<211> 65
<212> DNA
<213> Artificial

<220>
<223> Synthetic DNA

<400> 18
gccgtttgga ccagatgtca accaatatac aggctctaat gaaggagcat gcagagtcct 60

atccc 65

<210> 19
<211> 65
<212> DNA
<213> Artificial

<220>
<223> Synthetic DNA

<400> 19
ttatcaggag atggtggtag aggggtgtgg atgccgctga gatcaggcag tccagagggc 60

ggaca 65

<210> 20
<211> 65
<212> DNA
<213> Artificial

<220>
<223> Synthetic DNA

<400> 20
ctgtgcagtg cccggcttct attacttcaa cttccaagtg atctccaagt gggacctttg 60

tctgt 65

<210> 21
<211> 65
<212> DNA
<213> Artificial

<220>
<223> Synthetic DNA

<400> 21
ccccttgact gcctgaaacc cagaccagag ccctgtagat gttacagaac gaatgggtca 60

ataaa 65

<210> 22
<211> 65
<212> DNA
<213> Artificial

<220>

<223> Synthetic DNA

<400> 22
cctgcggctc cagaggggcg atgaggtgtg gctatcagtc aatgactaca atggcatggt 60

gggca 65

<210> 23
<211> 65
<212> DNA
<213> Artificial

<220>
<223> Synthetic DNA

<400> 23
agttggcgcg gttgcttcct gtagggtgac cctgccttgg ggaggagtgg gttaggggtt 60

ggata 65

<210> 24
<211> 65
<212> DNA
<213> Artificial

<220>
<223> Synthetic DNA

<400> 24
gcatggaggc agggttgatc actgagccgt gttggttatt cagttactat tgctaacaac 60

atggc 65

<210> 25
<211> 65
<212> DNA
<213> Artificial

<220>
<223> Synthetic DNA

<400> 25
agttcaccag tactttaatg tgggacttat ccagcccggg tcggtcaagg tctactccta 60

ttaca 65

<210> 26
<211> 65
<212> DNA
<213> Artificial

<220>
<223> Synthetic DNA

<400> 26
ctttgagtcc aagatcaccc aagtcctgca tttcagaaag gacaccatgg cctccatagg 60

tcaga 65

<210> 27
<211> 65
<212> DNA
<213> Artificial

<220>
<223> Synthetic DNA

<400> 27
cccaggatgg ggagaagtga tacactggct ttacttcggc acgtatttgg aagcagtgac 60

tttag 65

<210> 28
<211> 65
<212> DNA
<213> Artificial

<220>
<223> Synthetic DNA

<400> 28
aacaagaagg agatgaacat ccaacagctc accacctaca ggaagagtgt catgtccttg 60

gccga 65

<210> 29
<211> 65
<212> DNA
<213> Artificial

<220>
<223> Synthetic DNA

<400> 29
ccaaaacccc atgagctagg cctcaactac cacaaaacag cctatgagct ctgtttccag 60

gattc 65

<210> 30
<211> 65
<212> DNA
<213> Artificial

<220>
<223> Synthetic DNA

<400> 30
ccggtggagg ctgacttcct gtatgcttac tctacagcac ctggttacta ttcctggaga 60

aattc 65

<210> 31
<211> 65
<212> DNA
<213> Artificial

<220>

<223> Synthetic DNA

<400> 31
aagaactgcg tttcctaccg agatcctgtg aatggaacct ggtatattca gtcactttgc 60

cagag 65

<210> 32
<211> 65
<212> DNA
<213> Artificial

<220>
<223> Synthetic DNA

<400> 32
tttgctttgt gaaagtccac ttgagtttag atggtagatg tgccaagcct tgtttgttta 60

ttttt 65

<210> 33
<211> 65
<212> DNA
<213> Artificial

<220>
<223> Synthetic DNA

<400> 33
ggcctaaaat cacatgcctt tcggagccgc agatctctta gcactgaaaa agtcccgtca 60

tccct 65

<210> 34
<211> 65
<212> DNA
<213> Artificial

<220>
<223> Synthetic DNA

<400> 34
atcaggaagt tggtgagctg gtataagacc tcagtggagt gttccaggga tgccatcgtg 60

tttct 65

<210> 35
<211> 65
<212> DNA
<213> Artificial

<220>
<223> Synthetic DNA

<400> 35
cacttctgta ggagtgacca gtgtgacagt gaactagtgt gactcggact gtgatgcctt 60

aatta 65

<210> 36
<211> 65
<212> DNA
<213> Artificial

<220>
<223> Synthetic DNA

<400> 36
tctcctacag ccggaagatt ccacgccaat tcatcgttga ctattttgaa accagcagcc 60

tttgc 65

<210> 37
<211> 65
<212> DNA
<213> Artificial

<220>
<223> Synthetic DNA

<400> 37
agcagtcttt gctccaagcc agctgtggta ttcctgacca aaagaggcag acagatctgt 60

gctaa 65

<210> 38
<211> 65
<212> DNA
<213> Artificial

<220>
<223> Synthetic DNA

<400> 38
cggtcctaag ggataggagc tgtctgtagg aatgtgaaac agtcacgcct aaggaatggt 60

cttta 65

<210> 39
<211> 65
<212> DNA
<213> Artificial

<220>
<223> Synthetic DNA

<400> 39
gctgctttca tgtactaaag ctgaagatcc cccttcgggt gctgaaaagc tacgagagaa 60

tcaac 65

<210> 40
<211> 65
<212> DNA
<213> Artificial

<220>

<223> Synthetic DNA

<400> 40
ttgagtccct cttctgtccg gggctccaac cttctcagtt gccaaaacgc cccagtacct 60

tccaa 65

<210> 41
<211> 65
<212> DNA
<213> Artificial

<220>
<223> Synthetic DNA

<400> 41
ttgattgact aataataatg aggaaaacct gatgtgtaca ttacccgatt gaaagtgtgt 60

attgg 65

<210> 42
<211> 65
<212> DNA
<213> Artificial

<220>
<223> Synthetic DNA

<400> 42
ggcgcatcag aaggggataa agaggactct gttttctcac tagccactca cagatttcta 60

tctca 65

<210> 43
<211> 65
<212> DNA
<213> Artificial

<220>
<223> Synthetic DNA

<400> 43
gtttaataac cttgacagtt ttgcatgtga tgctatcact cattggctac ttgacatagc 60

caacg 65

<210> 44
<211> 65
<212> DNA
<213> Artificial

<220>
<223> Synthetic DNA

<400> 44
gagctggtgt gtgaatgttc cagttgtgag ctgtcagtta ggggcagtga ctgttcactt 60

gctta 65

<210> 45
<211> 65
<212> DNA
<213> Artificial

<220>
<223> Synthetic DNA

<400> 45
ctaccgctat ctaaagggaa cccccatttc tgacccatta gtagtcttga atgtggggct 60

ctgag 65

<210> 46
<211> 65
<212> DNA
<213> Artificial

<220>
<223> Synthetic DNA

<400> 46
tggccgattt aaacccaagt tgcccagttc tgagtagaaa actgagacta tgctgtgtgt 60

ggcgg 65

<210> 47
<211> 65
<212> DNA
<213> Artificial

<220>
<223> Synthetic DNA

<400> 47
gcctgggtta cctatcacaa ctctgtctca gagaacagaa atactttcca ggccagccag 60

agctt 65

<210> 48
<211> 65
<212> DNA
<213> Artificial

<220>
<223> Synthetic DNA

<400> 48
gtcgaatttg aagcaactgt gaattcaccc agggaggact gtggagacca taggtgacag 60

gtcat 65

<210> 49
<211> 65
<212> DNA
<213> Artificial

<220>

<223> Synthetic DNA

<400> 49
cctgcagcga ttcctgtcga ctcccttaca cagtattgat cttctgactg tggaaacaac 60

cttcc 65

<210> 50
<211> 65
<212> DNA
<213> Artificial

<220>
<223> Synthetic DNA

<400> 50
ggtgacagtg ccacctctgg cagctcccag aacactaaaa ccacaaagtg tttaggttgg 60

acatt 65

<210> 51
<211> 65
<212> DNA
<213> Artificial

<220>
<223> Synthetic DNA

<400> 51
aggtgtacat caagaatggg gacaagaaag ccagttgtga gagagatgct acaaaggccc 60

aaggc 65

<210> 52
<211> 65
<212> DNA
<213> Artificial

<220>
<223> Synthetic DNA

<400> 52
ccgagactca acagggaaat gtgggcctcc tccacctatt gacaatggag acatcacctc 60

cttgt 65

<210> 53
<211> 65
<212> DNA
<213> Artificial

<220>
<223> Synthetic DNA

<400> 53
ccttcttatc catggggcac aactgcaata acttctgacc tttgggcgaa agccgagaac 60

tcctg 65

<210> 54
<211> 65
<212> DNA
<213> Artificial

<220>
<223> Synthetic DNA

<400> 54
tcccagcagc ttctgtttac ttttcttaca cccatcaccg gcatatcatt gtactcggca 60

ggggc 65

<210> 55
<211> 65
<212> DNA
<213> Artificial

<220>
<223> Synthetic DNA

<400> 55
aagtccaaga actatgagtt catgtggaat cctcacctgg gctacatcct cacatgccca 60

tccaa 65

<210> 56
<211> 65
<212> DNA
<213> Artificial

<220>
<223> Synthetic DNA

<400> 56
cagactacag gcacttttaa gaacttgacc gaccttttct tctatgcgca gttggccacg 60

acgtg 65

<210> 57
<211> 65
<212> DNA
<213> Artificial

<220>
<223> Synthetic DNA

<400> 57
gggaagacct gaacagttat gacagtgatg accaggaaaa gcagtccaag aagaaacccc 60

gcctc 65

<210> 58
<211> 65
<212> DNA
<213> Artificial

<220>

<223>  Synthetic DNA

<400>  58
ctcctgcaaa cgcgatttgc ccgtatcttg gctgaatatg agtcgatgca gcagaaactc      60

aagca                                                                  65

<210>  59
<211>  65
<212>  DNA
<213>  Artificial

<220>
<223>  Synthetic DNA

<400>  59
ccaaacgtgt agtccttccc atatttattg aggtgaaggt cctgctctgt tacccagtcc      60

agcct                                                                  65

<210>  60
<211>  65
<212>  DNA
<213>  Artificial

<220>
<223>  Synthetic DNA

<400>  60
ctccgtgtca tttcttctca tcacatggga atctcagcac atgagagcca ttatggggcc      60

aagca                                                                  65

<210>  61
<211>  65
<212>  DNA
<213>  Artificial

<220>
<223>  Synthetic DNA

<400>  61
cctgaagatg acgatgactt ctctgagtac tctgtgtatt ctgtggagga ctaccaggag      60

cctga                                                                  65

<210>  62
<211>  65
<212>  DNA
<213>  Artificial

<220>
<223>  Synthetic DNA

<400>  62
ttccccaggt caaagccact aatccaatgg gttctggcgg taagagatta gtcaactccc      60

agagg                                                                  65

<210> 63
<211> 65
<212> DNA
<213> Artificial

<220>
<223> Synthetic DNA

<400> 63
ctagcctatc aggtctccct tccctggcta cccagagtga aactgattaa aaatgtggat 60

cccac 65

<210> 64
<211> 65
<212> DNA
<213> Artificial

<220>
<223> Synthetic DNA

<400> 64
attaacttct tggtcacatt tctgagtagg ctgaagtgcc tgctttctag gtaggggtga 60

gcgcc 65

<210> 65
<211> 65
<212> DNA
<213> Artificial

<220>
<223> Synthetic DNA

<400> 65
tctacaaaag cggtgtctat gacgacccct cctgtacggg caatgtgaat catggtgttc 60

ttgtg 65

<210> 66
<211> 65
<212> DNA
<213> Artificial

<220>
<223> Synthetic DNA

<400> 66
ctgctctgat ggcaccgtct ggtgaacgct ggcttctgac aacactatac aatttctttt 60

gaggg 65

<210> 67
<211> 65
<212> DNA
<213> Artificial

<220>

<223> Synthetic DNA

<400> 67
ggagccagac catcctggat aatgtgagaa catgcctaga tttacccaca aaacacaagt 60

ctgag 65

<210> 68
<211> 65
<212> DNA
<213> Artificial

<220>
<223> Synthetic DNA

<400> 68
ggatttcaat gtaatgttgt gagtaaccct tggacatttt atgtcttcct cgtaaggcac 60

agtgc 65

<210> 69
<211> 65
<212> DNA
<213> Artificial

<220>
<223> Synthetic DNA

<400> 69
tgcgggagga cctagaggaa cttcaggccc agtatcctaa tcgctttaag ctctggttca 60

ctctg 65

<210> 70
<211> 65
<212> DNA
<213> Artificial

<220>
<223> Synthetic DNA

<400> 70
cctccctaca agacaggctt ctcaaatgga tgcccttgca ggcaagtcct agttttgttt 60

tgttt 65

<210> 71
<211> 65
<212> DNA
<213> Artificial

<220>
<223> Synthetic DNA

<400> 71
gccctccacc accgttagcc aatccctgaa cagtcccgtc ttccaaccca gagtcagtga 60

cctcc 65

<210> 72
<211> 65
<212> DNA
<213> Artificial

<220>
<223> Synthetic DNA

<400> 72
aggacacaat tacctaagcc acacctggag actgaacagt catcctaatg actgacatca 60

tggct 65

<210> 73
<211> 65
<212> DNA
<213> Artificial

<220>
<223> Synthetic DNA

<400> 73
gaatccacgt gacacatgac tctctttagg agtcacccac agttcttgtg tgtacagatt 60

gcttt 65

<210> 74
<211> 65
<212> DNA
<213> Artificial

<220>
<223> Synthetic DNA

<400> 74
gtctacaaat acatgagcat ccgatctgac aggtccgtgc cttcagacat cttccagata 60

caggc 65

<210> 75
<211> 65
<212> DNA
<213> Artificial

<220>
<223> Synthetic DNA

<400> 75
ttcttgacac cgcattgaag ccactcacct gtgtgcgtct gggtatgaag tccagctcct 60

tccgt 65

<210> 76
<211> 65
<212> DNA
<213> Artificial

<220>

<223> Synthetic DNA

<400> 76
aaccaggctg atgatggtag agtgctacag acttggtact ccagtttcca cggctaatca 60

ctgct 65

<210> 77
<211> 65
<212> DNA
<213> Artificial

<220>
<223> Synthetic DNA

<400> 77
ccagacccct tcactccctg ctctccgatt tcagcacaat aaatacacta cagctgtggg 60

aaaag 65

<210> 78
<211> 65
<212> DNA
<213> Artificial

<220>
<223> Synthetic DNA

<400> 78
gttccgtaat ccaaaggcca aatgaggagc tggagactcc aggctttcac aggaaagaca 60

caggc 65

<210> 79
<211> 65
<212> DNA
<213> Artificial

<220>
<223> Synthetic DNA

<400> 79
ggggcatggg gcaacagtgg aaacctgctg ataccagtca tggtagtact tgtcattttc 60

actga 65

<210> 80
<211> 65
<212> DNA
<213> Artificial

<220>
<223> Synthetic DNA

<400> 80
agtggtatag gtgcagatca atggtgaagt gcttcttagc atgggacgag ccctagattc 60

catcc 65

<210> 81
<211> 65
<212> DNA
<213> Artificial

<220>
<223> Synthetic DNA

<400> 81
acctatgcat cagctaaatg gacacacagc ggaggggaaa tgttcgttgc cttaaatcaa 60

aaggg 65

<210> 82
<211> 65
<212> DNA
<213> Artificial

<220>
<223> Synthetic DNA

<400> 82
tcaattctgc cacctgctgg ttgctttggt accttggtct cttattcaaa cccacaccac 60

tcaag 65

<210> 83
<211> 65
<212> DNA
<213> Artificial

<220>
<223> Synthetic DNA

<400> 83
ctctttgggg tctagagatg agccctgggt ctctaaaatg gctctcttag aagttgtatg 60

tgcaa 65

<210> 84
<211> 65
<212> DNA
<213> Artificial

<220>
<223> Synthetic DNA

<400> 84
gtgacatgtc catctgcagt attgtgtgtt ttgatgccaa cacggggaca tcttcagttg 60

ttccc 65

<210> 85
<211> 65
<212> DNA
<213> Artificial

<220>

<223> Synthetic DNA

<400> 85
gaaggagaag ggctcactgc atacttatat ccctgaaacg acatctttag agctggcctc 60

atcac 65

<210> 86
<211> 65
<212> DNA
<213> Artificial

<220>
<223> Synthetic DNA

<400> 86
cttcacttca gtgctgattc agcccagagg gttagttagt tccctctgga cggctgctct 60

tgtag 65

<210> 87
<211> 65
<212> DNA
<213> Artificial

<220>
<223> Synthetic DNA

<400> 87
ggattcgccc attcctgtct cttcctaccc aaccactcaa ttaatctttc cttgcctgag 60

accag 65

<210> 88
<211> 65
<212> DNA
<213> Artificial

<220>
<223> Synthetic DNA

<400> 88
cggtgaggtt tctgtgacac ctgacactgc cagcctactt ctctaatgga ttcctgtgta 60

gctca 65

<210> 89
<211> 65
<212> DNA
<213> Artificial

<220>
<223> Synthetic DNA

<400> 89
gggtggctca gagcagagtc caacccctat tgatcaacca tgggcaaaga atatcccatg 60

tgagg 65

<210> 90
<211> 65
<212> DNA
<213> Artificial

<220>
<223> Synthetic DNA

<400> 90
gccagcgacc ctaacgccca ggttccaagg ctggttctgt ttagtataag aacagaaaag 60

cttca 65

<210> 91
<211> 65
<212> DNA
<213> Artificial

<220>
<223> Synthetic DNA

<400> 91
cctcctcagc cacgcccaag tgtactggga tacctaagga ccaatgtttt cttcctctta 60

gacac 65

<210> 92
<211> 65
<212> DNA
<213> Artificial

<220>
<223> Synthetic DNA

<400> 92
ccaagtgacc ctggtaaggg aactgtctgc agaatggaag aaatagccta agagacaggg 60

atggc 65

<210> 93
<211> 65
<212> DNA
<213> Artificial

<220>
<223> Synthetic DNA

<400> 93
caaaacccca ggaaggttgt atgtgtcggg ggacatactt ctgatgaggt gctgagcatt 60

ttgtc 65

<210> 94
<211> 65
<212> DNA
<213> Artificial

<220>

<223> Synthetic DNA

<400> 94
gtgggatggc tctcacctcc agtctatggg aacttcaagc ctttattgaa acacccacca 60

gctga 65

<210> 95
<211> 65
<212> DNA
<213> Artificial

<220>
<223> Synthetic DNA

<400> 95
gagtctctgc attggccagt aactatcctt gtagctatgc cccgtgtttg ccaggcctgg 60

gaatc 65

<210> 96
<211> 65
<212> DNA
<213> Artificial

<220>
<223> Synthetic DNA

<400> 96
tttcccatgg tgcagagcac cggtctacat ttactgccga gtgcgatcca atgctctgga 60

ctgag 65

<210> 97
<211> 65
<212> DNA
<213> Artificial

<220>
<223> Synthetic DNA

<400> 97
ggagtagaga gagtccctgg tgtcttagtt tccacagtaa ggtttgcctg tgtacagacc 60

tctgt 65

<210> 98
<211> 65
<212> DNA
<213> Artificial

<220>
<223> Synthetic DNA

<400> 98
ggtcagtttc acagggttat gccattttat tcaagtccgt tgctccgttg tgcagtctcc 60

accat 65

<210> 99
<211> 65
<212> DNA
<213> Artificial

<220>
<223> Synthetic DNA

<400> 99
tggagcgact catccatgag tgccgaggaa ttcacagata ccgtgtttgc caagatcgac 60

atcaa 65

<210> 100
<211> 65
<212> DNA
<213> Artificial

<220>
<223> Synthetic DNA

<400> 100
tgcttcattc cacagagtga tgtctgtggt ctaaatttgc ctggaggtgg gggaaacttt 60

gccag 65

<210> 101
<211> 65
<212> DNA
<213> Artificial

<220>
<223> Synthetic DNA

<400> 101
tcactggagc tgtggtggct tttgtgatga agatgagaag gagaaacaca ggtggaaaag 60

gaggg 65

<210> 102
<211> 65
<212> DNA
<213> Artificial

<220>
<223> Synthetic DNA

<400> 102
caggacccac tcaactatcc tccatctgtt atagagtgac tcctctgtca ccatgccctg 60

acttc 65

<210> 103
<211> 65
<212> DNA
<213> Artificial

<220>

<223> Synthetic DNA

<400> 103
atctgttccc attagcaggt gaaggaagct agggctgaaa caagagtttt ccgcgctctc 60

aggga 65

<210> 104
<211> 65
<212> DNA
<213> Artificial

<220>
<223> Synthetic DNA

<400> 104
gtgcctgttg tgtttggagg tcaggagttg ctgtgtatga cagtttcggc tacgcttgac 60

taaca 65

<210> 105
<211> 65
<212> DNA
<213> Artificial

<220>
<223> Synthetic DNA

<400> 105
tatcagtgtt ccagtagcct gggaagacat atagtctagc tgcccagttc cctggagatg 60

gtcat 65

<210> 106
<211> 65
<212> DNA
<213> Artificial

<220>
<223> Synthetic DNA

<400> 106
cgtatgtaca tggagatttg cttgaaagta gaaccctgat gctcgcacac ctgacctgtg 60

gaagc 65

<210> 107
<211> 65
<212> DNA
<213> Artificial

<220>
<223> Synthetic DNA

<400> 107
ctacttttgt tcccaatgtc agccaccatg ccttagcagc tgaacaatcg agcctcatgc 60

tcatg 65

<210> 108
<211> 65
<212> DNA
<213> Artificial

<220>
<223> Synthetic DNA

<400> 108
ttgctgcttt aggtgtctct tttcctccct ctctatctct actctccaac atgaaggcgc 60

tgagc 65

<210> 109
<211> 65
<212> DNA
<213> Artificial

<220>
<223> Synthetic DNA

<400> 109
ccaggaagat gccctgctgg ctgtgaggaa atacttccac aggatcactg tgtacctgag 60

agaga 65

<210> 110
<211> 65
<212> DNA
<213> Artificial

<220>
<223> Synthetic DNA

<400> 110
agctggctgc actccactag aattgccacg acgtacctat cacagtgacg tctacagtga 60

gaaat 65

<210> 111
<211> 65
<212> DNA
<213> Artificial

<220>
<223> Synthetic DNA

<400> 111
ggctccatcc atatctccta tcggtgtctc tgtatcctta aaccttgcaa acatcataca 60

gtgta 65

<210> 112
<211> 65
<212> DNA
<213> Artificial

<220>

<223> Synthetic DNA

<400> 112
tttctaagcc agtgaggttg aggtgagagg tttgccagag tttgtctacc tctgggtatc 60

ccttt 65

<210> 113
<211> 65
<212> DNA
<213> Artificial

<220>
<223> Synthetic DNA

<400> 113
cagcatcgga ctccacgttc agagatgtcg caaataatgg tgcgcttagt tcttcagatg 60

acttc 65

<210> 114
<211> 65
<212> DNA
<213> Artificial

<220>
<223> Synthetic DNA

<400> 114
ctgatccagg gatcttagct aacggaaaca actccttgga aaacctcgtt tgtacctctc 60

tccga 65

<210> 115
<211> 65
<212> DNA
<213> Artificial

<220>
<223> Synthetic DNA

<400> 115
ttcctccaga atgtgaaggt caacctcaaa gtctttaact cccttggcgc aaaagtgagc 60

tccag 65

<210> 116
<211> 65
<212> DNA
<213> Artificial

<220>
<223> Synthetic DNA

<400> 116
ggaacatcct taaatcctct gagcttgaca ggcatcctca cagcaggatt ttctaggtgg 60

tcagt 65

<210> 117
<211> 65
<212> DNA
<213> Artificial

<220>
<223> Synthetic DNA

<400> 117
cattaggcag cactctctag aacagaacct agctgtcaac gtgtggggga tgaattggtc 60

atagc 65

<210> 118
<211> 65
<212> DNA
<213> Artificial

<220>
<223> Synthetic DNA

<400> 118
atctactcgg caaacctagt gcgttatgcc taagcatatc agtttgtgga cattcctcac 60

tgtgg 65

<210> 119
<211> 65
<212> DNA
<213> Artificial

<220>
<223> Synthetic DNA

<400> 119
gtagatacca agtccccagg agctctaagt ttcccaatgc tcccattcac tctccagctg 60

tcttc 65

<210> 120
<211> 65
<212> DNA
<213> Artificial

<220>
<223> Synthetic DNA

<400> 120
ggtgctatcg acggtgctac ttcatctgtg tacaaaagac cgacgcatgg tctatgttgc 60

tacca 65

<210> 121
<211> 65
<212> DNA
<213> Artificial

<220>

<223>  Synthetic DNA

<400>  121
ttgactactt ggtcctcagt ggaaccactt gggatgacta gaggtgtggg gttgttggtt    60

gagtt    65

<210>  122
<211>  65
<212>  DNA
<213>  Artificial

<220>
<223>  Synthetic DNA

<400>  122
ttgccgcctt acgagaacag tgtgagtttc tgtagtgagt ctaaccccga ggagggaatt    60

tagtg    65

<210>  123
<211>  65
<212>  DNA
<213>  Artificial

<220>
<223>  Synthetic DNA

<400>  123
aaagccccat cttttggtat gccccggagt ccctatctga caacatcttc tcccgccaat    60

ctgac    65

<210>  124
<211>  65
<212>  DNA
<213>  Artificial

<220>
<223>  Synthetic DNA

<400>  124
tggtctcact accagttaaa gcaaaagact ttcaaacagt ggctctgtcc tccaagaagt    60

ggcaa    65

<210>  125
<211>  65
<212>  DNA
<213>  Artificial

<220>
<223>  Synthetic DNA

<400>  125
gccttttaaa ctttgtgtgt tgtgtgtctg tgcacatggg tacaggtgcc tgctcacttg    60

agagg    65

<210> 126
<211> 65
<212> DNA
<213> Artificial

<220>
<223> Synthetic DNA

<400> 126
tgacctccag cttcacccga gccaggagaa agtctttgtg aactgtgaag tgaagtcaaa 60

aagac 65

<210> 127
<211> 65
<212> DNA
<213> Artificial

<220>
<223> Synthetic DNA

<400> 127
actatgagga ggggcatctc aatggcacct ttgaatccca ggttcagtaa cgcccaccat 60

ctggc 65

<210> 128
<211> 65
<212> DNA
<213> Artificial

<220>
<223> Synthetic DNA

<400> 128
gtgggaaagg tctaccttcc caaagagaga agacacatga attcttggca gaggggatag 60

catgg 65

<210> 129
<211> 65
<212> DNA
<213> Artificial

<220>
<223> Synthetic DNA

<400> 129
tatttctctg ctgtttcagt tcttgagaaa ttggggaagt cctacagagg ctgcccctgc 60

cctca 65

<210> 130
<211> 65
<212> DNA
<213> Artificial

<220>

<223> Synthetic DNA

<400> 130
tacgagacac tgcagatggc gctcagctac atcatcgcgc tcacccgcat cctagccgaa 60

gccga 65

<210> 131
<211> 65
<212> DNA
<213> Artificial

<220>
<223> Synthetic DNA

<400> 131
ccgcctctgc catctgctac ggtgtcgtca gttgtatggc attaatttcc tgccactaga 60

gatat 65

<210> 132
<211> 65
<212> DNA
<213> Artificial

<220>
<223> Synthetic DNA

<400> 132
gcttctccaa gttgttccaa gaaagggtgc cctgtacctt tccactggca ggacattacc 60

attgt 65

<210> 133
<211> 65
<212> DNA
<213> Artificial

<220>
<223> Synthetic DNA

<400> 133
cctagttggc tgcctcccgc cactctgact aaaaggaatc ttaagagtgt acatttggag 60

gtgga 65

<210> 134
<211> 65
<212> DNA
<213> Artificial

<220>
<223> Synthetic DNA

<400> 134
cctcctctgt agttaaccag ccttctcctt cacctggtga cttcagattt aagagggtgg 60

cttct 65

<210>  135
<211>  65
<212>  DNA
<213>  Artificial

<220>
<223>  Synthetic DNA

<400>  135
actggagggc tgtatctata aatctatttg ccaataagtt cccaggcaga ggcaggtagg 60

agggg 65

<210>  136
<211>  65
<212>  DNA
<213>  Artificial

<220>
<223>  Synthetic DNA

<400>  136
gggcgcggct ccaaccgctg cataaatatt aaggtattca gttgccccta ctggaaggta 60

ttatg 65

<210>  137
<211>  65
<212>  DNA
<213>  Artificial

<220>
<223>  Synthetic DNA

<400>  137
agccaatgat tttgggagtc tgtagtatga atggactagt tattctgtga aaacatctag 60

agaaa 65

<210>  138
<211>  65
<212>  DNA
<213>  Artificial

<220>
<223>  Synthetic DNA

<400>  138
tgggccagca ctcttagctt tgataacttg acctgtggta tctctcgtgg agaggtgtgg 60

ctggc 65

<210>  139
<211>  65
<212>  DNA
<213>  Artificial

<220>

<223> Synthetic DNA

<400> 139
ccgaatttgg cgtccttctt ggttctgaaa tgaaatgtag ttgccacgca cagacggtgt 60

ctagc 65

<210> 140
<211> 65
<212> DNA
<213> Artificial

<220>
<223> Synthetic DNA

<400> 140
atcctgctgc cctcttcata tgctttgatg gactgtagac tccctttaac tctctctctg 60

gagta 65

<210> 141
<211> 65
<212> DNA
<213> Artificial

<220>
<223> Synthetic DNA

<400> 141
ggacccaaca tttaaggggg aaatgtgggt gagaggaaag tggagtactc tcagctaaca 60

cacag 65

<210> 142
<211> 65
<212> DNA
<213> Artificial

<220>
<223> Synthetic DNA

<400> 142
ggggcagcac atcttagaag ctaaatagtt ccctgccttt ctcagccagt aattccacat 60

tcagg 65

<210> 143
<211> 65
<212> DNA
<213> Artificial

<220>
<223> Synthetic DNA

<400> 143
gagttgggag tagccacctc atcagctact gtcatctgtc ctctaagggg cataataaat 60

gggag 65

<210> 144
<211> 65
<212> DNA
<213> Artificial

<220>
<223> Synthetic DNA

<400> 144
gactcaaatc ccacacaacc actgtaaggc atactcaggt caagacatga gaagaccagc 60

aggac 65

<210> 145
<211> 65
<212> DNA
<213> Artificial

<220>
<223> Synthetic DNA

<400> 145
gtgaccaaat ggaaagggtt ggtgtggaag caagtgagac ttcctaactg tacagcgtgg 60

gatgt 65

<210> 146
<211> 65
<212> DNA
<213> Artificial

<220>
<223> Synthetic DNA

<400> 146
gcctccaacc tgtcccatcc atcaaagctt tggccatgtt ttacctccct tctcatctat 60

ccttg 65

<210> 147
<211> 65
<212> DNA
<213> Artificial

<220>
<223> Synthetic DNA

<400> 147
aaggagcccc tcgaaacgaa gaagtgcgtg ttgagttata cgcaaggagt gctgggtact 60

gatat 65

<210> 148
<211> 65
<212> DNA
<213> Artificial

<220>

<223> Synthetic DNA

<400> 148
ctaacccta cagtggagca caggactgga tttattctgg cagctactgg gacagaaact 60

acttc 65

<210> 149
<211> 65
<212> DNA
<213> Artificial

<220>
<223> Synthetic DNA

<400> 149
acacaggctg ttggatcgcg gatctgtgtt gctcatgtgg ttgtttaaag gaaaccatga 60

tcgac 65

<210> 150
<211> 65
<212> DNA
<213> Artificial

<220>
<223> Synthetic DNA

<400> 150
ccttcagggg tttcctcagc tgcaggatat tctgcatttt tcagggggat ctatgcctga 60

gatgg 65

<210> 151
<211> 65
<212> DNA
<213> Artificial

<220>
<223> Synthetic DNA

<400> 151
aagaagaagg aagaagacag agtcgcagcc aagaaagtgg gcacagaagt ttgcaatggt 60

ggtcc 65

<210> 152
<211> 65
<212> DNA
<213> Artificial

<220>
<223> Synthetic DNA

<400> 152
aacactgctg tccacatgac ctccatttcc caaagtcctc tgctccagca actgcccttc 60

caggt 65

<210> 153
<211> 65
<212> DNA
<213> Artificial

<220>
<223> Synthetic DNA

<400> 153
gttgctggga cagagctgta gtgtcttggt cgtctgtgtt ggtacccaat acagtgtaga 60

catct 65

<210> 154
<211> 65
<212> DNA
<213> Artificial

<220>
<223> Synthetic DNA

<400> 154
tttattcccc actaaagaaa cggtttccta aggtctgagc tgtttcccag ggtgggctag 60

agtgg 65

<210> 155
<211> 65
<212> DNA
<213> Artificial

<220>
<223> Synthetic DNA

<400> 155
taccggagcc aaagtggaga ggatgaatcc cttaaccagc ctggaccaat caaaaccact 60

tttgc 65

<210> 156
<211> 65
<212> DNA
<213> Artificial

<220>
<223> Synthetic DNA

<400> 156
caccggttgt ctctgccggg actaactgcc aagccagatt ctcttgaata aagcattcta 60

gtctg 65

<210> 157
<211> 65
<212> DNA
<213> Artificial

<220>

<223> Synthetic DNA

<400> 157
catgctggtt aagatgcagc ctagccattg cctgagctgt tcatacaatg atccggaagt 60

gctag 65

<210> 158
<211> 65
<212> DNA
<213> Artificial

<220>
<223> Synthetic DNA

<400> 158
gcctggaaat gttctttcac tgacttgtgg cagcctggga agtcatctgt gagcttctat 60

gactt 65

<210> 159
<211> 65
<212> DNA
<213> Artificial

<220>
<223> Synthetic DNA

<400> 159
aacttcaccc ttgtttagcc cctattaagg tcgctttgga tgtggggaaa ggcccaacgg 60

tagaa 65

<210> 160
<211> 65
<212> DNA
<213> Artificial

<220>
<223> Synthetic DNA

<400> 160
gatgaagagg gctgagcgta ggtaatgcgg gctctcccca catcctttct gaatgggcac 60

ttcta 65

<210> 161
<211> 65
<212> DNA
<213> Artificial

<220>
<223> Synthetic DNA

<400> 161
cctctactct ccgtttgcat gatccgctct gttagatgca ttcattgtag ttcggaagca 60

agcgt 65

<210> 162
<211> 65
<212> DNA
<213> Artificial

<220>
<223> Synthetic DNA

<400> 162
gcgtgcactc agttccgtag gattccaaag cagaccccta gctggtcttt gaatctgcat 60

gtact 65

<210> 163
<211> 65
<212> DNA
<213> Artificial

<220>
<223> Synthetic DNA

<400> 163
aacaagggat cttgagagaa ggaactgtcg ctcagctggg agcggaatca ttatcgcaat 60

cacag 65

<210> 164
<211> 65
<212> DNA
<213> Artificial

<220>
<223> Synthetic DNA

<400> 164
cctcaatctc acctctacct tcctcaggaa aaaccagtgt gagaccaaga tcatggtact 60

gcagc 65

<210> 165
<211> 65
<212> DNA
<213> Artificial

<220>
<223> Synthetic DNA

<400> 165
ggcaagagag ttctggagac tgtgggacag ctaggatgaa gaactctatg ttataagcct 60

ctgtc 65

<210> 166
<211> 65
<212> DNA
<213> Artificial

<220>

<223> Synthetic DNA

<400> 166
tacaaagtcc acatcaaccc caactcgctc tttgacgtcc aggtgaaaag gattcatgag 60

tacaa 65

<210> 167
<211> 65
<212> DNA
<213> Artificial

<220>
<223> Synthetic DNA

<400> 167
gaggaggaat tcatcgaggg gaccctggcc aataaggaaa ttctgcgact gatccagttt 60

gaacc 65

<210> 168
<211> 65
<212> DNA
<213> Artificial

<220>
<223> Synthetic DNA

<400> 168
ttgtgtgtac ctgtatggta catgtgtggg agagccttca gagatgggaa ggtgtggaga 60

gccgt 65

<210> 169
<211> 65
<212> DNA
<213> Artificial

<220>
<223> Synthetic DNA

<400> 169
gcaagttcta tacagagttt gatcggcata acaatcgcat tggattcgcc ttggcccgct 60

aaggc 65

<210> 170
<211> 65
<212> DNA
<213> Artificial

<220>
<223> Synthetic DNA

<400> 170
cctgcccttg aggggattat atgagattta agggacttat gtggccagcc tacttcctgg 60

catgc 65

<210> 171
<211> 65
<212> DNA
<213> Artificial

<220>
<223> Synthetic DNA

<400> 171
cgcagggaaa cagggaacca atgcgctatt ctcattctac cgccactctg agcttaagga 60

actta 65

<210> 172
<211> 65
<212> DNA
<213> Artificial

<220>
<223> Synthetic DNA

<400> 172
aattcgttac tcagactcag ggaaggaaag gtccctgggg ttataggagt taagagcaag 60

cggag 65

<210> 173
<211> 65
<212> DNA
<213> Artificial

<220>
<223> Synthetic DNA

<400> 173
agggcaaaag cctgcatatc tcctggttct gaatgccaaa gacttgagtg aaattgccag 60

ggctg 65

<210> 174
<211> 65
<212> DNA
<213> Artificial

<220>
<223> Synthetic DNA

<400> 174
gggctttgac accctcctcc tcgctgcccg aaagacagta ttctggctac ctttggatca 60

agata 65

<210> 175
<211> 65
<212> DNA
<213> Artificial

<220>

<223> Synthetic DNA

<400> 175
ggtggagttt tgtacggctg ttaaaggtgg cccttctttg ctatttaagg ggctgaggtc 60

tttcc 65

<210> 176
<211> 65
<212> DNA
<213> Artificial

<220>
<223> Synthetic DNA

<400> 176
ggctgatgga tgatctggac cgtaacaagg atcaggaagt aaacttccag gagtatgtcg 60

ccttc 65

<210> 177
<211> 65
<212> DNA
<213> Artificial

<220>
<223> Synthetic DNA

<400> 177
gccttgaggg aagcccccgg tagactctca aagttatgct agatatcaaa gcatgagcat 60

tcctc 65

<210> 178
<211> 65
<212> DNA
<213> Artificial

<220>
<223> Synthetic DNA

<400> 178
caacccaaga aactcctggg ctaagtatct gacagtctca catctcaaca gtgtgaatta 60

agtgt 65

<210> 179
<211> 65
<212> DNA
<213> Artificial

<220>
<223> Synthetic DNA

<400> 179
ggcccttcca aagctgcaca atgcccctca attacggagg ctgagaatgt agtgggtata 60

cctct 65

<210> 180
<211> 65
<212> DNA
<213> Artificial

<220>
<223> Synthetic DNA

<400> 180
atgaccgctt cctttacttc agcaactggc tgcatgggga cattcggcag tatgacatct 60

ctaac 65

<210> 181
<211> 65
<212> DNA
<213> Artificial

<220>
<223> Synthetic DNA

<400> 181
atgaccgctt cctttacttc agcaactggc tgcatgggga cattcggcag tatgacatct 60

ctaac 65

<210> 182
<211> 65
<212> DNA
<213> Artificial

<220>
<223> Synthetic DNA

<400> 182
ttatagccaa gatagccaac cccaaatgag actagaactc cccaagtgtt gacgcttctt 60

cccgg 65

<210> 183
<211> 65
<212> DNA
<213> Artificial

<220>
<223> Synthetic DNA

<400> 183
gaatcctgga ccccagtagt acttaatgtc tcagtgctct acagaacccc cagagggaag 60

ctgat 65

<210> 184
<211> 65
<212> DNA
<213> Artificial

<220>

<223> Synthetic DNA

<400> 184
ttcctcttcc tgctctggga ccagcaacac aggttcccag tcttcatggg tcgtgtatat 60

gaccc 65

<210> 185
<211> 65
<212> DNA
<213> Artificial

<220>
<223> Synthetic DNA

<400> 185
tcacggccgt ctaccgctgg gacaagaaga ataaggagat gaagtttgca gtcaaattca 60

tgttc 65

<210> 186
<211> 65
<212> DNA
<213> Artificial

<220>
<223> Synthetic DNA

<400> 186
acctgctggg gcatgagtgt cacctgggca gagcttgcta ggtcattaaa cagagacatg 60

atgac 65

<210> 187
<211> 65
<212> DNA
<213> Artificial

<220>
<223> Synthetic DNA

<400> 187
ccttgtccgt cactcctttg aatgctgatt ttgtctcaag tcctttcagt tcacatagcc 60

gtggc 65

<210> 188
<211> 65
<212> DNA
<213> Artificial

<220>
<223> Synthetic DNA

<400> 188
ccactggctg aaagatggaa acgcaaacag tctgaccttc tgaggactac aatcaagtaa 60

gagcc 65

<210> 189
<211> 65
<212> DNA
<213> Artificial

<220>
<223> Synthetic DNA

<400> 189
cacttcctct tcagtcccta atcagccccc aaagaaacga cgtcataaca ccacttcctc 60

ctcat 65

<210> 190
<211> 65
<212> DNA
<213> Artificial

<220>
<223> Synthetic DNA

<400> 190
aggagactcc tgagttaaca ctgggaagac attggccagt cctagtcatc tctcggtcag 60

taggt 65

<210> 191
<211> 65
<212> DNA
<213> Artificial

<220>
<223> Synthetic DNA

<400> 191
ctgctcagac tatcacctgt acctccctga ataccagcgt ttaaccttca agacatccca 60

tgtgc 65

<210> 192
<211> 65
<212> DNA
<213> Artificial

<220>
<223> Synthetic DNA

<400> 192
cacctctccc caccccctgc cacttgaaac cttctactaa tcaagagaaa cttccaagcc 60

aacgg 65

<210> 193
<211> 65
<212> DNA
<213> Artificial

<220>

<223>  Synthetic DNA

<400>  193
caacaacgga aagggcagcc atgagtcaag tcagctggat gaaccaagtc tggaaacaca  60

cagac  65

<210>  194
<211>  65
<212>  DNA
<213>  Artificial

<220>
<223>  Synthetic DNA

<400>  194
tcaccgtttt gagggatgat gttttgtggc acgtgtgtga tcacagcctg atggttctgg  60

tcgtg  65

<210>  195
<211>  65
<212>  DNA
<213>  Artificial

<220>
<223>  Synthetic DNA

<400>  195
gaacttataa actgaaaggg tatttaggaa ggcaaggctt gggcattttt atggctttca  60

atcct  65

<210>  196
<211>  65
<212>  DNA
<213>  Artificial

<220>
<223>  Synthetic DNA

<400>  196
aggtttaggc aactaagttg gagttttact cctaagctag aagcttcgcc cagaccggtg  60

tgctc  65

<210>  197
<211>  65
<212>  DNA
<213>  Artificial

<220>
<223>  Synthetic DNA

<400>  197
ctcatgcagg tgccttccgt ctcaactgtt ccttggttaa gagaaaagaa ctggctggga  60

gacca  65

<210> 198
<211> 65
<212> DNA
<213> Artificial

<220>
<223> Synthetic DNA

<400> 198
gtccaatgac cctgtgcttg tagagtggcg ttttctttgc atccagagag gcagatttag 60

acacc 65

<210> 199
<211> 65
<212> DNA
<213> Artificial

<220>
<223> Synthetic DNA

<400> 199
agctacctgg gtccattcct cccccaaccc cagttccttc tattttccaa aagataaaaa 60

ccaaa 65

<210> 200
<211> 65
<212> DNA
<213> Artificial

<220>
<223> Synthetic DNA

<400> 200
aatcatatct tgcactgcct ggaattaagg acaatccgtt ctttctgcaa ctgtcttttc 60

acctc 65

<210> 201
<211> 65
<212> DNA
<213> Artificial

<220>
<223> Synthetic DNA

<400> 201
gaatgctggt ccactagtgg gatttctagg gttcaaaagt gacttcactt ccgggtcatc 60

atcag 65

<210> 202
<211> 65
<212> DNA
<213> Artificial

<220>

<223>  Synthetic DNA

<400>  202
gtgaagagtt tctcatcacg ggccgcctaa ggaacgggaa atttcacatc aatgcctgca    60

gcttc    65

<210>  203
<211>  65
<212>  DNA
<213>  Artificial

<220>
<223>  Synthetic DNA

<400>  203
cctccctccc ttactcccgt catgccagca actcgcaata tttcagatga cgtttacatg    60

gtagc    65

<210>  204
<211>  65
<212>  DNA
<213>  Artificial

<220>
<223>  Synthetic DNA

<400>  204
tagatctaag tcagctgttt gggttgagga ggagagaacc cgaggaaatg accatgctct    60

gggga    65

<210>  205
<211>  65
<212>  DNA
<213>  Artificial

<220>
<223>  Synthetic DNA

<400>  205
ggggaggaag aaaggtctaa catccttttc cttcatcatt ctcatttctg gacatgcctt    60

gtgag    65

<210>  206
<211>  65
<212>  DNA
<213>  Artificial

<220>
<223>  Synthetic DNA

<400>  206
aagctcttcg actccacgac tcttgaacat cagaagactt tccgaacaga gcgtcctgtc    60

aactc    65

<210> 207
<211> 65
<212> DNA
<213> Artificial

<220>
<223> Synthetic DNA

<400> 207
ccttcagtgt ctttgctaga tcaagtgcag acgctgcaca caatctctag ttcctctagt 60

tctgg 65

<210> 208
<211> 65
<212> DNA
<213> Artificial

<220>
<223> Synthetic DNA

<400> 208
cgaatggcta aggaggtata acgccgatat ttctaccttc ccgttggaaa acgcacctat 60

tggac 65

<210> 209
<211> 65
<212> DNA
<213> Artificial

<220>
<223> Synthetic DNA

<400> 209
gtttgcagaa gtatgtagca cttgtcctcc aagctttcag gtgtaagggg gaaaggtgaa 60

ccaca 65

<210> 210
<211> 65
<212> DNA
<213> Artificial

<220>
<223> Synthetic DNA

<400> 210
tgatcccttg ctgaatgcaa ggagctaacc agaaaagttc tgcttgacaa gtccccatcg 60

ttgaa 65

<210> 211
<211> 65
<212> DNA
<213> Artificial

<220>

<223>  Synthetic DNA

<400>  211
ctagcttgtc ctgagaagat atttaatttt gctaacactc agctctgccc tcccttgtcc        60

ccacc                                                                    65

<210>  212
<211>  65
<212>  DNA
<213>  Artificial

<220>
<223>  Synthetic DNA

<400>  212
aacaattgtc aaggaacctc atgtctcacc tcaggggcca gggtactctc tcacttaacc        60

accct                                                                    65

<210>  213
<211>  65
<212>  DNA
<213>  Artificial

<220>
<223>  Synthetic DNA

<400>  213
ccaagtctgt gtttattgaa ccatgtggat tactgcggga gaggactggc actcatgtgc        60

aaaaa                                                                    65

<210>  214
<211>  65
<212>  DNA
<213>  Artificial

<220>
<223>  Synthetic DNA

<400>  214
gacaaatgac ttgtagcttc agatgtcttt gcgccatcag cactcagaaa ggaaggggtc        60

tgagg                                                                    65

<210>  215
<211>  65
<212>  DNA
<213>  Artificial

<220>
<223>  Synthetic DNA

<400>  215
cgctgtctta gtactgtcac tgttgtaaag gcacatgttg gaacacatcc agtcctgctg        60

acctg                                                                    65

<210> 216
<211> 65
<212> DNA
<213> Artificial

<220>
<223> Synthetic DNA

<400> 216
gcgtctattt cttctctgga gacaaatact accgagtcaa ccttagaacc cggcgagtgg 60

actct 65

<210> 217
<211> 65
<212> DNA
<213> Artificial

<220>
<223> Synthetic DNA

<400> 217
ccatcgtgca ccgcaagtgc ttctaggcgg actgttactg agctgcgttt tacacccttt 60

ctttg 65

<210> 218
<211> 65
<212> DNA
<213> Artificial

<220>
<223> Synthetic DNA

<400> 218
gggctgccat ttgcagtggc aaagtggaga ttgttgccat caacgacccc ttcattgacc 60

tcaac 65

<210> 219
<211> 65
<212> DNA
<213> Artificial

<220>
<223> Synthetic DNA

<400> 219
atcagatgag gatatgggat tcggtctctt cgactaatcc cgccaaagca accaagtcag 60

cctgc 65

<210> 220
<211> 65
<212> DNA
<213> Artificial

<220>

<223> Synthetic DNA

<400> 220
gcatgttgac tcgtcctctg aaccaaagca cggacaggat taagagtgat ccaactttca 60

agtcg 65

<210> 221
<211> 65
<212> DNA
<213> Artificial

<220>
<223> Synthetic DNA

<400> 221
gcaggacctg tggccaagtt cttagtagct gtttatctgt gtgtaggact gtagaactgt 60

agagg 65

<210> 222
<211> 65
<212> DNA
<213> Artificial

<220>
<223> Synthetic DNA

<400> 222
acttgattga tgaatggacc tgagttttta cccggaggac cttagcacaa gaagaactgt 60

tgtcc 65

<210> 223
<211> 65
<212> DNA
<213> Artificial

<220>
<223> Synthetic DNA

<400> 223
gtccctgtgc ccacagattg cacgtgtagg gaggtgagtg cttgtatccc aaattggttc 60

taggt 65

<210> 224
<211> 65
<212> DNA
<213> Artificial

<220>
<223> Synthetic DNA

<400> 224
ccccaacttt caaacctctt taatttccta gctagaatcc cacaagcagc atagacctga 60

aagtg 65

<210>  225
<211>  65
<212>  DNA
<213>  Artificial

<220>
<223>  Synthetic DNA

<400>  225
cctgctcttc gtgttctctg ccctgctgga gtacgccgcc gtcaactttg tgtctcggca    60

acaca    65

<210>  226
<211>  65
<212>  DNA
<213>  Artificial

<220>
<223>  Synthetic DNA

<400>  226
ctgtcgctac atcactgaac ctctgctccc cacgggagcc gtgactgtaa tcgccctacg    60

ggtca    65

<210>  227
<211>  65
<212>  DNA
<213>  Artificial

<220>
<223>  Synthetic DNA

<400>  227
cccgtgcaga gctgcgcttg cagagattaa aatcaagtgt ggagcaacat gtggaactct    60

accag    65

<210>  228
<211>  65
<212>  DNA
<213>  Artificial

<220>
<223>  Synthetic DNA

<400>  228
aatcctctgg aaccccacac aggtctctgt tgaagactct gccaaccacg aggatcagta    60

cgaaa    65

<210>  229
<211>  65
<212>  DNA
<213>  Artificial

<220>

<223> Synthetic DNA

<400> 229
ttcaaggacc attctgctag atgccctact ccctgtgggt gaaaagtggg caaaggtctc 60

taagg 65

<210> 230
<211> 65
<212> DNA
<213> Artificial

<220>
<223> Synthetic DNA

<400> 230
gctggtacac acatgactca aaacaacaga tacatcattc cctagcagtc tggccagccc 60

cgttg 65

<210> 231
<211> 65
<212> DNA
<213> Artificial

<220>
<223> Synthetic DNA

<400> 231
gtcaaggcag taaggtgttc ttgggagcca cacttagact ctttccaaag atgtggaggg 60

aacag 65

<210> 232
<211> 65
<212> DNA
<213> Artificial

<220>
<223> Synthetic DNA

<400> 232
ggctgttgga atttacgcat aaagcagact gcatagatcc aatattgact gacccaagca 60

tgtta 65

<210> 233
<211> 65
<212> DNA
<213> Artificial

<220>
<223> Synthetic DNA

<400> 233
ctaggcttct tcaaatggag ctccagaatg tgcacgggac acatccgatc tatatttaga 60

gacct 65

<210>  234
<211>  65
<212>  DNA
<213>  Artificial

<220>
<223>  Synthetic DNA

<400>  234
tgaacgttgc agtgtgaagt ctgtactggc tagggatggt agcccagcgt gagttcaggc    60

atgaa    65

<210>  235
<211>  65
<212>  DNA
<213>  Artificial

<220>
<223>  Synthetic DNA

<400>  235
aagcagcttc cggctggtag atttttgtct tgatgggctg tccataagaa ctcccagttc    60

tttcc    65

<210>  236
<211>  65
<212>  DNA
<213>  Artificial

<220>
<223>  Synthetic DNA

<400>  236
aaataaagtg gcagccctta gacactgtga cggtcgtgtg cattgtggga gtggactttt    60

atgga    65

<210>  237
<211>  65
<212>  DNA
<213>  Artificial

<220>
<223>  Synthetic DNA

<400>  237
cctattcatc ttgttggaac cccaccccat tttccctgac agaacattcc taagtctcaa    60

gggct    65

<210>  238
<211>  65
<212>  DNA
<213>  Artificial

<220>

<223> Synthetic DNA

<400> 238
ccctgtctga tttgtaggaa tagataccct gcatgctatc attggctcat actctctccc 60

ccggc 65

<210> 239
<211> 65
<212> DNA
<213> Artificial

<220>
<223> Synthetic DNA

<400> 239
aaacacgtct tccctcgaag gttcccgtcg acctagggag gaccttacct gttcgtgaaa 60

cacac 65

<210> 240
<211> 65
<212> DNA
<213> Artificial

<220>
<223> Synthetic DNA

<400> 240
ttttgtgcct caaagttcgg ttggggctac cttgggatcc caaacagcta atatggtcac 60

caact 65

<210> 241
<211> 65
<212> DNA
<213> Artificial

<220>
<223> Synthetic DNA

<400> 241
gtgggggca gtttatttgt tgagagtgtg accaaaagtt acatgtttgc acctttctag 60

ttgaa 65

<210> 242
<211> 65
<212> DNA
<213> Artificial

<220>
<223> Synthetic DNA

<400> 242
taggcgcgga gatgggggtg cccgaacgtc ccaccctgct gcttttactc tccttgctac 60

tgatt 65

<210> 243
<211> 65
<212> DNA
<213> Artificial

<220>
<223> Synthetic DNA

<400> 243
gtagtgaatg accaagaata aggagaaaag ccaactcctt catcaggcat agagagctgc 60

agcaa 65


<210> 244
<211> 65
<212> DNA
<213> Artificial

<220>
<223> Synthetic DNA

<400> 244
cttgataacc gcagccacaa agccttagtg actttcctct acctggtaag acagagctct 60

tcatg 65


<210> 245
<211> 65
<212> DNA
<213> Artificial

<220>
<223> Synthetic DNA

<400> 245
cagaacactt agatggtgca gataaatctt gggaccacat tcctctaagc acggtttcta 60

gagtg 65


<210> 246
<211> 65
<212> DNA
<213> Artificial

<220>
<223> Synthetic DNA

<400> 246
gcctccggga actttccagg ttggcacctg aatgccttac tctcagcagt ctgaggctcg 60

cttgc 65


<210> 247
<211> 65
<212> DNA
<213> Artificial

<220>

<223>  Synthetic DNA

<400>  247
atagtgcaca aggatggtgg gaataaatga agcatcccac actcaacctc ccaacatcca        60

taact                                                                     65

<210>  248
<211>  65
<212>  DNA
<213>  Artificial

<220>
<223>  Synthetic DNA

<400>  248
gctgtggcat tgatgatacc tgcctcagtc aataaatact gaatatcaaa gcagtggatt        60

gcgta                                                                     65

<210>  249
<211>  65
<212>  DNA
<213>  Artificial

<220>
<223>  Synthetic DNA

<400>  249
agcctagcac cccaaagtca tgcacccagt atcctcttgt atgactgtat atgtctatgt        60

ctggg                                                                     65

<210>  250
<211>  65
<212>  DNA
<213>  Artificial

<220>
<223>  Synthetic DNA

<400>  250
gcaagccaga ataaagactg tgccagctgg tcagtcgccc ttttgagacc actcctttgt        60

gctcc                                                                     65

<210>  251
<211>  65
<212>  DNA
<213>  Artificial

<220>
<223>  Synthetic DNA

<400>  251
ggacgacatt gcaggcattc agaagttata tggaaagagg aacacgctgt gatagatgca        60

gacag                                                                     65

<210> 252
<211> 65
<212> DNA
<213> Artificial

<220>
<223> Synthetic DNA

<400> 252
ccagatggta ctaggggaca cagaagatct ggaacagagc caagacgtca catctgactg 60

cagac 65

<210> 253
<211> 65
<212> DNA
<213> Artificial

<220>
<223> Synthetic DNA

<400> 253
gtcctggcac tgaagccagc atgagatcca tcattcttat gtcagctcaa gggtcaaaag 60

gactt 65

<210> 254
<211> 65
<212> DNA
<213> Artificial

<220>
<223> Synthetic DNA

<400> 254
cccgatgctg ctttcccctg tggggatctc aggagctgtt tgaggatata ttttcacttt 60

gtgat 65

<210> 255
<211> 65
<212> DNA
<213> Artificial

<220>
<223> Synthetic DNA

<400> 255
gtgtcggcat aagccgaaga tatcggtaga gttatattga gcagatcccc cggtgaagga 60

tttaa 65

**Description**

Field of the Invention

The present invention relates to a method for evaluating conditions of an eye disease in a subject organism, and to a method for evaluating inhibitory or restorative functions on the eye disease of the subject organism by using the method for evaluating the conditions.

Background Art

Glaucoma, diabetic retinopathy, age-related macular degeneration, retinitis pigmentosa and the like are listed as the current leading causes of blindness. Those eye diseases are often caused by degenerated retinal cells. Therefore, to unravel the cause of an eye disease and establish its treatment, it is essential to examine what causes degeneration of retinal cells and how to unravel the mechanism.

However, much of the detail in how an eye disease develops is unknown, and there are few markers available that are effective in evaluating symptoms and developing therapeutic drugs, while not many methods are established for efficiently evaluating such markers. Conventionally employed diagnostic methods are ophthalmoscopy (fundus photography, radiography with contrast medium), measurement by optical coherence tomography, vision testing, Amsler testing and the like (non-patent publication 1). Also, genetic polymorphisms are compared to determine risk factors (patent publication 1). In addition, to develop therapeutic drugs, part of the gene is evaluated as a marker. However, for unraveling disease mechanisms and developing drugs, no method is available that enables efficient evaluation of molecular markers all at once. Thus, conventional methods such as Western Blotting and PCR have been employed, but such methods take a long time to conduct.

PRIOR ART PUBLICATION

PATENT PUBLICATION

Patent publication 1: JP2007-528371A

NON-PATENT PUBLICATION

Non-patent publication 1: "Age-Related Macular Degeneration" [online], Japan Ophthalmological Society [Internet search conducted June 6, 2012] (URL: http://www.nichigan.or.jp/public/disease/momaku_karei.jsp)

SUMMARY OF THE INVENTION

PROBLEMS TO BE SOLVED BY THE INVENTION

The present invention was carried out in consideration of the aforementioned problems. For evaluating conditions of eye diseases, the objective of the present invention is to provide a method that is capable of collectively analyzing molecular markers related to treatment or prevention of eye diseases such as age-related macular degeneration so that the genes essential to unraveling eye diseases are specified and efficient treatment and preventive methods are established.

SOLUTIONS TO THE PROBLEMS

The inventors of the present invention have studied intensively to solve the above problems and have found that conditions of an eye disease in a subject organism are evaluated by focusing on how a particular gene is expressed. Accordingly, the present invention has been completed.

Namely, the present invention is described as follows.

(1) Equipped with probes to detect at least one type of gene selected from among gene clusters 1 below, a microarray for evaluating the condition of an eye disease.
(2) The microarray described in (1) above to evaluate the presence of a disorder in the periphery of a retina.
(3) The microarray described in (1) above to evaluate the presence of a disorder in the retina.
(4) The microarray described in (1) above to evaluate the presence of light damage to the retina.
(5) The microarray described in (1) above to evaluate the presence of inflammation in the retina.

(6) The microarray described in (1) above to evaluate the presence of a disorder in the retinal pigment epithelium or choroid.
(7) The microarray described in (1) above to evaluate the presence of light damage to the retinal pigment epithelium or choroid.
(8) The microarray described in (1) above to evaluate the presence of inflammation in the retinal pigment epithelium or choroid.
(9) Equipped with probes to detect at least one type of gene selected from among gene clusters below, a microarray to evaluate the presence of a disorder in the eye.
<gene clusters>
Hspalb, Il1a, Gsk3a, H2-K1, IL1b
(10) Equipped with probes to detect at least one type of gene selected from among gene clusters below, a microarray to evaluate the presence of a disorder in the periphery of a retina.
<gene clusters>
Hspalb, Il1a, Gsk3a, H2-K1, IL1b
(11) Equipped with probes to detect at least one type of gene selected from among gene clusters below, a microarray to evaluate the presence of a disorder in the retina.
<gene clusters>
Il1a, H2-K1, Il1b, Il6, Hspa2
(12) Equipped with probes to detect at least one type of gene selected from among gene clusters below, a microarray to evaluate the presence of light damage to the retina.
<gene clusters>
Gsk3a, Rpe65, Gpr143, Hfe, Trip1
(13) Equipped with probes to detect at least one type of gene selected from among gene clusters below, a microarray to evaluate the presence of inflammation in the retina.
<gene clusters>
H2-K1, Il6, Cxcl1, Hfe, Trip1
(14) Equipped with probes to detect at least one type of gene selected from among gene clusters below, a microarray to evaluate the presence of a disorder in the retinal pigment epithelium or choroid.
<gene clusters>
Hspalb, Il1a, Gsk3a, H2-K1, IL1b
(15) Equipped with probes to detect at least one type of gene selected from among gene clusters below, a microarray to evaluate the presence of light damage to the retinal pigment epithelium or choroid.
<gene clusters>
Hspalb, Gsk3a, Il1b, Icam1, Hspa2
(16) Equipped with probes to detect at least one type of gene selected from among gene clusters below, a microarray to evaluate the presence of inflammation in the retinal pigment epithelium or choroid.
<gene clusters>
Hspalb, Il1a, H2-K1, Il1b, Icam1
(17) A microarray equipped with probes having at least one base sequence selected from among SEQ ID Nos. 1~219 and 221~254.
(18) A method for evaluating the condition of an eye disease in a subject organism, characterized by using a control to compare the detection results of the gene obtained from a sample taken from the subject organism by using the microarray according to any of microarrays in (1)~(17) above.
(19) A method for evaluating inhibitory or restorative functions of a test substance for an eye disease, characterized by using a control to compare the detection results of the gene obtained from a sample taken from a subject organism that has contacted, ingested or been administered the test substance by using the microarray according to any of (1)~(17) above.
(20) A method for evaluating the condition of an eye disease in a subject organism, characterized by using a control to compare through a multivariate analysis the detection results of the gene obtained from a sample taken from the subject organism by using the microarray according to any of (1)~(17) above.
(21) A method for evaluating inhibitory or restorative functions of a test substance for an eye disease, characterized by using a control to compare through a multivariate analysis the detection results of the gene obtained from a sample taken from a subject organism that has contacted, ingested or been administered the test substance by using the microarray according to any of (1)~(17) above.
<gene clusters 1>
A2m, Abca4, Ace, Adam17, Adam19, Adam28, Adam9, Adipor1, Adipor2, Agt, Aif1, Akt3, Amy2a2, Amy2a3, Amy2a4, Amy2a5, Angpt-1, Angpt-2, Aoc3, Apbb1, apln, Arr3, At1r, At2r, Atp6ap2, Best1, Bmp4, C1qa, C1qb, C1qc, C1ql1, C1s, C3, C4, Calb1, Calb2, Casp14, Casp3, Casp8, Casp9, Cckbr, Ccl17, Ccl2, Ccl3, Ccl4, Ccl7, Ccl8, Ccnd3, Ccr2, Cd44, Cd45, Cd55, Cd59a, Cd74, Cdh1, Cdh3, Cdh5, Cdr2, Cebpd, Cfb, Cfh, Chga, Chrna7, Ckb, Cldn5, Clip1, Cnga1, Cnga3, Cntf, Col7al, Col8a2, Cp, Cp, Crx, Ctgf, Ctnna1, Ctss, Cxcl1, Cxcl12, Cxcl2, cxcr4, Cyb5r1, Darc, Doc2b, Edn2, Ednrb, Efemp1, Efna5, Egf, egln3, Elovl4, Eno3, Epo, Erap1, esm1, Fgf16, Fgf7, Fgfr1, Flt1, Fos, Furin, Gabrb3, Gem, Gfap, Glut1, Gnao1, Gnat1, Gnat2, Gngt2, Gpnmb, Gpr143, Grem2, Grm2, Grm6, Gsk3a, Gsk3b, Guca1a, Guk1, H2-K1, Hfe, Hif1a, Hspa1a, Hspalb, Hspa2, Icam1, Id3, Ifna1, Ifnr, Igfl, Igf1r, Igfbp3, Il10, Il17a, Il1a, Il1b, Il6, Il6r, Il6st, Irf6, Irs1, Isgf3g, Itgav, Jak3, Jun, Kdr, Lgals3, Lipc, Lmo1, Loxl1, Mapk1, Mapk3, Mark2, Math5, Mef2c, Mkks, Mmp1, Mmp14, Mmp2, Mmp7, Mmp8, Mmp9, Msr1, Nes, Nfkb1, Nos3, Np, Nr2e3, Nrl, Nrp1, Nt5e, Opal, Opn1mw, Opn1sw, Osbpl1a, Pax6, Pde6a, Pde6b, Pdgfb, Pdpn, Pecam1, Pex1, Pgf, Pig7, Pkia, Pla2g5, Polg2, Ppara, Prkca, Prnp, Prom1, Ptgds, Ptgs1, Ptgs2, Pxmp3, Pygm, Rcv1, Rdh9, Ren, Rho, Robo4, Rom1, Rpe65, Rs1, Rxrg, S100a6, Sag, Scd1, Sdc2, Sele, Selenbpl, Selenbp2, Selp, Serpina3n, Serpinf1, Serping1, Sfrp5, Sill, Slc16a1, Slc16a4, Slc1a3, Socs3, Sox9, Sparc, Spp1, Stat1, Stat3, Stat5a, Stat6, Synpr, Tgfb1, Tgfb2, Tgfb3, Tgfbr2, Timp1, Timp2, Timp3, Tlr3, Tlr4, Tnfa, Tnfrsf1a, Trip1, Ttpa, Tyrp1, Usp9x, Vcam1, Vegfa, Vegfb, Vegfc, Vegfd, Vldlr, Vtn

(22) A method for evaluating the condition of an eye disease by using a microarray to measure the expression variation of a gene that is classified at least as (a)~(c) or (d)~(f) below respectively:

(a) a gene associated with both light damage and retinal inflammation caused by an inflammation-inducing substance in the retina;
(b) a gene associated with light damage to the retina, but excluding genes classified as (a) above;
(c) a gene associated with retinal inflammation caused by an inflammation-inducing substance, but excluding genes classified as (a) above;
(d) a gene associated with both light damage and inflammation caused by an inflammation-inducing substance in the retinal pigment epithelial cells and choroid;
(e) a gene associated with light damage to the retinal pigment epithelial cells and choroid, but excluding genes classified as (d) above;
(f) a gene associated with inflammation of the retinal pigment epithelial cells and choroid caused by an inflammation-inducing substance, but excluding genes classified as (d) above

(23) A method for evaluating inhibitory or restorative functions of a test substance for an eye disease by using a microarray to measure the expression variation of a gene that is classified at least as (a)~(c) or (d)~(f) below respectively:

(a) a gene associated with both light damage to the retina and retinal inflammation caused by an inflammation-inducing substance;
(b) a gene associated with light damage to the retina, but excluding genes classified as (a) above;
(c) a gene associated with retinal inflammation caused by an inflammation-inducing substance, but excluding genes classified as (a) above;
(d) a gene associated with both light damage and inflammation caused by an inflammation-inducing substance in the retinal pigment epithelial cells and choroid;
(e) a gene associated with light damage to the retinal pigment epithelial cells and choroid, but excluding genes classified as (d) above;
(f) a gene associated with inflammation of the retinal pigment epithelial cells and choroid caused by an inflammation-inducing substance, but excluding genes classified as (d) above

(24) The method described in (22) or (23) above, in which a gene classified as (a) is at least one cluster selected from a group of At1r, Jak3, Ccnd3, H2-K1, C1ql1, Id3, Tgfb2, Ckb, Gnat1, Efna5, Crx, Rom1, Arr3, Gsk3a, Adipor1, Hspalb, Guk1, Abca4, egln3, Gngt2, Clip1, Prnp, Sparc, Elovl4, Gsk3b, Itgav, Vegfa, Vegfb, Vegfc, Vegfd, Sdc2 and Trip1;
a gene classified as (b) is at least one cluster selected from a group of At2r, Pig7, Pgf, Rxrg, Col7a1, Casp9, Pecam1, Rpe65, Cckbr, Cd59a, Opn1mw, Grm6, Pkia, Darc, Apbb1, Prom1, Adam9, Cyb5r1, Gpr143, Atp6ap2, Nr2e3, Pde6a, Nrl, Cnga1, Hif1a, Gnat2 and Mmp2;
a gene classified as (c) is at least one cluster selected from a group of Cxcll, Il6, Selenbp2, Nfkb1, Cldn5, Sox9, Cp, Grm2, Pax6, Prkca, Mark2, Ppara, Gem, Opn1sw, Robo4, Rho, Glut1 and Pex1;
a gene classified as (d) is at least one cluster selected from a group of cxcr4, At2r, Vcam1, Mef2c, Pkia, Scd1, Loxl1, H2-K1, Pxmp3, Erap1, Pgf, Tgfb3, Pdpn, Gsk3a, Fgf7, Ccl2, Cntf, Col8a2, Pecam1, Cd59a, Rxrg, C1s, Ccl7, Osbpl1a, Glut1, Selenbp2, Serpinf1, Gpnmb, Hspa2, Nes, Stat1, Egf, C1qb, Mmp14, Timp2, Lmo1, Lgals3, Mmp8, Flt1, Cldn5, Mmp2, Vegfa, Vegfb, Vegfc, Vegfd, Hspalb, Kdr and Stat6;
a gene classified as (e) is at least one cluster selected from a group of Cfb, Mmp9, Calb2, Robo4, Gpr143, Cd44, Pig7, Il1b, C3, Gem, Cd55, Cebpd, Stat3 and Cnd3; and
a gene classified as (f) is at least one cluster selected from a group of Cxcl1, Timp1, Cxcl2, Il6, Igfl, Icam1, Lipc, At1r, Spp1, Ctss, C1qc, Nfkb1, Grem2, Abca4, Apbb1, Chrna7, Cyb5r1, Pdgfb, Isgf3g, Nos3 and Clip1.
(25) The method described in any of (22)~(24) above, characterized by using the determination formula below to evaluate the condition of an eye disease.
determination formula

$$M=(Xli\times\eta 1 / \beta 1+ \cdots + Xki\times\eta k / \beta k) (/\eta 1+ \cdots + \eta k )$$

When M1 max + $\sigma_{m1}$ <M2 min is satisfied, the condition of an evaluation sample cluster is the same as that in each disease model.
[in the formula, "M" represents the Mahalanobis distance to show the distance from the base space, "Xni (n=1~k)" represents a gene expression level or gene expression ratio, "ηn (n=1~k)" represents a signal-to-noise ratio (S/N) in each sample, and "βn (n=1~k) represents the sensitivity of each sample.
"M1" represents a normal sample cluster or a control sample cluster. "M1 max" indicates the maximum value of "M1" and "σ m1" represents the standard deviation from "M1". "M2" represents an evaluation sample cluster, and "M2 min" indicates the minimum value of "M2".
(26) A microarray to evaluate the condition of an eye disease in which the nucleic acids or part of the nucleic acids described in (i), (ii) or (iii) below are mounted.

(i) nucleic acids containing a gene selected from (a)~(c) or (d)~(f) below:

(a) a gene associated with both light damage to the retina and retinal inflammation caused by an inflammation-inducing substance;
(b) a gene associated with light damage to the retina, but excluding genes classified as (a) above;
(c) a gene associated with retinal inflammation caused by an inflammation-inducing substance, but excluding genes classified as (a) above;
(d) a gene associated with both light damage and inflammation caused by an inflammation-inducing substance in the retinal pigment epithelial cells and choroid;
(e) a gene associated with light damage to the retinal pigment epithelial cells and choroid, but excluding genes classified as (d) above;
(f) a gene associated with inflammation of the retinal pigment epithelial cells and choroid caused by an inflammation-inducing substance, but excluding genes classified as (d) above

(ii) nucleic acids composed of a base sequence complementary to the base sequence of the nucleic acids in (i) above; and
(iii) nucleic acids capable of hybridizing under stringent conditions with nucleic acids composed of a base sequence complementary to that in the nucleic acids in (i) or (ii) above.

(27) A microarray described in (26) above, in which a gene classified as (a) is at least one cluster selected from a group of At1r, Jak3, Ccnd3, H2-K1, C1ql1, Id3, Tgfb2, Ckb, Gnat1, Efna5, Crx, Rom1, Arr3, Gsk3a, Adipor1, Hspalb, Guk1, Abca4, egln3, Gngt2, Clip1, Prnp, Sparc, Elovl4, Gsk3b, Itgav, Vegfa, Vegfb, Vegfc, Vegfd, Sdc2 and Trip1;
a gene classified as (b) is at least one cluster selected from a group of At2r, Pig7, Pgf, Rxrg, Col7a1, Casp9, Pecam1, Rpe65, Cckbr, Cd59a, Opn1mw, Grm6, Pkia, Darc, Apbb1, Prom1, Adam9, Cyb5r1, Gpr143, Atp6ap2, Nr2e3, Pde6a, Nrl, Cnga1, Hif1a, Gnat2 and Mmp2;
a gene classified as (c) is at least one cluster selected from a group of Cxcl1, Il6, Selenbp2, Nfkb1, Cldn5, Sox9, Cp, Grm2, Pax6, Prkca, Mark2, Ppara, Gem, Opn1sw, Robo4, Rho, Glut1 and Pex1;
a gene classified as (d) is at least one cluster selected from a group of cxcr4, At2r, Vcam1, Mef2c, Pkia, Scd1, Loxl1, H2-K1, Pxmp3, Erap1, Pgf, Tgfb3, Pdpn, Gsk3a, Fgf7, Ccl2, Cntf, Col8a2, Pecam1, Cd59a, Rxrg, C1s, Ccl7, Osbpl1a, Glut1, Selenbp2, Serpinf1, Gpnmb, Hspa2, Nes, Stat1, Egf, C1qb, Mmp14, Timp2, Lmo1, Lgals3, Mmp8, Flt1, Cldn5, Mmp2, Vegfa, Vegfb, Vegfc, Vegfd, Hspalb, Kdr and Stat6;
a gene classified as (e) is at least one cluster selected from a group of Cfb, Mmp9, Calb2, Robo4, Gpr143, Cd44, Pig7, Il1b, C3, Gem, Cd55, Cebpd, Stat3 and Ccnd3; and
a gene classified as (f) is at least one cluster selected from a group of Cxcl1, Timp1, Cxcl2, Il6, Igf1, Icam1, Lipc, At1r, Spp1, Ctss, C1qc, Nfkb1, Grem2, Abca4, Apbb1, Chrna7, Cyb5r1, Pdgfb, Isgf3g, Nos3 and Clip1.
(27) Equipped with a probe to detect at least one type of gene selected from among the clusters of Hspalb, Gsk3a and H2-K1, a microarray to evaluate the condition of an eye disease.

EFFECTS OF THE INVENTION

The embodiments of the present invention are capable of conducting objective evaluation of the condition of an eye disease in a subject organism. Also, the methods according to the embodiments are capable of evaluating inhibitory or restorative functions of foods or drugs on eye diseases.

BRIEF DESCRIPTION OF THE DRAWINGS

Fig. 1 is a view schematically showing an array fixing device to be used in the embodiments of the present invention;
Fig. 2A is a view showing measurement results of the gene expression variation in a retina;
Fig. 2B is a view showing measurement results of the gene expression variation in a retina;
Fig. 2C is a view showing measurement results of the gene expression variation in a retina;
Fig. 2D is a view showing measurement results of the gene expression variation in a retina;
Fig. 2E is a view showing measurement results of the gene expression variation in a retina;
Fig. 2F is a view showing measurement results of the gene expression variation in a retina;
Fig. 2G is a view showing measurement results of the gene expression variation in a retina;
Fig. 2H is a view showing measurement results of the gene expression variation in a retina;
Fig. 2I is a view showing measurement results of the gene expression variation in a retina;
Fig. 2J is a view showing measurement results of the gene expression variation in a retina;
Fig. 2K is a view showing measurement results of the gene expression variation in a retina;
Fig. 2L is a view showing measurement results of the gene expression variation in a retina;
Fig. 3A is a view showing measurement results of the gene expression variation in a retinal pigment epithelium and choroid;
Fig. 3B is a view showing measurement results of the gene expression variation in a retinal pigment epithelium and choroid;
Fig. 3C is a view showing measurement results of the gene expression variation in a retinal pigment epithelium and choroid;
Fig. 3D is a view showing measurement results of the gene expression variation in a retinal pigment epithelium and choroid;
Fig. 3E is a view showing measurement results of the gene expression variation in a retinal pigment epithelium and choroid;
Fig. 3F is a view showing measurement results of the gene expression variation in a retinal pigment epithelium and choroid;
Fig. 3G is a view showing measurement results of the gene expression variation in a retinal pigment epithelium and choroid;
Fig. 3H is a view showing measurement results of the gene expression variation in a retinal pigment epithelium and choroid;
Fig. 3I is a view showing measurement results of the gene expression variation in a retinal pigment epithelium and choroid;
Fig. 3J is a view showing measurement results of the gene expression variation in a retinal pigment epithelium and choroid;
Fig. 3K is a view showing measurement results of the gene expression variation in a retinal pigment epithelium and choroid;
Fig. 3L is a view showing measurement results of the gene expression variation in a retinal pigment epithelium and choroid; and
Fig. 3M is a view showing measurement results of the gene expression variation in a retinal pigment epithelium and choroid.

DETAILED DESCRIPTION OF THE EMBODIMENTS

In the following, the present invention is described in detail. The embodiments below are examples to describe the present invention. The present invention is not limited to those embodiments. Thus, various modifications are possible within a scope that does not deviate from the gist of the present invention. Also, the present application is based upon and claims the benefit of priority to Japanese Patent Application No. 2013-080395, filed April 8, 2013. The entire contents of the specification and accompanying drawings are incorporated herein by reference.

1. Summary

The present invention relates to a microarray used to evaluate the condition of an eye disease, and to a method for evaluating the condition of an eye disease in the subject organism by detecting a gene in a sample taken from a subject organism using the microarray. In addition, the present invention relates to a method for evaluating inhibitory or restorative functions of a test substance for an eye disease by retrieving a sample from a subject organism that has contacted, ingested or been administered the test substance, and by detecting the gene in the sample and measuring the gene expression variations.

The inventors of the present invention have conducted intensive studies about genes associated with many kinds of diseases and identified genes associated with eye diseases, especially age-related macular degeneration in the retina. The inventors have found that the conditions of eye diseases are evaluated by focusing on gene expression variations, and have completed the present invention.

Namely, the present invention relates to a method for evaluating the condition of an eye disease in a subject organism by using as an index the expression level of at least one type of gene selected from among predetermined gene clusters, and to a method for evaluating the functions (effects) of a test substance for the eye disease.

2. Gene

The gene to be detected in the present embodiment is at least one type of gene selected from among the gene clusters below. Each gene is denoted according to official symbols specified by NCBI (the National Center for Biotechnology Information). Each gene below is denoted by using an official symbol specified for the mouse species. However, when genes are common to other animal species, denoted symbols do not specify any particular animal species.

<gene clusters>

A2m, Abca4, Ace, Adam17, Adam19, Adam28, Adam9, Adipor1, Adipor2, Agt, Aif1, Akt3, Amy2a2, Amy2a3, Amy2a4, Amy2a5, Angpt-1, Angpt-2, Aoc3, Apbb1, apln, Arr3, At1r, At2r, Atp6ap2, Best1, Bmp4, C1qa, C1qb, C1qc, C1ql1, C1s, C3, C4, Calb1, Calb2, Casp14, Casp3, Casp8, Casp9, Cckbr, Ccl17, Ccl2, Ccl3, Ccl4, Ccl7, Ccl8, Ccnd3, Ccr2, Cd44, Cd45, Cd55, Cd59a, Cd74, Cdh1, Cdh3, Cdh5, Cdr2, Cebpd, Cfb, Cfh, Chga, Chrna7, Ckb, Cldn5, Clip1, Cnga1, Cnga3, Cntf, Col7a1, Col8a2, Cp, Cp, Crx, Ctgf, Ctnna1, Ctss, Cxcl1, Cxcl12, Cxcl2, cxcr4, Cyb5r1, Darc, Doc2b, Edn2, Ednrb, Efemp1, Efna5, Egf, egln3, Elovl4, Eno3, Epo, Erap1, esm1, Fgf16, Fgf7, Fgfr1, Flt1, Fos, Furin, Gabrb3, Gem, Gfap, Glut1, Gnao1, Gnat1, Gnat2, Gngt2, Gpnmb, Gpr143, Grem2, Grm2, Grm6, Gsk3a, Gsk3b, Guca1a, Guk1, H2-K1, Hfe, Hif1a, Hspa1a, Hspalb, Hspa2, Icam1, Id3, Ifna1, Ifnr, Igfl, Igf1r, Igfbp3, Il10, Il17a, Il1a, Il1b, I16, Il6r, Il6st, Irf6, Irs1, Isgf3g, Itgav, Jak3, Jun, Kdr, Lgals3, Lipc, Lmo1, Loxl1, Mapk1, Mapk3, Mark2, Math5, Mef2c, Mkks, Mmp1, Mmp14, Mmp2, Mmp7, Mmp8, Mmp9, Msr1, Nes, Nfkb1, Nos3, Np, Nr2e3, Nrl, Nrp1, Nt5e, Opal, Opn1mw, Opn1sw, Osbplla, Pax6, Pde6a, Pde6b, Pdgfb, Pdpn, Pecam1, Pex1, Pgf, Pig7, Pkia, Pla2g5, Polg2, Ppara, Prkca, Prnp, Prom1, Ptgds, Ptgs1, Ptgs2, Pxmp3, Pygm, Rcv1, Rdh9, Ren, Rho, Robo4, Rom1, Rpe65, Rs1, Rxrg, S100a6, Sag, Scd1, Sdc2, Sele, Selenbp1, Selenbp2, Selp, Serpina3n, Serpinf1, Serping1, Sfrp5, Sill, Slc16a1, Slc16a4, Slc1a3, Socs3, Sox9, Sparc, Spp1, Stat1, Stat3, Stat5a, Stat6, Synpr, Tgfb1, Tgfb2, Tgfb3, Tgfbr2, Timp1, Timp2, Timp3, Tlr3, Tlr4, Tnfa, Tnfrsf1a, Trip1, Ttpa, Tyrp1, Usp9x, Vcam1, Vegfa, Vegfb, Vegfc, Vegfd, Vldlr, Vtn

The expression levels of a gene included in the above gene clusters vary depending on the condition of an eye disease in a subject organism. The genes to be detected in the present embodiment are classified as shown in Table 4.

(i) genes that show variation in the expression level when light damage occurs in the retina
(ii) genes that show variation in the expression level when inflammation is caused in the retina
(iii) genes that show variation in the expression level when light damage occurs in the retinal pigment epithelium or choroid
(iv) genes that show variation in the expression level when inflammation is caused in the retinal pigment epithelium or choroid

According to the embodiments of the present invention, by detecting at least one type of gene selected from among gene clusters described in (i)~(iv) above respectively and by comparing the detection results with a control, the condition of an eye disease in a subject organism is evaluated. For example, by detecting at least one type of gene selected from among gene clusters in (i) and by comparing the detected results with a control, whether or not light damage has occurred in the retina of the subject organism is objectively evaluated. Also, the method is capable of evaluating the condition of retinal disorder such as light damage to the retina or age-related macular degeneration caused by light damage. The same applies to other gene clusters.

In addition, genes subject to being detected in another embodiment of the present invention are classified as (a)~(c) or (d)~(f) below.

(a) a gene associated with both light damage to the retina and retinal inflammation caused by an inflammation-inducing substance;
(b) a gene associated with light damage to the retina, but excluding genes classified as (a) above;
(c) a gene associated with retinal inflammation caused by an inflammation-inducing substance, but excluding genes classified as (a) above;
(d) regarding retinal pigment epithelial cells and choroid, a gene associated with both light damage and inflammation caused by an inflammation-inducing substance;
(e) a gene associated with light damage to the retinal pigment epithelial cells and choroid, but excluding genes classified as (d) above;
(f) a gene associated with inflammation of the retinal pigment epithelial cells and choroid caused by an inflammation-inducing substance, but excluding genes classified as (d) above

A gene classified as (a) above is at least one type selected from among Atlr, Jak3, Ccnd3, H2-K1, C1ql1, Id3, Tgfb2, Ckb, Gnat1, Efna5, Crx, Rom1, Arr3, Gsk3a, Adipor1, Hspalb, Guk1, Abca4, egln3, Gngt2, Clipl, Prnp, Sparc, Elovl4, Gsk3b, Itgav, Vegfa, Vegfb, Vegfc, Vegfd, Sdc2 and Trip1; preferably at least one type selected from among At1r, Jak3, Ccnd3, H2-K1, C1ql1, Id3, Tgfb2, Ckb, Gnat1, Efna5, Crx, Rom1, Arr3, Gsk3a, Adipor1, Hspalb, Guk1, Abca4, egln3, Vegfa, Vegfb, Vegfc and Vegfd; more preferably at least one type selected from among At1r, Jak3, Ccnd3, H2-K1, C1ql1, Id3, Tgfb2, Vegfa, Vegfb, Vegfc and Vegfd;
a gene classified as (b) above is at least one type selected from among At2r, Pig7, Pgf, Rxrg, Col7a1, Casp9, Pecam1, Rpe65, Cckbr, Cd59a, Opn1mw, Grm6, Pkia, Darc, Apbb1, Prom1, Adam9, Cyb5r1, Gpr143, Atp6ap2, Nr2e3, Pde6a, Nrl, Cnga1, Hif1a, Gnat2 and Mmp2; preferably at least one type selected from among At2r, Pig7, Pgf, Rxrg, Col7a1, Casp9, Pecam1, Rpe65, Cckbr, Cd59a, Opn1mw, Grm6, Pkia, Darc, Apbb1, Prom1, Adam9, Cyb5r1, Gpr143 and Atp6ap2; more preferably at least one type selected from among At2r, Pig7, Pgf, Rxrg, Col7a1 and Casp9;
a gene classified as (c) above is at least one type selected from among Cxcll, Il6, Selenbp2, Nfkb1, Cldn5, Sox9, Cp, Grm2, Pax6, Prkca, Mark2, Ppara, Gem, Opn1sw, Robo4, Rho, Glut1 and Pex1; preferably at least one type selected from among Cxcl1, Il6, Selenbp2, Nfkb1, Cldn5, Sox9, Cp, Grm2, Pax6, Prkca, Mark2, Ppara and Gem; more preferably at least one type selected from among Cxcl1, Il6, Selenbp2 and Nfkb1;
a gene classified as (d) above is at least one type selected from among cxcr4, At2r, Vcam1, Mef2c, Pkia, Scd1, Loxl1, H2-K1, Pxmp3, Erap1, Pgf, Tgfb3, Pdpn, Gsk3a, Fgf7, Ccl2, Cntf, Col8a2, Pecam1, Cd59a, Rxrg, C1s, Ccl7, Osbplla, Glut1, Selenbp2, Serpinf1, Gpnmb, Hspa2, Nes, Stat1, Egf, C1qb, Mmp14, Timp2, Lmo1, Lgals3, Mmp8, Flt1, Cldn5, Mmp2, Vegfa, Vegfb, Vegfc, Vegfd, Hspalb, Kdr and Stat6; preferably at least one type selected from among cxcr4, At2r, Vcam1, Mef2c, Pkia, Scd1, Loxll, H2-K1, Pxmp3, Erap1, Pgf, Tgfb3, Pdpn, Gsk3a, Fgf7, Ccl2, Cntf, Col8a2, Pecam1, Cd59a, Rxrg, C1s, Ccl7, Osbplla, Glut1, Selenbp2, Serpinf1, Gpnmb, Hspa2, Vegfa, Vegfb, Vegfc and Vegfd; more preferably at least one type selected from among cxcr4, At2r, Vcam1, Mef2c, Pkia, Scd1, Loxl1, H2-K1, Pxmp3, Erap1, Pgf, Tgfb3, Pdpn, Gsk3a, Fgf7, Vegfa, Vegfb, Vegfc and Vegfd;
a gene classified as (e) above is at least one type selected from among Cfb, Mmp9, Calb2, Robo4, Gpr143, Cd44, Pig7, Il1b, C3, Gem, Cd55, Cebpd, Stat3 and Ccnd3; preferably at least one type selected from among Cfb, Mmp9, Calb2, Robo4, Gpr143, and Cd44; more preferably at least one type selected from among Cfb, Mmp9, Calb2, and Robo4; and a gene classified as (f) above is at least one type selected from among Cxcll, Timp1, Cxcl2, Il6, Igfl, Icam1, Lipc, At1r, Spp1, Ctss, C1qc, Nfkb1, Grem2, Abca4, Apbb1, Chrna7, Cyb5r1, Pdgfb, Isgf3g, Nos3, and Clip1; preferably at least one type selected from among Cxcl1, Timp1, Cxcl2, Il6, Igf1, Icam1, Lipc, At1r, Spp1, Ctss, C1qc, and Nfkb1; and more preferably at least one type selected from among Cxcl1, Timp1, Cxcl2, Il6, and Igfl.

A gene classified as any of (a)~(c) above and a gene classified as any of (d)~(f) above may be combined freely to be used for evaluating the condition of an eye disease. More specifically, the following combinations may be used:

a combination of a gene classified as (a) and a gene classified as (d);
a combination of a gene classified as (a) and a gene classified as (e);
a combination of a gene classified as (a) and a gene classified as (f);
a combination of a gene classified as (b) and a gene classified as (d);
a combination of a gene classified as (b) and a gene classified as (e);
a combination of a gene classified as (b) and a gene classified as (f);
a combination of a gene classified as (c) and a gene classified as (d);
a combination of a gene classified as (c) and a gene classified as (e); and
a combination of a gene classified as (c) and a gene classified as (f).

Also, to distinguish light damage to the retina from retinal inflammation caused by an inflammation-inducing substance, a combination of Cxcl1, At2r, Vegfa, Cxcl1, Cfb and Fgf7 may be used for purposes of making such distinction. Regarding disorder in the retinal pigment epithelium and choroid, to distinguish light damage from inflammation caused by an inflammation-inducing substance, a combination of Il6, Pig7, Vegfa, Timp1, Mmp9 and Vegfc may be used for such purposes of distinction.

In yet another embodiment, it is preferred to detect at least one type of gene selected from among Hspalb, Gsk3a and H2-K1 when evaluating the condition of an eye disease.

In the embodiments of the present invention, an "associated gene" indicates such a gene that shows variation in its expression level in the case of light damage or inflammation caused by an inflammation-inducing substance in the retina, retinal pigment epithelial cells and/or choroid. In the present invention, the condition of an eye disease is evaluated by detecting the associated gene (by determining its expression level). Also, in the embodiments of the present invention, "nucleic acids" include RNA and DNA (cDNA or the like). Moreover, in the embodiments of the present invention, "part of nucleic acids" indicates a polynucleotide having part of the base sequence of nucleic acids. Such nucleic acids may be used as later-described probes.

In the embodiments of the present invention, "at least one type selected from among" gene clusters is defined to include "all the genes" of gene clusters and "combinations of all the genes" of gene clusters.

3. Probe

In the embodiments of the present invention, "to detect a gene" means to determine the expression level of the gene, that is, to measure the level of mRNA or the level of nucleic acids amplified from DNA or mRNA. A probe to detect a gene means the nucleic acids to be hybridized under stringent conditions with its mRNA, cDNA or their antisense strand.

Stringent conditions for hybridization are, for example, "0.12M Tri·HCl/0.12M NaCl/0.5% Tween-20, 50°C", "0.12M Tris·HCl/0.12M NaCl/0.5% Tween-20, 42°C" or "0.12M Tris·HCl/0.12M NaCl/0.5% Tween-20, 37°C"; more stringent conditions are, for example, "0.12M Tris·HCl/0.12M NaCl/0.5% Tween-20, 65°C," "0.12M Tris·HCl/0.12M NaCl/0.5% Tween-20, 68°C" or "0.06M Tris·HCl/0.06M NaCl/0.5% Tween-20, 65°C". More specifically, the temperature is maintained at 65°C for at least an hour after a probe is added so as to hybridize the nucleic acids. Then, washing is conducted at 65°C for 20 minutes 2~4 times in a buffer of 0.12M Tris·HCl/0.12M NaCl/0.5% Tween-20, and a final washing is conducted at 65°C for 10 minutes in a buffer of 0.12M Tris·HCl/0.12M NaCl. More stringent conditions may be set by increasing the temperature for hybridization or washing. In addition to the conditions of a buffer such as base concentration and temperature, it is an option for a person in the art to add several more conditions such as probe concentration, probe length and reaction time. "Molecular Cloning, A Laboratory Manual, 2nd ed." (Cold Spring Harbor Press, 1989), "Current Protocols in Molecular Biology" (John Wiley & Sons, 1987-1997) or the like may be referred to for detailed procedures of hybridization methods.

Also, the embodiments of the present invention provide probes having base sequences shown in SEQ ID Nos. 1~219 and 221~254. Probes having base sequences shown in SEQ ID Nos. 1~219 and 221~254 (probe numbers 1~219 and 221-254) are shown in Table 1 below. In addition, as for a microarray, it is sufficient if it is mounted with probes having base sequences listed in SEQ ID Nos. 1~219 and 221~254. The microarray may further mount negative control (N.C.) probes. As for N.C. probes, they are not limited specifically as long as no gene of the target animal species is detected under stringent conditions. For example, probes mounting YPL088W-713 and 0mpA shown in Table 1 below (SEQ ID Nos. 220 and 255 respectively) may be used. However, that is not the only option.

Table 1

[Table 1-1-1]

| Probe Sequence List | | |
|---|---|---|
| Probe # | Symbol | sequence |
| 1 | A2m | gtagctggagaagggtgtgtctacctccagacatccttgaaatatagtgttctcccgagggagaa |
| 2 | Abca4 | tccagaacccctgactggtggctttggccttagcgatactttcattctgagtggatctgcttttg |
| 3 | Ace | tgaattacttcaagccactgacagaatggctcgtcaccgagaacaggagacatggagagacactg |
| 4 | Adam9 | ctctatggtacgaggtgtttagtatacccaagcagataggtgtcgatcgaacaggagcagggaga |
| 5 | Adipor1 | ccccttacccccgtccttactttgtaacctggctgataacgggccatccatttttgtagcacact |
| 6 | Agt | tttctgggcagagtgaetanccccngagtgtggtgtgaggccttgtgcatagccatggagacaa |
| 7 | Aif1 | gctgaagagattaattagagaggtgtccagtggctccgaggagacgttcagctactctgactttc |

(continued)

| Probe Sequence List | | |
|---|---|---|
| Probe # | Symbol | sequence |
| 8 | Akt3 | cgtctgtactgtctacatcacggttcccttagcttgctcctggtagtgcattacaggcaagcatg |
| 9 | Amy2a2 | gcctgacctgatagagcagggtgattctgctggagctatgggtcaegatatggagacctggaagg |
| 10 | Amy2a3 | gcctgacctgatagagcagggtgattctgctggagctatgggtcasgatatggagacctggaagg |
| 11 | Amy2a4 | gcctgacctgatagagcagggtgattctgctggagctatgggtcaegatatggagacctggaagg |
| 12 | Amy2a5 | ccaagaggtcattgatctgggtggtgaggcaattaaaggtagtgagtactttggaaatggccgtg |
| 13 | Apbb1 | attccttcacagaagtcattacactggactgatgacatgaggggccagaagcaaagccagccttg |
| 14 | Arr3 | gagttaagaggatccgatagcctatctctgataattctgtgtggaagcccccactgcaacactct |
| 15 | At1r | ggtgtgccctactcagtctccagaacagccctgtaggtccagccctctctgcaaccagagaacct |
| 16 | At2r | ctgcctgtcccatattataccaggtcacctaagaccttcctggattgatgctgacctatgaggta |
| 17 | Atp6ap2 | ggaaatacgcactgagagaactgtaaaccacttagtcatgttactcgcgctggagaacgcactgg |
| 18 | Best1 | gccgtttggaccagatgtcaaccaatatacaggctctaatgaaggagcatgcagagtcctatccc |
| 19 | Bmp4 | ttatcaggagatggtggtagaggggtgtggatgccgctgagatcaggcagtccagagggcggaca |
| 20 | C1qa | ctgtgcagtgcccggcttctattacttcaacttccaagtgatctcceagtgggacctttgtctgt |
| 21 | C1qb | ccccttgactgcctgaaacccagaccagagccctgtagatgttacagaacgaatgggtcaataaa |
| 22 | C1qc | cctgcggctccagaggggcgatgaggtgtggctatcagtcaatgactacaatggcatggtgggca |
| 23 | C1ql1 | agttggcgcggttgcttcctgtagggtgaccctgccttggggaggagtgggttaggggttggata |
| 24 | C1s | gcatggaggcagggttgatcactgagccgtgttggttattcagttactattgctaacaacatggc |
| 25 | C3 | agttcaccagtactttaatgtgggacttatccagcacgggtcggtcaaggtctactcctattaca |
| 26 | C4 | ctttgagtccaagatcacccnagtcctgcatttcagaaaggacaccatggcctccataggtcaga |
| 27 | Calb1 | cccaggatggggegaagtgatacactggctttacttcggcacgtatttggaagcagtgactttag |
| 28 | Calb2 | aacaagaaggagatgaacatccaacagctcaccacctacaggaagagtgtcatgtccttggccga |

[Table 1-1-2]

| | | |
|---|---|---|
| 29 | Casp14 | ccaaaaccccatgagctaggcctcaactaccacaaaacagcctatgagctctgtttccaggattc |
| 30 | Casp3 | ccggtggaggctgacttcctgtatgcttactctacagcacctggttactattcctggagaaattc |
| 31 | Casp8 | aagaactgcgtttcctaccgagatcctgtgaatggaacctggtatattcagtcactttgccagag |
| 32 | Casp9 | tttgctttgtgaaagtccacttgagtttagatggtagatgtgccaagccttgtttgtttattttt |
| 33 | Cckbr | ggcctaaaatcacatgcctttcggagccgcagatctcttagcactgaaaaagtcccgtcatccct |
| 34 | Ccl17 | atcaggaagttggtgagctggtataagacctcagtggagtgttccagggatgccatcgtgtttct |
| 35 | Ccl2 | cacttctgtaggagtgaccagtgtgacagtgaactagtgtgactcggactgtgatgccttaatta |
| 36 | Ccl3 | tctcctacagccggaagattccacgccaattcatcgttgactattttgaaaccagcagcctttgc |
| 37 | Ccl4 | agcagtctttgctccaagccagctgtggtattcctgaccaaaagaggcagacagatctgtgctaa |
| 38 | Ccl7 | cggtcctaagggataggagctgtctgtaggaatgtgaaacagtcacgcctaaggaatggtcttta |
| 39 | Ccl8 | gctgctttcatgtactaaagctgaagatccccccttcgggtgctgaaaagctacgagagaatcaac |
| 40 | Ccnd3 | ttgagtccctcttctgtccggggctccaaccttctcagttgccaaascgccccagtaccttccaa |
| 41 | Cd44 | ttgattgactaataataatgaggaaaacctgatgtgtacattacccgattgaaagtgtgtattgg |

(continued)

| | | |
|---|---|---|
| 42 | Cd45 | ggcgcatcagaaggggataaagaggactctgttttctcactagccactcacagatttctatctca |
| 43 | Cd55 | gtttaataaccttgacagttttgcatgtgatgctatcactcattggctacttgacatagccaacg |
| 44 | Cd59a | gagctggtgtgtgaatgttccagttgtgagctgtcagttaggggcagtgactgttcacttgctta |
| 45 | Cd74 | ctaccgctatctaaagggaacccccatttctgacccattagtagtcttgaatgtggggctctgag |
| 46 | Cdh1 | tggccgatttaaacccaagttgcccagttctgagtagaaaactgagactatgctgtgtgtggcgg |
| 47 | Cdh3 | gcctgggttacctatcacaactctgtctcagagaacagaaatactttccaggccagccagagctt |
| 48 | Cdh5 | gtcgaatttgaagcaactgtgaattcacccagggaggactgtggagaccataggtgacaggtcat |
| 49 | Cdr2 | cctgcagcgattcctgtcgactcccttacacagtattgatcttctgactgtggaaacaaccttcc |
| 50 | Cebpd | ggtgacagtgccacctctggcagctcccagaacactaaaaccacaaagtgtttaggttggacatt |
| 51 | Cfb | aggtgtacatcaagaatggggacaagaaagccagttgtgagagagatgctacaaaggcccaaggc |
| 52 | Cfh | ccgagactcaacagggaaatgtgggcctcctccacctattgacaatggagacatcacctccttgt |
| 53 | Chga | ccttcttatccatggggcacaactgcaataacttctgacctttgggcgaaagccgagaactcctg |
| 54 | Chrna7 | tcccagcagcttctgtttacttttcttacacccatcaccggcatatcattgtactcggcaggggc |
| 55 | Ckb | aagtccaagaactatgagttcatgtggaatcctcacctgggctacatcctcacatgcccatccaa |

[Table 1-2-1]

| | | |
|---|---|---|
| 56 | Cldn5 | cagactacaggcacttttaagaacttgaccgaccttttcttctatgcgcagttggccacgacgtg |
| 57 | Clip1 | gggaagacctgaacagttatgacagtgatgaccaggaaaagcagtccaagaagaaaccccgcctc |
| 58 | Cnga1 | ctcctgcaaacgcgatttgcccgtatcttggctgaatatgagtcgatgcagcagaaactcaagca |
| 59 | Cnga3 | ccaaacgtgtagtccttcccatatttattgaggtgaaggtcctgctctgttacccagtccagcct |
| 60 | Cntf | ctccgtgtcatttcttctcatcacatgggaatctcagcacatgagagccattatggggccaagca |
| 61 | Col7a1 | cctgaagatgacgatgacttctctgagtactctgtgtattctgtggaggactaccaggagcctga |
| 62 | Col8a2 | ttccccaggtcaaagccactaatccaatgggttctggcggtaagagettagtcaactcccagagg |
| 63 | Crx | ctagcctatcaggtatcccttcoctggctacccagagtgaaactgattaaaaatgtggatcccac |
| 64 | Ctnna1 | attaacttcttggtcacatttctgagtaggctgaagtgcctgctttctaggtaggggtgagcgcc |
| 65 | Ctss | tctacaaaagcggtgtctatgacgacccctcctgtacgggcaatgtgaatcatggtgttcttgtg |
| 66 | Cxcl1 | ctgctctgatggcaccgtctggtgaacgctggcttctgacaacactatacaatttcttttgaggg |
| 67 | Cxcl12 | ggagccagaccatcctggataatgtgagaacatgcctagatttacccacaaaacacaagtctgag |
| 68 | Cxcl2 | ggatttcaatgtaatgttgtgagtaacccttggacattttatgtcttcctcgtcaaggcacagtgc |
| 69 | Cyb5r1 | tgcgggaggacctagaggaacttcaggcccagtatcctaatcgctttaagctctggttcactctg |
| 70 | Darc | cctccctacaagacaggcttctcaaatggatgcccttgcaggcaagtcctagttttgttttgttt |
| 71 | Doc2b | gccctccaccaccgttagccaatccctgaacagtcccgtcttccaacccagagtcagtgacctcc |
| 72 | Edn2 | aggacacaattacctaagccacacctggagactgaacagtcatcctaatgactgacatcatggct |
| 73 | Ednrb | gaatccacgtgacacatgactctctttaggegtcacccacagttcttgtgtgtacagattgcttt |
| 74 | Efemp1 | gtctacaaatacatgagcatccgatctgacaggtccgtgccttcagacatcttccagatacaggc |
| 75 | Efna5 | ttcttgacaccgcattgaagccactcacctgtgtgcgtctgggtatgaagtccagctccttccgt |
| 76 | Egf | aaccaggctgatgatggtagagtgctacagacttggtactccagtttccacggctaatcactgct |
| 77 | Elovl4 | ccagaccccttcactccctgctctccgatttcagcacaataaatacactacagctgtgggaaaag |

(continued)

| | | |
|---|---|---|
| 78 | Eno3 | gttccgtaatccaaaggccaaatgaggagctggagactccaggctttcacaggaaagacacaggc |
| 79 | Erap1 | ggggcatggggcaacagtggaaacctgctgataccagtcatggtagtacttgtcattttcactga |
| 80 | Fgf16 | agtgatataggtgcagatcaatggtgaagtgcttcttagcatgggacgagccctagattccatcc |
| 81 | Fgf7 | acctatgcatcagctaaatggacacacagcggaggggaaatgttcgttgccttaaatcaaaaggg |
| 82 | Fgfr1 | tcaattctgccacctgctggttgctttggtaccttggtctcttattcaaacccacaccactcaag |
| 83 | Flt1 | ctctttggggtctagagatgagccctgggtctctaaaatggctctcttagaagttgtatgtgcaa |
| 84 | Gabrb3 | gtgacatgtccatctgcagtattgtgtgttttgatgccaacacggggacatcttcagttgttccc |

[Table 1-2-2]

| | | |
|---|---|---|
| 85 | Gem | gaaggagaagggctcactgcatacttatatccctgaaacgacatctttagagctggcctcatcac |
| 86 | Gfap | cttcacttcagtgctgattcagcccagagggttagttagttccctctggacggctgctcttgtag |
| 87 | Glut1 | ggattcgcccattcctgtctcttcctacccaaccactcaattaatctttccttgcctgagaccag |
| 88 | Gnao1 | cggtgaggtttctgtgacacctgacactgccagcctacttctctaatggattcctgtgtagctca |
| 89 | Gnat1 | gggtggctcagagcagagtccaacccctattgatcaaccatgggcaaagaatatcccatgtgagg |
| 90 | Gnat2 | gccagcgaccctaacgaccaggttccaaggctggttctgtttagtataagaacagaaaagcttca |
| 91 | Gngt2 | cctcctcagccacgcccaagtgtactgggatacctaaggaccaatgttttcttcctcttagacac |
| 92 | Gpnmb | ccaagtgaccctggtaagggaactgtotgcagaatggaagaaatagcctaagagacagggatggc |
| 93 | Gpr143 | caaaaccccaggaaggttgtatgtgtcgggggacatacttctgatgaggtgctgagcattttgtc |
| 94 | Grem2 | gtgggatggctctcacctccagtctatgggaacttcaagcctttattgaaacacccaccagctga |
| 95 | Grm2 | gagtctctgcattggccagtaactatccttgtagctatgccccgtgtttgccaggcctgggaatc |
| 96 | Grm6 | tttcccatggtgcagagcaccggtctacatttactgccgagtgcgatccaatgctctggactgag |
| 97 | Gsk3a | ggagtagagagagtccctggtgtcttagtttccacagtaaggtttgcctgtgtacagacctctgt |
| 98 | Gsk3b | ggtcagtttcacagggttatgccattttattcaagtccgttgctccgttgtgcagtctccaccat |
| 99 | Guca1a | tggagcgactcatccatgagtgccgaggaattcacagataccgtgtttgccaagatcgacatcaa |
| 100 | Guk1 | tgcttcattccacagagtgatgtctgtggtctaaatttgcctggaggtgggggaaactttgccag |
| 101 | H2-K1 | tcactggagctgtggtggcttttgtgatgaagatgagaaggagaaacacaggtggaaaaggaggg |
| 102 | Hfe | caggacccactcaactatcctccatctgttatagagtgactcctctgtcaccatgccctgacttc |
| 103 | Hif1a | atctgttcccattagcaggtgaaggaagctagggctgaaacaagagttttccgcgctctcaggga |
| 104 | Hspa1a | gtgcctgttgtgtttggaggtcaggegttgctgtgtatgacagtttcggctacgattgactaaca |
| 105 | Hspa1b | tatcagtgttccagtagcctgggaagacatatagtctagctgcccagttccctggagatggtcat |
| 106 | Hspa2 | cgtatgtacatggagatttgcttgaaagtagaaccctgatgctcgcacacctgacctgtggaagc |
| 107 | Icam1 | ctacttttgttcccaatgtcagccaccatgccttagcagctgaacaatcgagcctcatgctcatg |
| 108 | Id3 | ttgctgctttaggtgtctcttttcctccctctctatctctactctccaacatgaaggcgctgagc |
| 109 | Ifna1 | ccaggaagatgccctgctggctgtgaggaaatacttccacaggatcactgtgtacctgagagaga |
| 110 | Ifnr | agctggctgcactccactagaattgccacgacgtacctatcacagtgacgtctacagtgagaaat |

[Table 1-3-1]

| | | |
|---|---|---|
| 111 | Igf1 | ggctccatcccatatctcctatcggtgtctctgtatccttaaaccttgcaaacatcatacagtgta |

(continued)

| | | |
|---|---|---|
| 112 | Igf1r | tttctaagccagtgaggttgaggtgagaggtttgccagagtttgtctacctctgggtatcccttt |
| 113 | Igfbp3 | cagcatcggactccacgttcagagatgtcgcaaataatggtgcgcttagttcttcagatgacttc |
| 114 | Il10 | ctgatccagggatcttagctaacggaaacaactccttggaaaacctcgtttgtacctctctccga |
| 115 | Il17a | ttcctccagaatgtgaaggtcaacctcaaagtctttaactcccttggcgcaaaagtgagctccag |
| 116 | Il1a | ggaacatccttaaatcctctgagcttgacaggcatcctcacagcaggattttctaggtggtcagt |
| 117 | Il1b | cattaggcagcactctctagaacagaacctagctgtcaacgtgtgggggatgaattggtcatagc |
| 118 | Il6 | atctactcggcaaacctagtgcgttatgcctaagcatatcagtttgtggacattcctcactgtgg |
| 119 | Irf6 | gtagataccaagtccccaggagctctaagtttcccaatgctcccattcactctccagctgtcttc |
| 120 | Irs1 | ggtgctatcgacggtgctacttcatctgtgtacaaaagaccgacgcatggtctatgttgctacca |
| 121 | Isgf3g | ttgactacttggtcctcagtggaaccacttgggatgactagaggtgtggggttgttggttgagtt |
| 122 | Itgav | ttgccgccttacgagaacagtgtgagtttctgtagtgagtctaaccccgaggagggaatttagtg |
| 123 | Jak3 | aaagccccatcttttggtatgccccggagtccctatctgacaacatcttctcccgccaatctgac |
| 124 | Kdr | tggtctcactaccagttaaagcaaaagactttcaaacagtggctctgtcctccaagaagtggcaa |
| 125 | Lgals3 | gccttttaaactttgtgtgttgtgtgtctgtgcacatgggtacaggtgcctgctcacttgagagg |
| 126 | Lipc | tgacctccagcttcacccgagccaggagaaagtctttgtgaactgtgaagtgaagtcaaaaagac |
| 127 | Lmo1 | actatgaggaggggcatctcaatggcacctttgaatcccaggttcagteacgcccaccatctggc |
| 128 | Loxl1 | gtgggaaaggtctaccttcccaaagagagaagacacatgaattcttggcagaggggatagcatgg |
| 129 | Mark2 | tatttctctgctgtttcagttcttgagaaattggggaagtcctacagaggctgcccctgccctca |
| 130 | Math5 | tacgagacactgcagatggcgctcagctacatcatcgcgctcacccgcatcctagccgaagccga |
| 131 | Mef2c | ccgcctctgccatctgctacggtgtcgtcagttgtatggcattaatttcctgccactagagatat |
| 132 | Mkks | gcttctccaagttgttccaagaaagggtgccctgtacctttccactggcaggacattaccattgt |
| 133 | Mmp14 | cctagttggctgcctcccgccactctgactaaaaggaatcttaagagtgtacatttggaggtgga |
| 134 | Mmp2 | cctcctctgtagttaaccagccttctccttcacctggtgacttcagatttaagagggtggcttct |
| 135 | Mmp8 | actggagggctgtatctataaatctatttgccaataagttcccaggcagaggcaggtaggagggg |
| 136 | Mmp9 | gggcgcggctccaaccgctgcateaatattaaggtattcagttgcccctactggaaggtattatg |
| 137 | Msr1 | agccaatgattttgggagtctgtagtatgaatggactagttattctgtgaaaacatctagagaaa |
| 138 | Nes | tgggccagcactcttagctttgataacttgacctgtggtatctctcgtggagaggtgtggctggc |

[Table 1-3-2]

| | | |
|---|---|---|
| 139 | Nfkb1 | ccgaatttggcgtocttcttggttctgaaatgaaatgtagttgccacgcacagacggtgtctagc |
| 140 | Nos3 | atcctgctgccctcttcatatgctttgatggactgtagactccctttaactctctctctggagta |
| 141 | Np | ggacccaacatttaagggggaaatgtgggtgagaggaaagtggagtactctcagctaacacacag |
| 142 | Nr2e3 | ggggcagcacatcttagaagctaaatagttccctgcctttctcagccagtaattccacattcagg |
| 143 | Nrl | gagttgggagtagccacctcatcagctactgtcatctgtcctctaaggggcataataaatgggag |
| 144 | Nt5e | gactcaaatcocacacaaccactgtaaggcatactcaggtcaagacatgagaagaccagcaggac |
| 145 | Opa1 | gtgaccaaatggaaagggttggtgtggaagcaagtgagacttcctaactgtacagcgtgggatgt |
| 146 | Opt1mw | gcctccaacctgtcccatccatcaaagctttggccatgttttacctcccttctcatctatccttg |
| 147 | Opn1sw | aaggagcccctcgaaacgaagaagtgcgtgttgagttatacgcaaggagtgctgggtactgatat |

(continued)

| | | |
|---|---|---|
| 148 | Osbpl1a | ctaacccctacagtggagcacaggactggatttattctggcagctactgggacagaaactacttc |
| 149 | Pax6 | acacaggctgttggatcgcggatctgtgttgctcatgtggttgtttaaaggaaaccatgatcgac |
| 150 | Pde6a | ccttcaggggtttcctcagctgcaggatattctgcatttttcagggggatctatgcctgagatgg |
| 151 | Pde6b | aagaagaaggaagaagacagagtcgcagccaagaaagtgggcacagaagtttgcaatggtggtcc |
| 152 | Pdgfb | aacactgctgtccacatgacctccatttcccaaagtcctctgctccagcaactgcccttccaggt |
| 153 | Pdpn | gttgctgggacagagctgtagtgtcttggtcgtctgtgttggtacccaatacagtgtagacatct |
| 154 | Pecam1 | tttattccccactaaagaaacggtttcctaaggtctgagctgtttcccagggtgggctagagtgg |
| 155 | Pex1 | taccggagccaaagtggagaggatgaatcccttaaccagcctggaccaatcaaaaccacttttgc |
| 156 | Pgf | caccggttgtctctgccgggactaactgccaagccagattctcttgaataaagcattctagtctg |
| 157 | Pig7 | catgctggttaagatgcagcctagccattgactgagctgttcatacaatgatccggaagtgctag |
| 158 | Pkia | gcctggaaatgttctttcactgacttgtggcagcctgggaagtcatctgtgagcttctatgactt |
| 159 | Polg2 | aacttcacccttgtttagcccctattaaggtcgctttggatgtggggaaaggcccaacggtagaa |
| 160 | Ppara | gatgaagagggctgagcgtaggtaatgcgggctctccccacatcctttctgaatggcacttcta |
| 161 | Prkca | cctctactctccgtttgcatgatccgctctgttagatgcattcattgtagttcggaagcaagcgt |
| 162 | Prnp | gcgtgcactcagttccgtaggattccaaagcagacccctagctggtctttgaatctgcatgtact |
| 163 | Prom1 | aacaagggatcttgagagaaggaactgtcgctcagctgggagcggaatcattatcgcaatcacag |
| 164 | Ptgds | cctcaatctcacctctaccttcctcaggaaaaaccagtgtgagaccaagatcatggtactgcagc |
| 165 | Pxmp3 | ggcaagagagttctggagactgtgggacagctaggatgaagaactctatgttataagcctctgtc |

[Table 1-4-1]

| | | |
|---|---|---|
| 166 | Pygm | tacaaagtccacatcaaccccaactcgctctttgacgtccaggtgaaaaggattcatgagtacaa |
| 167 | Rcv1 | gaggaggaattcatcgaggggaccctggccaataaggaaattctgcgactgatccagtttgaacc |
| 168 | Rdh9 | ttgtgtgtacctgtatggtacatgtgtgggagagccttcagagatgggaaggtgtggagagccgt |
| 169 | Ren | gcaagttctatacagagtttgatcggcataacaatcgcattggattcgccttggcccgctaaggc |
| 170 | Rho | cctgcccttgaggggattatatgagatttaagggacttatgtggccagcctacttcctggcatgc |
| 171 | Robo4 | cgcagggaaacagggeaccaatgcgctattctcattctaccgccactctgagcttaaggaactta |
| 172 | Rom 1 | aattcgttactcagactcagggaaggaaaggtccctggggttataggagttaagagcaagcggag |
| 173 | Rpe65 | agggcaaaagcctgcatatctcctggttctgaatgccaaagacttgagtgaaattgccagggctg |
| 174 | Rs1 | gggctttgacaccctcctcctcgctgcccgaaagacagtattctggctacctttggatcaagata |
| 175 | Rxrg | ggtggagttttgtacggctgttaaaggtggcccttctttgctatttaaggggctgaggtctttcc |
| 176 | S100a6 | ggctgatggatgatctggaccgtaacaaggatcaggaagtaaecttccaggagtatgtcgccttc |
| 177 | Sag | gccttgagggaagcccacggtagactctcaaagttatgctagatatcanagcatgagcattcctc |
| 178 | Scd1 | caacccaagaaactcctgggctaagtatctgacagtctcacatctcaacagtgtgaattaagtgt |
| 179 | Sdc2 | ggcccttccasagctgcacaatgcccctcaattacggaggctgagaatgtagtgggtatacctct |
| 180 | Selenbp1 | atgaccgcttcctttacttcagcaactggctgcatggggacattcggcagtatgacatctctaac |
| 181 | Selenbp2 | atgaccgcttcctttacttcagcaactggctgcatggggacattcggcagtatgacatctctaac |
| 182 | Serpina3n | ttatagccaagatagccaaccccaaatgagactagaactccccaagtgttgacgcttcttcccgg |
| 183 | Serpinf1 | gaatcctggaccccagtagtacttaatgtctcagtgctctacagaacccccagagggaagctgat |

(continued)

| | | |
|---|---|---|
| 184 | Serping1 | ttcctcttcctgctctgggaccagcaacacaggttcccagtcttcatgggtcgtgtatatgaccc |
| 185 | Sfrp5 | tcacggccgtctaccgctgggacaagaagaataaggagatgaagtttgcagtcaaattcatgttc |
| 186 | Sil1 | acctgctggggcatgagtgtcacctgggcagagcttgctaggtcattaaacagagacatgatgac |
| 187 | Slc16a1 | ccttgtccgtcactcctttgaatgctgattttgtctcaagtcctttcagttcacatagccgtggc |
| 188 | Slc16a4 | ccactggctgaaagatggaaacgcaaacagtctgaccttctgaggactacaatcaagtaagagcc |
| 189 | Slc1a3 | cacttcctcttcagtccctaatcagccccaaagaaacgacgtcataacaccacttcctcctcat |
| 190 | Socs3 | aggagactcctgagttaacactgggaagacattggccagtcctagtcatctctcggtcagtaggt |
| 191 | Sox9 | ctgctcagactatcacctgtacctccctgaataccagcgtttaaccttcaagacatcccatgtgc |
| 192 | Sparc | cacctctccccaccacctgccacttgaaaccttctactaatcaagagaaacttccaagcaaacgg |
| 193 | Spp1 | caacaacggaaagggcagccatgagtcaagtcagctggatgaaccaagtctggaaacacacagac |
| 194 | Stat1 | tcaccgttttgagggatgatgttttgtggcacgtgtgtgatcacagcctgatggttctggtcgtg |

[Table 1-4-2]

| | | |
|---|---|---|
| 195 | Stat3 | gaacttataaactgaaagggtatttaggaaggcaaggcttgggcatttttatggctttcaatcct |
| 196 | Stat5a | aggtttaggcaactaagttggagttttactcctaagctagaagcttcgcccagaccggtgtgctc |
| 197 | Stat6 | ctcatgcaggtgccttccgtctcsactgttccttggttaagagaaaagaactggctgggagacca |
| 198 | Synpr | gtccaatgaccctgtgcttgtagagtggcgttttctttgcatccagagaggcagatttagacacc |
| 199 | Tgfb2 | agctacctgggtccattcctcccccaaccccagttccttctattttccaaaagataaaaaccaaa |
| 200 | Tgfb3 | aatcatatcttgcactgcctggaattaaggacaatccgttctttctgcaactgtcttttcacctc |
| 201 | Tgfbr2 | gaatgctggtccactagtgggatttctagggttcaaaagtgacttcacttccgggtcatcatcag |
| 202 | Timp1 | gtgaagagtttctcatcacgggccgcctaaggaacgggaaatttcacatcaatgcctgcagcttc |
| 203 | Timp2 | cctccctcccttactcccgtcatgccagcaactcgcaatatttcagatgacgtttacatggtagc |
| 204 | Timp3 | tagatctaagtcagctgtttgggttgaggaggagagaacccgaggaaatgaccatgctctgggga |
| 205 | Tlr4 | ggggaggaagaaaggtctaacatccttttccttcatcattctcatttctggacatgccttgtgag |
| 206 | Trip1 | aagctcttcgactccacgactcttgaacatcagaagactttccgaacagagcgtcctgtcaactc |
| 207 | Ttpa | ccttcagtgtctttgctagatcaagtgcagacgctgcacacaatctctagttcctctagttctgg |
| 208 | Tyrp1 | cgaatggataaggaggtataacgccgatatttctaccttcccgttggaaaacgcacctattggac |
| 209 | Usp9x | gtttgcagaagtatgtagcacttgtcctccaagctttcaggtgtaagggggaaaggtgaaccaca |
| 210 | Vcam1 | tgatcccttgctgaatgcaaggagctaaccagasaagttctgcttgacaagtccccatcgttgaa |
| 211 | Vegfa | ctagcttgtcctgagaagatatttaattttgctaacactcagctctgccctcccttgtccccacc |
| 212 | Vegfb | aacaattgtcaaggaacctcatgtctcacctcaggggccagggtactctctcacttaaccaccct |
| 213 | Vegfc | ccaagtctgtgtttattgaaccatgtggattactgcgggagaggactggcactcatgtgcaaaaa |
| 214 | Vegfd | gacaaatgacttgtagcttcagatgtctttgcgccatcagcactcagaaaggaaggggtctgagg |
| 215 | Vldlr | cgctgtcttagtactgtcactgttgtaaaggcacatgttggaacacatccagtcctgctgacctg |
| 216 | Vtn | gcgtctatttcttctctggagacaaatactaccgagtcaaccttagaacccggcgagtggactct |
| 217 | Actb | ccatcgtgcaccgcaagtgcttctaggcggactgttactgagctgcgttttacaccctttctttg |
| 218 | GAPDH | gggctgccatttgcagtggcaaagtggagattgttgccatcaacgaccccttcattgacctcaac |
| 219 | Arbp | atcagatgaggatatgggattcggtctcttcgactaatcccgccaaagcaaccaagtcagcctgc |

(continued)

| | | |
|---|---|---|
| 220 | YPL088W-7 | gcatgttgactcgtcctctgaaccaaagcacggacaggattaagagtgatccaactttcaagtcg |

[Table 1-5-1]

| | | |
|---|---|---|
| 221 | cxcr4 | gcaggacctgtggccaagttcttagtagctgtttatctgtgtgtaggactgtagaactgtagagg |
| 222 | esm1 | acttgattgatgaatggacctgagtttttacccggaggaccttagcacaagaagaactgttgtcc |
| 223 | apln | gtccctgtgcccacagattgcacgtgtagggaggtgagtgcttgtatcccaaattggttctaggt |
| 224 | egln3 | ccccaactttcaaacctctttaatttcctagctagaatcccacaagcagcatagacctgaaagtg |
| 225 | Cp | cctgctcttcgtgttctctgccctgctggagtacgccgccgtcaactttgtgtctcggcaacaca |
| 226 | Tnfa | ctgtcgctacatcactgaacctctgctccccacgggagccgtgactgtaatcgccctacgggtca |
| 227 | Tgfb1 | ccogtgcagagotgogcttgcagagattaaaatcaagtgtggagcaacatgtggaactctaccag |
| 228 | Il6r | aatcctctggaaccccacacaggtctctgttgaagactctgccaaccacgaggatcagtacgaaa |
| 229 | Tnfrsf1a | ttcaaggaccattctgctagatgccctactccctgtgggtgaaaagtgggcaaaggtctctaagg |
| 230 | Aoc3 | gctggtacacacatgactcaaaacaacagatacatcattccctagcagtctggccagccccgttg |
| 231 | Ptgs1 | gtcaaggcagtaaggtgttcttgggagccacacttagactctttccaaagatgtggagggaacag |
| 232 | Ptgs2 | ggctgttggaatttacgcataaagcagactgcatagatccaatattgactgacccaagcatgtta |
| 233 | Pla2g5 | Ictaggcttcttcaaatggagctccagaatgtgcacgggacacatccgatctatatttagagacct |
| 234 | Il6st | tgaacgttgcagtgtgaagtctgtactggctagggatggtagcccagcgtgagttcaggcatgaa |
| 235 | Nrp1 | aagcagcttccggctggtagatttttgtcttgatgggctgtccataagaactcccagttctttcc |
| 236 | Mapk1 | aaataaagtggcagcccttagacactgtgacggtcgtgtgcattgtgggagtggacttttatgga |
| 237 | Mapk3 | cctattcatcttgttggaaccccaccccattttccctgacagaacattcctaagtctcaagggct |
| 238 | Jun | ccctgtctgatttgtaggaatagataccctgcatgctatcattggctcatactctctcccccggc |

[Table 1-5-2]

| | | |
|---|---|---|
| 239 | Fos | aaacacgtcttccctcgaaggttcccgtcgacctagggaggaccttacctgttcgtgaaacacac |
| 240 | Tlr3 | ttttgtgcctcaaagttcggttggggctaccttgggatcccaaacagctaatatggtcaccaact |
| 241 | Ctgf | gtggggggcagtttatttgttgagagtgtgaccaaaagttacatgtttgcacctttctagttgaa |
| 242 | Epo | taggcgcggagatgggggtgcccgaacgtcccaccctgctgcttttactctccttgctactgatt |
| 243 | Ccr2 | gtagtgaatgaccaagaataaggagaaaagccaactccttcatcaggcatagagagctgcagcaa |
| 244 | Angpt-1 | cttgataaccgcagccacaaagccttagtgactttcctctacctggtaagacagagctcttcatg |
| 245 | Angpt-2 | cagaacacttagatggtgcagataaatcttgggaccacattcctetaagcacggtttctagagtg |
| 246 | Adipor2 | gcctccgggaactttccaggttggcacctgaatgccttactctcagcagtctgaggctcgcttgc |
| 247 | Adam28 | atagtgcacaaggatggtgggaataaatgaagcatcccacactcaacctcccaacatccataact |
| 248 | Adam17 | gctgtggcattgatgatacctgcctcagtcaataaatactgaatatcaaagcagtggattgcgta |
| 249 | Adam19 | agcctagcaccccaaagtcatgcacccagtatcctcttgtatgactgtatatgtctatgtctggg |
| 250 | Mmp1 | gcaagccagaataaagactgtgccagctggtcagtcgccccttttgagaccactcctttgtgctcc |
| 251 | Mmp7 | ggacgacattgcaggcattcagaagttatatggaaagaggaacacgctgtgatagatgcagacag |
| 252 | Selp | ccagatggtactaggggacacagaagatctggaacagagccaagacgtcacatctgactgcagac |
| 253 | Sele | gtcctggcactgaagccagcatgagatccatcattcttatgtcagctcaagggtcaaaaggactt |

(continued)

| | | |
|---|---|---|
| 254 | Furin | cccgatgctgctttcccctgtggggatctcaggagctgtttgaggatatattttcactttgtgat |
| N.C. | OmpA | gtgtcggcataagccgaagatatcggtagagttatattgagcagatcccccggtgaaggatttaa |

4. Microarray

A microarray is where numerous probes are immobilized densely but independent of each other on a carrier. An embodiment of the present invention provides a microarray with mounted probes to detect at least one type of a gene selected from the gene clusters above. Also, the embodiment provides a microarray to evaluate disorders in the periphery of a retina, disorders in the retina, light damage to the retina, inflammation in the retina, disorders in the retinal pigment epithelium or choroid, light damage to the retinal pigment epithelium or choroid, or inflammation in the retinal pigment epithelium or choroid. The microarray related to the present invention is not limited specifically as long as the probes of the embodiment (for example, oligonucleotide probes) are immobilized on a carrier. More specifically, the embodiments of the present invention provide a microarray with mounted probes having at least one type of base sequence shown in SEQ ID Nos. 1-291 and 221-254.

The carrier of a microarray is not limited to any specific shape, and a flat plate, a rod shape or beads may be used. When a flat plate is used as a carrier, predetermined probes are immobilized by type at certain intervals (for example, spotting methods or the like; refer to Science 270, 467~470 (1995), for example). In addition, predetermined probes may be synthesized by type at specific positions of the flat-plate carrier (photolithographic methods or the like; refer to Science 251, 767~773 (1991), for example).

Another preferred example of a carrier is one that uses hollow fibers. When hollow fibers are used as a carrier, oligonucleotide probes are immobilized by type in hollow fibers, and all the hollow fibers are gathered and immobilized. Then the fibers are cut repeatedly in a longitudinal direction to obtain a nucleic-acid microarray. Such a nucleic-acid microarray is described as a type with oligonucleotide probes immobilized in a substrate with through holes, and is referred to as a so-called "through-hole type microarray" (refer to Fig. 1 in Japanese Patent No. 3510882, for example).

The method for immobilizing probes on a carrier is not limited specifically, and any binding method may be employed. Also, it is not always necessary to immobilize probes directly on a carrier. For example, a carrier is coated in advance with a polymer such as polylysine, and probes may be immobilized on the coated carrier. Moreover, when a tubular body such as one having hollow fibers is used as a carrier, probes may be immobilized on a gelatinous material held in the tubular body.

In the following, producing a microarray is described in detail by referring to a through-hole type nucleic-acid microarray using hollow fibers. The microarray is prepared by the steps (i)~(iv) below.

(i) a step for producing an array by arranging multiple hollow fibers in a three-dimensional formation in such a way that the hollow fibers have the same longitudinal direction;
(ii) a step for producing a block body by embedding the array;
(iii) by introducing a gel precursor polymerizable solution containing an oligonucleotide probe into the hollow portion of each hollow fiber of the block body and initiating polymerization reactions, a step for holding the gelatinous material containing the oligonucleotide probe in the hollow portion;
(iv) a step for slicing the block body by cutting in a direction that intersects the longitudinal direction of the hollow fibers.

The material for the hollow fibers is not limited specifically, but the material described in JP2004-163211A or the like is preferred.

The hollow fibers are three-dimensionally arranged to have the same longitudinal lengths (step (i)). Such methods are, for example, arranging in parallel multiple hollow fibers at certain intervals on a sheet material such as an adhesive sheet, and having them set as a sheet, and then the sheet is rolled into a spiral shape (refer to JP H11-108928A); and laminating two porous plates each having multiple holes at certain intervals in such a way that those holes correspond to each other, passing hollow fibers through the holes, preliminarily fixing the two porous plates to have a distance between them, filling curable resin material around hollow fibers between two porous plates and curing the resin material (refer to JP2001-133453A).

The arrayed body is embedded so as not to disturb the array (step (ii)). Examples of such a method are filling polyurethane resin or epoxy resin into spaces among fibers, adhering fibers to each other by thermally fusing fibers, and the like.

In the hollow portion of each hollow fiber of the embedded array, a polymerizable gel precursor solution (gel-forming solution) containing a probe is filled and polymerization reactions are initiated in the hollowed portion (step (iii)). Accordingly, a gelatinous material with the immobilized probe is held in the hollowed portion of each hollow fiber.

A polymerizable gel precursor solution is a type of solution containing reactive substance such as gel forming polymerizable monomers, and when the monomer is polymerized or crosslinked, the solution becomes a gel. Examples of such monomers are acrylamide, dimethyl acrylamide, vinyl pyrrolidone, methylene bisacrylamide and the like. The solution may include a polymerization initiator or the like. After the probe is immobilized in the hollow fibers, the block body is thinly sliced by cutting in a direction that intersects the longitudinal direction of the hollow fibers (preferably at right angles) (step (iv)). The thinly sliced pieces obtained above are used as a nucleic-acid microarray. The thickness of the array is preferred to be approximately 0.01 to 1 mm. The block body may be cut by using a microtome, a laser or the like. Preferred examples of a through-hole microarray are a nucleic-acid microarray (Genopal) made by Mitsubishi Rayon Co., Ltd. and the like. In the evaluation method of the present embodiment, those microarrays may be used.

5. Eye Disease

In the embodiments of the present invention, "eye disease" means a disease associated with the eye. For example, diseases of the eyelid and lacrimal apparatus such as hordeolums, chalazions and neonatal dacryocystitis; diseases of the conjunctiva such as conjunctivitis and pterygium; diseases of the cornea such as corneal infections and corneal endothelial disorders; diseases of the uvea (iris, corpus ciliare, choroid) such as uveitis and Behcet’s disease; diseases of the retina such as diabetic retinopathy, retinal detachment, retinal vein occlusion, central serous chorioretinopathy, age-related macular degeneration and retinitis pigmentosa; cataracts, glaucoma, optic neuropathy or the like. However, those are not the only examples. In the present embodiment, preferred examples of an eye disease are disorders of the retina, retinal pigment epithelium and choroid, more preferably age-related macular degeneration.

The above eye diseases include disorders of the retinal periphery. "The retinal periphery" indicates portions that include the retina composed of retinal arteries, retinal veins, macula flava, optic disk and central fossa, and portions that include the retinal pigment epithelium, choroid and sclera. "Disorder" includes damage and dysfunctions observed in those portions. They include, for example, cell death, inflammation, angiogenesis, accumulated waste products containing protein and lipids, and dysfunction of cells with light-sensitive receptors. Light damage is one of the disorders caused by light, and includes cell death, inflammation and dysfunction of cells with light-sensitive receptors. Also, since angiogenesis is observed when oxygen deficiency occurs in the retina caused by bleeding, the above eye diseases include those associated with angiogenesis. Examples of eye diseases associated with angiogenesis are diabetic retinopathy, age-related macular degeneration and the like. The "condition" of an eye disease indicates a particular condition associated with the eye disease. Examples of such conditions include the disorders listed above.

6. Subject Organism

In the embodiments of the present invention, a "subject organism" indicates a target organism that contacts, ingests or is administered a test substance, and includes individual animals, animal tissues and animal cells (including cultured cells). The animals are preferred to be mammals; and the mammals are preferred to be rodents such as mice, rats, rabbits and hamsters, monkeys, hogs, cats, dogs, cows, horses and humans. However, when subject organisms are used for experimental purposes, they should be nonhuman mammals, preferably rodents. The cells used in the embodiments are not limited specifically, and examples are cell lines from mammals such as humans, mice and rats as well as cell lines derived from those cell lines, primary cell lines from various tissues, stem cells, ES cells, iPS cells, and the like. The cells to be used in the embodiments are preferred to be those related to the eyes. Examples are retinal cells (visual cells, nerve cells), pigment epithelial / choroid cells and the like.

7. Method for Evaluating Condition of Eye Disease in Subject Organism

The present invention relates to a method for evaluating the condition of an eye disease in the subject organism by detecting a particular gene in a sample taken from a subject organism and comparing the detection results with a control. The aforementioned microarrays may be used in the methods of the embodiments.

In the embodiments of the present invention, regarding a gene in a sample taken from a subject organism, the condition of an eye disease in the subject organism is evaluated by comparing the expression level of the gene of a normal subject organism (a control) and the expression level of the gene of a subject organism affected by the eye disease, and by checking to see if variations (increase or decrease) are observed in the gene expression level or if the gene itself is expressed or not.

Also, the same method may be used to evaluate disorders, and conditions of inflammation, angiogenesis and age-related macular degeneration in the retina and retinal pigment epithelium/choroid of the eye of the subject organism.

7-1 Sample Taken From Subject Organism

A sample taken from a subject organism means a bio-sample isolated or taken from the subject organism. Samples include part of the eye such as the retina, retinal pigment epithelium and choroid, bodily fluids such as blood, other tissues or parts of organs, homogenates, cell homogenates, or nucleic acids retrieved from such samples. As a sample for continuous measurement, cell homogenates and body fluids such as blood are preferred. When conditions of an eye disease are directly checked, the retina, retinal pigment epithelium and choroid are preferred.

In the embodiments of the present invention, a control means a measurement result obtained from a control organism to be compared with a measurement result obtained from a subject organism and to have a determination made accordingly. For example, a control to be compared with measurement results regarding the condition of an eye disease in a subject organism is the result obtained from a subject organism that is not affected by the eye disease. Also, a control in the method for evaluating inhibitory or restorative functions of a test substance for the eye disease is a measurement result in a subject organism that has not contacted, ingested or been administered the test substance.

7-2 Extraction of Nucleic Acids

The level of mRNA in a sample is measured by extracting nucleic acids from the sample. Extraction of nucleic acids and treatment of the extracted nucleic acids are conducted by using a method that is suitable for measuring the expression level of the gene.

Extraction of mRNA is conducted by the method below, for example; however, that is not the only option.

First, to an organ or cells frozen at -80°C or by using liquid nitrogen, a 4M guanidine thiocyanate solution is added to make a total amount of 0.5 g/10 mL, and homogenized. Then, 1 mL of 2M sodium acetate, 10 mL of phenol, and 10 mL of chloroform are added and stirred well. The mixture is centrifuged for 10 minutes at 10000 rpm, and the aqueous phase is recovered. Next, an equal volume of isopropanol is added to the mixture, further centrifuged for 10 minutes at 10000 rpm to precipitate RNA. The mixture is dissolved again in 10 mL of 4M guanidine thiocyanate solution, to which 1 mL of 2M sodium acetate, 5 mL of phenol, and 1 mL of chloroform are added and stirred well. The aqueous phase is recovered in the same manner as above, and an equal volume of isopropanol is added to precipitate RNA. To the precipitate, 20 mL of 70% ethanol is added to form a suspension, which is then centrifuged to precipitate RNA. The precipitate is dissolved in 5 mL of a TNES buffer (0.1M Tris-HCl (pH7,4), 50 mM NaCl, 10 mM EDTA, 0.2% SDS), and Proteinase K is added to make a total amount of 200 μg/mL. The mixture is reacted at 37°C for 30 minutes, extracted by using acidified phenol and chloroform, and the extract is precipitated with ethanol. As for other methods, a commercially available reagent kit such as an RNeasy mini kit (QIAGEN) may be used. In that case, the RNA is extracted according to the supplied protocol.

7-3 Measuring Nucleic Acids

In the analysis method using the microarray, the mRNA level of a gene is determined as follows: mRNA, cDNA or cRNA (aRNA) derived from a bio-sample is hybridized in a microarray in which probes capable of hybridizing with a particular gene are densely synthesized and immobilized on a chip. The aforementioned microarrays may be used in the present embodiment. Especially a nucleic-acid microarray manufactured by Mitsubishi Rayon Co., Ltd. (Genopal™) is preferred because it is capable of densely immobilizing oligonucleotide probes by using polymer gels.

In the nucleic-acid microarray made by Mitsubishi Rayon (Genopal™), the oligonucleotide probes complementary to each corresponding sequence are densely immobilized. Using a T7 oligo dT primer from the RNA derived from a bio-sample, the double-strand cDNA is synthesized, and then aRNA is synthesized by *in vitro Transcription.* Biotin is incorporated at the time of aRNA synthesis, and the sample is labeled. The biotin-labeled aRNA is fragmented and hybridized to a DNA array. After the hybridization, aRNA is detected by streptavidin or the like modified with a fluorescent dye. The fluorescence is identified using a fluorescence detector, the resulting array images are quantified, and the value is set as the expression level of mRNA. Moreover, nucleic acid microarrays to be used are not limited to the above, and the same analysis may also be conducted using several other nucleic-acid microarrays available for a person skilled in the art.

7-4 Analysis of Measurement Results

As data to be used to evaluate the measurement results, values of the expression level having at least a determination value are used. As the determination value, an average value X of a negative control may be used. In addition, a value obtained by adding the standard deviation $\sigma$ to X is preferred, more preferably a value obtained by adding $X + 2\sigma$, even more preferably a value obtained by adding $X + 3\sigma$. A negative control is a gene that should not be detected in the sample from the test organism. For example, a negative control is a gene of an organism which is a different species from the subject organism, and is a probe with a sequence that will not hybridize under stringent conditions with the sequence of a complementary strand mounted in other probes (N.C. in Table 1). The error between the data of the obtained samples is corrected by the expression level of housekeeping genes (gapdh, actin, arbp, etc.). Variations in the expression level are determined by the corrected data. Variations in the expression level are statistically determined through calibration. From each subject organism, at least n=3 samples are acquired, and t-calibration is carried out. If value P is 0.05 or less, or if value P is even lower, such as 0.01 or less, it is determined that the level shows significant variations (increase or decrease).

7-5. Evaluation of Condition of Eye Disease

In the embodiments of the present invention, to assess the condition of an eye disease, the gene expression data in a subject organism that is not affected by the eye disease are compared with the gene expression data in a subject organism that is affected by the eye disease. The gene expression data to be compared may be obtained from the same subject organism or from multiple different subject organisms. Alternatively, data stored previously in a database may also be used. In the present embodiment, the expression level may be measured by using samples derived from multiple subject organisms. Thus, the expression level is measured in a predetermined number of test organisms (primary population), which is set as basic data values so as to be compared with the expression level obtained from the samples of one or more subject organisms. Making comparisons with a control not only includes comparing the increase or decrease in expression level, but also includes comparing the presence or absence of values. For example, it includes comparing samples regarding a gene that is not expressed in the basic data but is expressed in a subject organism.

Also, data on expression levels are processed again (to obtain average value, etc.) by incorporating the data from the subject organism into the values of the primary population so that the number of cases in the subject organisms (primary population) is increased. By increasing the number of cases, accuracy in the critical value of the expression level is improved, and detection accuracy is also improved by properly modifying the critical value.

Furthermore, a database is created by storing the measurement results of gene expression levels of the same type of subject organisms so that changes in the gene expression levels may be evaluated as a pattern. Such a method can be carried out by using a multivariate analysis, for example, principal component analysis, factor analysis, discriminant analysis, quantification theory (type I, type II, type III, type IV), cluster analysis, multi-dimensional scaling method (MDS), multiple regression analysis, conjoint analysis, comparison of the pattern using the Mahalanobis-Taguchi system (MT method), and a prediction of the effects.

The embodiments of the present invention are also capable of providing a database for storing such data as well as providing an analysis device that can read data and a program necessary for comparative analysis to execute analysis. Using such an analysis device, the condition of the eye disease in a subject organism is evaluated by a simplified method such as retrieving the stored data from the database and comparing the stored data with the measured data obtained from the subject organism.

In the embodiments of the present invention, the following formula is used to evaluate the condition of an eye disease.

By using the following formula, variations in the gene expression and the condition of a disease are determined quantitatively. Thus, it is possible to evaluate whether the condition of retinal epithelial cells and choroid is normal or not, and furthermore, what is the condition of the disease. Namely, by using the following formula, variations in the gene expression level and the condition of a disease can be associated.
determination formula

$$M=(Xli\times\eta 1 / \beta 1+ \cdots + Xki\times\eta k / \beta k) (/\eta 1+ \cdots + \eta k )$$

When M1 max + $\sigma$ m1 <M2 min is satisfied, the condition of the evaluation sample cluster is the same as that in each disease model.

In the formula, "M" represents the Mahalanobis distance to show the distance from the base space, "Xni (n=1~k)" is input data for each sample and represents the gene expression level or gene expression ratio, and "$\eta$ (n=1~k)" indicates "$\eta$=(S$\beta$ -Ve)/Ve/r, representing the (S/N) ratio in each sample. Each term is as follows: S$\beta$=L2/r; variation in a proportional term, Se=ST-S$\beta$; error variation, Ve=Se(/1-1); error variance, ST=X2i1+X2i2+··· +X2il; total variation.

"$\beta$n (n=1~k)" indicates $\beta$=L/r, representing the sensitivity of each sample. Each term is as follows: L=M1Xi1+M2Xi2+··· + M1Xi1; linear equation, r = M21 + M22 + ··· + M21; valid divisor.

"M1" represents a normal sample cluster or a control cluster. "M1 max" indicates the maximum value of "M1" and "$\sigma$ m1" represents standard deviation from "M1". "M2" represents an evaluation sample cluster, and "M2 min" indicates the minimum value of "M2".

8. Method for Evaluating Inhibitory or Restorative Function of Test Substance for Eye Disease

The present invention relates to a method for evaluating inhibitory or restorative functions of a test substance on an eye disease in a subject organism by detecting a particular gene that is present in a sample taken from the subject organism that has contacted, ingested or been administered the test substance, and by comparing the detection results with a control result. The method related to the present invention can be carried out by using the above-described microarrays.

In the present invention, to evaluate the inhibitory functions of a test substance on a gene included in the gene clusters, the gene expression level obtained from a subject organism that has contacted, ingested or been administered the test substance in advance and is affected by an eye disease is compared with the gene expression level obtained from a subject organism that has not contacted, ingested or been administered any test substance and is affected by the eye disease.

In addition, to evaluate the restorative effects of a test substance on a gene included in the gene clusters, the gene expression level obtained from a disease-affected subject organism after it contacts, ingests or is administered the test substance is compared with the gene expression level obtained from a disease-affected subject organism that contacts, ingests or is administered no test substance.

Furthermore, inhibitory or restorative functions of a test substance on disorders of the retina and retinal pigment epithelium and choroid, and on inflammatory conditions and angiogenesis, may be evaluated in the same manner as above, and inhibitory or restorative functions of a test substance on age-related macular degeneration may also be evaluated.

In the embodiments of the present invention, contact, ingestion or administration means local administration (to the eye), oral administration, intraperitoneal administration or intravenous administration, if the subject is an animal. If the subject is cells, the test substance is added to the culture liquid and the cells are cultivated accordingly.

In the embodiments of the present invention, a "test substance" means a substance that a subject organism contacts, ingests or is administered, including food and drugs.

Food means all food and drinks - fresh food, processed food, beverages, condiments, processing materials such as food additives, homogenized plants, extracts from plants, mixtures thereof, food ingredients and the like. Pharmaceuticals include drugs, quasi-drugs, drug candidates, drug mixtures, cosmetics, cosmetic ingredients, fragrances, coloring agents and the like.

The concentration level when a test substance is added or the concentration level for a test substance to be ingested can be selected at any concentration, but it is preferred to be carried out at a concentration that does not cause toxicity in the subject organism so as to avoid any impact for extraction of nucleic acids. No toxicity indicates, in the case of animals, that death, partial necrosis, inflammation and the like are not observed; in the case of cells, release of inflammatory cytokines is not observed, or the cell survival rate is 90% or greater.

In the present invention, if the subject organism is a cell, the concentration of cells is not limited specifically, but it is preferred to be such a level at which sufficient nucleic acids are collected at the time of cell harvest, that is, final concentration of at least $1.0\times10^{5}$ cells/well, more preferably a final concentration of at least $5.0\times10^{5}$ cells/well.

The inhibitory or restorative functions of a test substance for an eye disease are evaluated by measuring variations in the expression level of each gene among a sample taken from a subject organism that has contacted, ingested or been administered the test substance, and a sample taken from a subject organism before it contacts, ingests or is administered the test substance or a sample taken from a subject organism that has contacted, ingested or been administered a blank (placebo or substance containing only a solvent).

Data pertaining to which gene of which group showed a change in expression level are analyzed, and the analysis results are used to determine the effects of a test substance on inflammation in the retina, angiogenesis or the like. If the test substance is observed to be effective, effects that can be expected from the test substance are also evaluated and predicted. It is preferred to create a database from the measurement results of the known effects of the components or the known conditions of a mouse model by using the same type of cells or animals so that changes in the gene expression levels are evaluated as a pattern. Such methods include those using a multivariate analysis, as described above.

Association with the detection results of a gene and functions (effects) of a test substance can be carried out as follows. The gene detection results are compared with a control, and if a change is observed in a gene expression level only when a subject organism has contacted, ingested or been administered the test substance, the test substance is determined to have been effective on the gene and to have had inhibitory or restorative effects on the condition of an eye disease.

Alternatively, an MT method as a multivariate analysis is carried out as follows: the control data are set as a base space, and the data from a subject organism affected by an eye disease are set as a signal space, and the distance with the base space is shown by the Mahalanobis distance to determine the value (the distance set as a determination base). Then, the data from a subject organism that has contacted, ingested or been administered a test substance is set as another signal space and the distance from the base space is shown by the Mahalanobis distance. When it is below the determination value described above, the test substance is determined to have inhibitory or restorative effects on the condition of the eye disease (reference: Basic Offline Quality Engineering, Genichi Taguchi, Yoshiko Yokoyama, Japanese Standards Association).

For such determination, the determination formula described above in "7-5 Evaluation of Conditions of Eye Disease" may be used.

By using the method related to the present invention, a substance (food, drugs, etc.) having inhibitory or restorative functions on the condition of an eye disease may be screened.

In the following, examples of the present invention are described in detail. However, the present invention is not limited to those examples.

## EXAMPLE 1

### 1. Nucleic-acid microarray

Example 1 was conducted by using a nucleic-acid microarray (Genopal™, Mitsubishi Rayon Co., Ltd.) that mounts the probes described in Table 1 (SEQ ID Nos.: 1-254).

### 2. Mouse Model with Eye Disease

As examples of a mouse model with an eye disease, the present example uses the following: a mouse with choroidal neovascularization caused by irradiating a laser to induce angiogenesis (CNV mouse); and a mouse with inflammation induced using lipopolysaccharide (LPS) as stimulus (LPS mouse). Information pertaining to each mouse is shown in Table 2 below. The "ID term" column of Table 2 shows the same as those in Figs. 2 and 3 and Table 3.

[Table 2]

| animal | | | |
|---|---|---|---|
| ID term | animal | age in weeks | condition |
| CTR | C57BL/6 mouse | 8-10 weeks | normal |
| LPS | C57BL/6 mouse | 8-10 weeks | LPS stimulus: 6 hrs after intraperitoneal administration of LPS 0.2 mg (PBS (-) solution) |
| CNV | C57BL/6 mouse | 8-10 weeks | CNV model: 1 week after inducing angiogenesis by 10 μs irradiation of laser (wavelength 532 nm, output 100mW) |
| | | | |
| site | | | |
| ID term | site | | |
| Retina | retina | | |
| R/C | retinal pigment epith elium/choroic | | |

### 3. Preparation of Sample Solution

Total RNA was extracted from the retina and pigment epithelium/choroid samples taken from the mice model above. Table 3 shows the measured sample groups.

[Table 3]

| measurement sample (total 8 groups) | | | |
|---|---|---|---|
| sample group | ID term | content | # of samples |
| 1 | CTR_Retina | retina of normal mouse | 6 |
| 2 | CTR_R/C | retinal pigment epithelium/choroid of normal mouse | 6 |
| 3 | LPS_Retina | retina of LPS mouse | 5 |
| 4 | LPS_R/C | retinal pigment epithelium/choroid of LPS mouse | 5 |
| 5 | CNV_Retina | retina of CNV mouse model | 5 |
| 6 | LPS_R/C | retinal pigment epithelium/choroid of CNV mouse model | 5 |

Using a MessageAmp II-Biotin Enhanced aRNA Amplification Kit (Applied Biosystems), aRNA was prepared from 1 μg of the extracted total RNA according to the attached protocol. Then, 5 μg of the obtained aRNA was placed in a plastic tube, 4 μL of 5x Array Fragmentation Buffer included in MessageAmpII-Biotin Enhanced Kit (Applied Biosystems) was added and diluted to the total of 20 μL. The mixture was stirred well, and heated at 94°C for 7.5 minutes for fragmentation. A sample solution to be reacted with Genopal was prepared by mixing 24 μL of 1M Tris-HCl solution (Invitrogen), 24 μL of 1M NaCl solution (nacalai tesque) and 20 μL of 0.5% Tween 20 solution, respectively, and diluted to 180 μL by using Nuclease-free water. Then, 20 μL of the solution after fragmentation was mixed to prepare a sample solution.

3. Measurement

The nucleic-acid microarray (hereinafter also referred to as a "DNA chip") was immersed in the sample solution, and hybridization reactions were conducted at 65°C for 16 hours. After removing the sample solution used for the hybridization from the DNA chip, the DNA chip was immersed in a 65°C 0.12M TNT solution (0.12M Tris-HCl, 0.12M NaCl, 0.5% Tween 20 solution) (for 20 minutes 2 times), then immersed in a 0.12M TN solution (0.12M Tris-HCl, 0.12M NaCl) heated to 65°C for 10 minutes, and washed. To detect a signal in the DNA chip, a DNA chip detector device (MB-M3A, manufactured by Yokogawa Electric Corp.: laser wavelength: 633 nm) was used to measure the fluorescence intensity of Cy5 (exposure time: 0.1 sec., 1 sec., 4 sec., 40 sec.).

4. Data analysis

Data were analyzed as follows:

(i) subtract the average value of the background;
(ii) correct the values by using a positive control gene (β-actin, GAPDH, RPLP0);
(iii) calculate the mean value, the expression ratio and the P value after correction (expression ratio is shown based on the CTR).
(iv) pick a gene with a significant change (a P value of 0.05 or less); and
(v) create a determination formula by using a bilateral T method which is one of the MT methods used to assign gene weights.

5. Results

Measurement results of a retina site (Retina) are shown in Figure 2A-L, and measurement results of the retinal pigment epithelium and choroid site (R/C) are shown in Figure 3A-M. Also, genes that show significant variations in expression levels in each measurement sample are shown in Table 4 below. Table 4 shows gene clusters that show changes in expression levels in CNV mice model and gene clusters that show changes in expression levels in LPS mice, respectively. Genes are listed in Table 5 in the order from a gene that showed greater contribution to a gene that showed less contribution to the calculation with respect to a normal mouse.
<u>Table 4</u>

[Table 4-1-1]

| gene clusters retina | | | |
|---|---|---|---|
| group i | | group ii | |
| significant change in CNV | | significant change in LPS | |
| probe # | symbol | probe # | symbol |
| 97 | Gsk3a | 101 | H2-K1 |
| 93 | Gpr143 | 118 | Il6 |
| 173 | Rpe65 | 66 | Cxcl1 |
| 102 | Hfe | 102 | Hfe |
| 206 | Trip1 | 206 | Trip1 |
| 78 | Eno3 | 78 | Eno3 |
| 166 | Gygm | 166 | Pygm |
| 85 | Gem | 171 | Robo4 |
| 123 | Jak3 | 85 | Gem |
| 143 | Nrl | 123 | Jak3 |
| 13 | Apbb1 | 87 | Glut1 |
| 2 | Abca4 | 2 | Abca4 |
| 120 | Irs1 | 120 | Irs1 |
| 175 | Rxrg | 40 | Ccnd3 |
| 40 | Ccnd3 | 139 | Nfkb1 |
| 209 | Usp9x | 56 | Cldn5 |
| 215 | Vldir | 209 | Usp9x |
| 17 | Atp6ap2 | 215 | Vldlr |
| 136 | Mmp9 | 159 | Pax6 |
| 238 | Jun | 238 | Jun |
| 186 | Sil1 | 186 | Sil1 |
| 44 | Cd59a | 49 | Cdr2 |
| 49 | Cdr2 | 3 | Ace |
| 154 | Pecam1 | 5 | Adipor1 |
| 158 | Pkia | 7 | Aif1 |
| 157 | Pig7 | 223 | Apln |
| 156 | Pgf | 14 | Arr3 |
| 4 | Adam9 | 15 | At1r |

[Table 4-1-2]

| gene clusters retina | | | |
|---|---|---|---|
| group i | | group ii | |
| significant change in CNV | | significant change in LPS | |
| probe # | symbol | probe # | symbol |
| 5 | Adipor1 | 18 | Best1 |
| 7 | Aif1 | 23 | C1ql1 |
| 8 | Akt3 | 47 | Cdh3 |
| 230 | Aoc3 | 55 | Ckb |
| 14 | Arr3 | 57 | Clip1 |
| 15 | At1r | 217 | Cp |
| 16 | At2r | 63 | Crx |
| 18 | Best1 | 221 | Cxcr4 |
| 23 | C1gl1 | 73 | Ednrb |
| 32 | Casp9 | 75 | Efna5 |
| 33 | Cckbr | 12 | Egln3 |
| 47 | Cdh3 | 77 | Elovl4 |
| 55 | Ckb | 242 | Epo |
| 57 | Clip1 | 254 | Furin |
| 58 | Cnga1 | 89 | Gnat1 |
| 61 | Col7a1 | 91 | Gngt2 |
| 225 | Cp | 95 | Grm2 |
| 63 | Crx | 98 | Gsk3b |
| 67 | Cxcl12 | 100 | Guk1 |
| 69 | Cyb5r1 | 108 | Id3 |
| 70 | Darc | 122 | Itgav |
| 73 | Ednrb | 124 | Kdr |
| 75 | Efna5 | 237 | Mapk3 |
| 12 | Egln3 | 129 | Mark2 |
| 77 | Elovl4 | 134 | Mmp2 |
| 242 | Epo | 147 | Opn1sw |
| 83 | Flt1 | 155 | Pex1 |
| 254 | Furin | 160 | Ppara |
| 88 | Gnao1 | 161 | Prkca |
| 89 | Gnat1 | 162 | Prnp |
| 90 | Gnat2 | 163 | Prom1 |
| 91 | Gngt2 | 231 | Ptgs1 |
| 95 | Grm2 | 232 | Ptgs2 |
| 96 | Grm6 | 170 | Rho |

(continued)

| gene clusters retina | | | |
|---|---|---|---|
| group i | | group ii | |
| significant change in CNV | | significant change in LPS | |
| probe # | symbol | probe # | symbol |
| 98 | Gsk3b | 172 | Rom1 |

[Table 4-1-3]

| retina pigment epithelium/choroid | | | |
|---|---|---|---|
| group iii | | group iv | |
| significant change in CNV | | significant change in LPS | |
| probe # | symbol | probe # | symbol |
| 105 | Hspa1b | 105 | Hspa1b |
| 97 | Gsk3a | 116 | Il1a |
| 117 | Il1b | 101 | H2-K1 |
| 107 | Icam1 | 117 | Il1b |
| 106 | Hspa2 | 107 | Isam1 |
| 111 | Igf1 | 118 | Il6 |
| 93 | Gpr143 | 106 | Hspa2 |
| 92 | Gpnmb | 111 | Igf1 |
| 173 | Rpe65 | 93 | Gpr143 |
| 165 | Pxmp3 | 66 | Cxcl1 |
| 102 | Hfe | 92 | Gpnmb |
| 203 | Timp2 | 173 | Rpe65 |
| 82 | Fgfr1 | 165 | Pxmp3 |
| 79 | Erap1 | 102 | Hfe |
| 78 | Eno3 | 193 | Spp1 |
| 166 | Pygm | 65 | Ctss |
| 62 | Col8a2 | 203 | Timp2 |
| 171 | Robo4 | 82 | Fgfr1 |
| 51 | Cfb | 121 | Isgf3g |
| 85 | Gem | 79 | Erap1 |
| 131 | Mef2c | 78 | Eno3 |
| 194 | Stat1 | 68 | Cxcl2 |
| 205 | Tlr4 | 166 | Pygm |
| 21 | C1qb | 62 | Cola2 |
| 195 | Stat3 | 51 | Cfb |
| 87 | Glut1 | 190 | Socs3 |

(continued)

| retina pigment epithelium/choroid | | | |
|---|---|---|---|
| group iii | | group iv | |
| significant change in CNV | | significant change in LPS | |
| probe # | symbol | probe # | symbol |
| 38 | Ccl7 | 131 | Mef2c |
| 120 | Irs1 | 194 | Stat1 |
| 64 | Ctnna1 | 226 | Tnfa |

[Table 4-1-4]

| retina pigment epithelium/choroid | | | |
|---|---|---|---|
| group iii | | group iv | |
| significant change is CNV | | significant change in LPS | |
| probe # | symbol | probe # | symbol |
| 148 | Osbpl1a | 205 | Tlr4 |
| 128 | Loxl1 | 21 | C1qb |
| 175 | Rxrg | 13 | Apbb1 |
| 204 | Timp3 | 195 | Stat3 |
| 40 | Ccnd3 | 87 | Glut1 |
| 212 | Vegfb | 38 | Ccl7 |
| 210 | Vcam1 | 2 | Abca4 |
| 213 | Vegfc | 120 | Irs1 |
| 60 | Cntf | 64 | Ctnna1 |
| 56 | Cldn5 | 148 | Osbpl1a |
| 241 | Ctqf | 128 | Loxl1 |
| 209 | Usp9x | 175 | Rxrg |
| 215 | Vldlr | 204 | Timp3 |
| 200 | Tgfb3 | 202 | Timp1 |
| 138 | Nes | 40 | Ccnd3 |
| 133 | Mmp14 | 212 | Vegfb |
| 136 | Mmp9 | 210 | Vcam1 |
| 25 | C3 | 213 | Vegfc |
| 164 | Ptgds | 60 | Cntf |
| 153 | Pdpn | 139 | Nfkb1 |
| 233 | Pla2g5 | 56 | Cldn5 |
| 127 | Lmo1 | 241 | Ctgf |
| 222 | Esm1 | 209 | Usp9x |
| 50 | Cebpd | 215 | Vldlr |
| 238 | Jun | 200 | Tgfb3 |

(continued)

| retina pigment epithelium/choroid | | | |
|---|---|---|---|
| group iii | | group iv | |
| significant change is CNV | | significant change in LPS | |
| probe # | symbol | probe # | symbol |
| 187 | Slc16a1 | 149 | Pax6 |
| 186 | Sil1 | 17 | Atp6ap2 |
| 178 | Scd1 | 138 | Nes |
| 35 | Ccl2 | 133 | Mmp14 |
| 234 | Il6st | 36 | Ccl3 |
| 41 | Cd44 | 22 | C1qc |
| 44 | Cd59a | 153 | Pdpn |
| 183 | Serpinf1 | 233 | Pla2g5 |
| 49 | Cdr2 | 244 | Angpt-1 |

[Table 4-2-1]

| gene clusters retina | | | |
|---|---|---|---|
| gro up i | | group ii | |
| significant change in CNV | | significant change in LPS | |
| probe # | symbol | probe # | symbol |
| 100 | Guk1 | 179 | Sdc2 |
| 103 | Hif1a | 180 | Selenbp1 |
| 108 | Id3 | 181 | Selenbp2 |
| 109 | Ifna1 | 185 | Sfrp5 |
| 110 | Ifnr | 191 | Sox9 |
| 112 | Igf1r | 192 | Sparc |
| 122 | Itgav | 199 | Tgfb2 |
| 124 | Kdr | 211 | Veqfa |

(continued)

| gene clusters retina | | | |
|---|---|---|---|
| gro up i | | group ii | |
| significant change in CNV | | significant change in LPS | |
| probe # | symbol | probe # | symbol |
| 134 | Mmp2 | | |
| 142 | Nr2e3 | | |
| 145 | Opa1 | | |
| 146 | Opn1mw | | |
| 147 | Opn1sw | | |
| 150 | Pde6a | | |
| 161 | Prkca | | |
| 162 | Prnp | | |
| 163 | Prom1 | | |
| 231 | Ptgs1 | | |
| 172 | Rom1 | | |
| 179 | Sdc2 | | |
| 180 | Selenbp1 | | |
| 191 | Sox9 | | |
| 192 | Sparc | | |
| 198 | Synpr | | |
| 199 | Tgfb2 | | |
| 208 | Tyrp1 | | |
| 211 | Vegfa | | |
| 214 | Vegfd | | |

[Table 4-2-2]

| retina pigment epithelium/choroid | | | |
|---|---|---|---|
| group iii | | group iv | |
| significant change in CNV | | significant change in LPS | |
| probe # | symbol | probe # | symbol |
| 154 | Pecam1 | 127 | Lmo1 |
| 158 | Pkia | 222 | Esm1 |
| 48 | Cdh5 | 50 | Cebpd |
| 28 | Calb2 | 187 | Slc16a1 |
| 249 | Adam19 | 186 | Sil1 |
| 24 | C1s | 178 | Scd1 |
| 248 | Adam17 | 37 | Ccl4 |
| 157 | Pig7 | 227 | Tgfb1 |

(continued)

| retina pigment epithelium/choroid | | | |
|---|---|---|---|
| group iii | | group iv | |
| significant change in CNV | | significant change in LPS | |
| probe # | symbol | probe # | symbol |
| 156 | Ppf | 35 | Ccl2 |
| 16 | At2r | 239 | Fos |
| 18 | Best1 | 234 | Il6st |
| 43 | Cd55 | 44 | Cd59a |
| 9 | Cxcr4 | 183 | Serpinf1 |
| 76 | Egf | 49 | Cdr2 |
| 81 | Fgf7 | 236 | Mapk1 |
| 83 | Flt1 | 154 | Pecam1 |
| 122 | Itgav | 158 | Pkia |
| 124 | Kdr | 48 | Cdh5 |
| 125 | Lgals3 | 249 | Adam19 |
| 134 | Mmp2 | 24 | C1s |
| 135 | Mmp8 | 248 | Adam17 |
| 180 | Selenbp1 | 229 | Tnfrsf1a |
| 181 | Selenbp2 | 250 | Mmp1 |
| 197 | Stat6 | 251 | Mmp7 |
| 214 | Vegfd | 252 | Selp |

(continued)

| retina pigment epithelium/choroid | | | |
|---|---|---|---|
| group iii | | group iv | |
| significant change in CNV | | significant change in LPS | |
| probe # | symbol | probe # | symbol |
| | | 156 | Pgf |
| | | 15 | At1r |
| | | 16 | At2r |
| | | 39 | Ccl8 |
| | | 54 | Chrna7 |
| | | 57 | Clip1 |
| | | 9 | Cxcr4 |
| | | 69 | Cyb5r1 |
| | | 76 | Egf |
| | | 81 | Fgf7 |
| | | 83 | Flt1 |
| | | 94 | Grem2 |
| | | 124 | Kdr |
| | | 125 | Lgals3 |
| | | 126 | Lipc |
| | | 134 | Mmp2 |
| | | 135 | Mmp8 |
| | | 140 | Nos3 |
| | | 152 | Pdgfb |
| | | 253 | Sele |
| | | 180 | Selenbp1 |
| | | 181 | Selenbp2 |
| | | 197 | Stat6 |

Table 5

[Table 5-1-1]

| conribution order to formula | gene name | η |
|---|---|---|
| 1 | Hspa1b | 28.7609 |
| 2 | Il1a | 28.75654 |
| 3 | Gsk3a | 28.73853 |
| 4 | H2-K1 | 28.66572 |
| 5 | Il1b | 28.35073 |
| 6 | Icam1 | 28.17819 |
| 7 | Il6 | 28.16219 |

(continued)

| conribution order to formula | gene name | η |
|---|---|---|
| 8 | Hspa2 | 28.10675 |
| 9 | Igf1 | 28.06117 |
| 10 | Gpr143 | 27.93883 |
| 11 | Cscl1 | 27.90516 |
| 12 | Gpnmb | 27.83096 |
| 13 | Rpe65 | 27.76219 |
| 14 | Pxmp3 | 27.6787 |
| 15 | Irf6 | 27.63083 |
| 16 | Hfe | 27.58361 |
| 17 | Spp1 | 27.42699 |
| 18 | Ctss | 27.42325 |
| 19 | Math5 | 27.40593 |
| 20 | Doc2b | 27.39983 |
| 21 | Timp2 | 27.33578 |
| 22 | Fpfr1 | 27.31464 |
| 23 | Isgf3g | 27.27673 |
| 24 | Erap1 | 27.25751 |
| 25 | Trip1 | 27.24695 |
| 26 | Eno3 | 27.22714 |
| 27 | Cxcl2 | 27.21546 |
| 28 | Pygm | 27.16802 |
| 29 | Col8a2 | 27.14725 |

[Table 5-1-2]

| conribution order to formula | gene name | η |
|---|---|---|
| 30 | Lobo4 | 27.08653 |
| 31 | Cfb | 27.05906 |
| 32 | Socs3 | 27.05319 |
| 33 | Gem | 27.05211 |
| 34 | Mef2c | 27.0515 |
| 35 | Stat1 | 27.04586 |
| 36 | Tnfa | 27.03798 |
| 37 | Tlr4 | 27.03179 |
| 38 | Jak3 | 27.00856 |
| 39 | Np | 27.0025 |
| 40 | C1qb | 26.98939 |

(continued)

| conribution order to formula | gene name | η |
|---|---|---|
| 41 | Gabrb3 | 26.98768 |
| 42 | Nrl | 26.95518 |
| 43 | Apbb1 | 26.93522 |
| 44 | Stat3 | 26.93438 |
| 45 | Glut1 | 26.91839 |
| 46 | Ccl7 | 26.81131 |
| 47 | Abca4 | 26.89867 |
| 48 | Irs1 | 26.86307 |
| 49 | Ctnna1 | 26.84505 |
| 50 | Osbpl1a | 26.75947 |
| 51 | Loxl1 | 26.74482 |
| 52 | Rxrg | 26.7149 |
| 53 | Timp3 | 26.67336 |
| 54 | Timp1 | 26.66398 |
| 55 | Ccnd3 | 26.65889 |
| 56 | Vegfb | 26.6176 |
| 57 | Vcam1 | 26.60984 |
| 58 | Vegfc | 26.60278 |
| 59 | Cntf | 26.5788 |
| 60 | Nfkb1 | 26.55388 |

[Table 5-1-3]

| conribution order to formula | gene name | η |
|---|---|---|
| 61 | Nt5e | 26.54656 |
| 62 | Cnga3 | 26.53593 |
| 63 | Cldn5 | 26.53553 |
| 64 | Ctqf | 26.52942 |
| 65 | Msr1 | 26.50954 |
| 66 | Actb | 26.49759 |
| 67 | Usp9x | 26.49715 |
| 68 | Vldlr | 26.47908 |
| 69 | Tgfb3 | 26.4436 |
| 70 | Pax6 | 26.43603 |
| 71 | Atp6ap2 | 26.41118 |
| 72 | Nes | 26.33202 |
| 73 | Mmp14 | 26.30448 |

(continued)

| conribution order to formula | gene name | η |
|---|---|---|
| 74 | Ccl3 | 26.29844 |
| 75 | Mmp9 | 26.29316 |
| 76 | Ttpa | 26.29071 |
| 77 | Rs1 | 26.16883 |
| 78 | C1qc | 26.16276 |
| 79 | Slc16a4 | 26.16204 |
| 80 | C3 | 26.13161 |
| 81 | Ptgds | 26.12598 |
| 82 | Pdon | 26.11834 |
| 83 | Pla2g5 | 26.11101 |
| 84 | Angpt-1 | 26.07373 |
| 85 | Lmo1 | 26.03846 |
| 86 | Elm1 | 25.95695 |

[Table 5-1-4]

| conribution order to formula | gene name | η |
|---|---|---|
| 87 | Cebpd | 25.93072 |
| 88 | Jun | 25.88974 |
| 89 | Slc16a1 | 25.88433 |
| 90 | Sil1 | 25.87845 |
| 91 | IScd1 | 25.86244 |
| 92 | Ccl4 | 25.83053 |
| 93 | Tgfb1 | 25.72079 |
| 94 | Ccl2 | 25.67051 |
| 95 | Fos | 25.66059 |
| 96 | Il6st | 25.64978 |
| 97 | Cd44 | 25.61791 |
| 98 | Cd59a | 25.61382 |
| 99 | Serpinf1 | 25.57669 |
| 100 | Cdr2 | 25.57435 |
| 101 | Mapk1 | 25.57417 |
| 102 | Cd45 | 25.55897 |
| 103 | Pecam1 | 25.52968 |
| 104 | Pkia | 25.50032 |
| 105 | Cdh5 | 25.18971 |
| 106 | Calb2 | 24.99968 |

(continued)

| conribution order to formula | gene name | η |
|---|---|---|
| 107 | Adam19 | 24.87039 |
| 108 | C1s | 24.84763 |
| 109 | Adam17 | 24.66254 |
| 110 | Tnfrsf1a | 24.56724 |
| 111 | Pig7 | 24.46434 |
| 112 | Mmp1 | 24.41771 |
| 113 | Mmp7 | 24.26331 |
| 114 | Selp | 23.96141 |
| 115 | Pgf | 23.87452 |

Moreover, the results of a study using the determination formula are shown below. In the table, η represents the signal/noise ratio of the determination formula (S/N); the greater the value, the more preferable it is.

[Table 6]

| name of genes | | | | | | determination formula S/N | |
|---|---|---|---|---|---|---|---|
| | | | | | | retina η | retinal pigment epithelium/ choroid η |
| Cxcl1 | At2r | At1r | Cfb | | | 6.088 | 6.635 |
| 116 | Pig7 | Jak3 | Timp1 | Mmp9 | At2r | 1.647 | 1.560 |
| Selenbp2 | Pgf | Sil1 | Cxcl2 | Calb2 | Eno3 | 3.844 | -5.020 |
| Nfkb1 | Rxrg | Usp9x | Il6 | Robo4 | Vcam1 | 5.507 | 4.392 |
| Cxcl1 | At2r | Tgfb2 | Cxcl1 | Cfb | Fgf7 | 6.236 | 4.384 |
| Cxcl1 | At2r | Vegfa | Cxcl1 | Cfb | Fgf7 | 8.165 | 5.239 |
| Cxcl1 | At2r | Vegfa | Cxcl1 | Cfb | Vegfb | 6.489 | 4.090 |
| Cxcl1 | At2r | Vegfa | Cxcl1 | Cfb | Vegfc | 7.277 | 5.030 |
| Il6 | Pig7 | Vegfa | Timp1 | Mmp9 | Vegfc | 4.588 | 19.102 |
| Cxcl1 | At2r | Vegfa | Cxcl1 | Cfb | Vegfd | 7.277 | 3.696 |
| Cxcl1 | At2r | Vegfa | Cxcl1 | Cfb | Vldlr | 6.487 | 2.478 |

From those results, to determine the condition of the retina, namely, to determine whether the condition resulted from light damage or from inflammation caused by an inflammation-inducing substance, it was shown that such conditions are preferred to be determined by focusing on genes Cxcl1, At2r, Vegfa, Cxcl1, Cfb and Fgf7. Also, to determine the condition in the retinal epithelial cells and choroid, namely, to determine whether the condition resulted from light damage or from inflammation caused by an inflammation-inducing substance, it was shown that such conditions are preferred to be determined by focusing on genes I16, Pig7, Vegfa, Timp1, Mmp9 and Vegfc.

The results above have identified genes that show variations when a disorder caused by light, inflammation or angiogenesis occurs in the retina and retinal pigment epithelium/choroid of the eye. Also, genes that vary in the case of age-related macular degeneration have been also identified. By analyzing variations in the expression levels of these genes in the samples taken from a subject organism, the conditions of eye diseases in the subject organism, especially age-related macular degeneration, are evaluated. Also, from a sample taken from a subject organism that has contacted, ingested or been administered a test substance, the inhibitory or restorative functions of the test substance on the eye disease of the subject organism are evaluated by analyzing the change in the expression levels of those genes.

Namely, it was found that the method related to the present invention is capable of conducting objective evaluation of eye diseases, especially age-related macular degeneration, in a subject organism. Also, it was found that the method is capable of evaluating inhibitory or restorative effects of a test substance on eye diseases, especially on age-related macular degeneration

DESCRIPTION OF NUMERICAL REFERENCES

11 through hole
21 porous plate
31 hollow fiber
41 plate material

Sequence Text

SEQ ID Nos. 1~255 synthetic DNA

**Claims**

**1.** A method for evaluating the condition of an eye disease by using a microarray to measure the expression variation of a gene that is classified at least as (a)~(c) or (d)~(f) below respectively:

(a) a gene associated with both light damage to the retina and retinal inflammation caused by an inflammation-inducing substance;
(b) a gene associated with light damage to the retina, but excluding genes classified as (a) above;
(c) a gene associated with retinal inflammation caused by an inflammation-inducing substance, but excluding genes classified as (a) above;
(d) regarding retinal pigment epithelial cells and choroid, a gene associated with both light damage and inflammation caused by an inflammation-inducing substance;
(e) a gene associated with light damage to the retinal pigment epithelial cells and choroid, but excluding genes classified as (d) above;
(f) a gene associated with inflammation of the retinal pigment epithelial cells and choroid caused by an inflammation-inducing substance, but excluding genes classified as (d) above.

**2.** A method for evaluating inhibitory or restorative functions of a test substance for an eye disease by using a microarray to measure the expression variation of a gene that is classified at least as (a)~(c) or (d)~(f) below respectively:

(a) a gene associated with both light damage to the retina and retinal inflammation caused by an inflammation-inducing substance;
(b) a gene associated with light damage to the retina, but excluding genes classified as (a) above;
(c) a gene associated with retinal inflammation caused by an inflammation-inducing substance, but excluding genes classified as (a) above;
(d) regarding retinal pigment epithelial cells and choroid, a gene associated with both light damage and inflammation caused by an inflammation-inducing substance;
(e) a gene associated with light damage to the retinal pigment epithelial cells and choroid, but excluding genes classified as (d) above;
(f) a gene associated with inflammation of the retinal pigment epithelial cells and choroid caused by an inflammation-inducing substance, but excluding genes classified as (d) above.

**3.** The method according to Claim 1 or 2, wherein a gene classified as (a) is at least one cluster selected from a group of At1r, Jak3, Ccnd3, H2-K1, C1q11, Id3, Tgfb2, Ckb, Gnat1, Efna5, Crx, Rom1, Arr3, Gsk3a, Adipor1, Hspa1b, Guk1, Abca4, egln3, Gngt2, Clip1, Prnp, Sparc, Elovl4, Gsk3b, Itgav, Vegfa, Vegfb, Vegfc, Vegfd, Sdc2, and Trip1;
a gene classified as (b) is at least one cluster selected from a group of At2r, Pig7, Pgf, Rxrg, Col7al, Casp9, Pecam1, Rpe65, Cckbr, Cd59a, Opn1mw, Grm6, Pkia, Darc, Apbb1, Prom1, Adam9, Cyb5r1, Gpr143, Atp6ap2, Nr2e3, Pde6a, Nrl, Cnga1, Hif1a, Gnat2, and Mmp2;
a gene classified as (c) is at least one cluster selected from a group of Cxcll, I16, Selenbp2, Nfkb1, Cldn5, Sox9, Cp, Grm2, Pax6, Prkca, Mark2, Ppara, Gem, Opn1sw, Robo4, Rho, Glut1, and Pex1;
a gene classified as (d) is at least one cluster selected from a group of cxcr4, At2r, Vcam1, Mef2c, Pkia, Scd1, Loxl1, H2-K1, Pxmp3, Erap1, Pgf, Tgfb3, Pdpn, Gsk3a, Fgf7, Ccl2, Cntf, Col8a2, Pecam1, Cd59a, Rxrg, C1s, Ccl7, Osbpl1a, Glut1, Selenbp2, Serpinfl, Gpnmb, Hspa2, Nes, Stat1, Egf, C1qb, Mmp14, Timp2, Lmo1, Lgals3, Mmp8, Flt1, Cldn5, Mmp2, Vegfa, Vegfb, Vegfc, Vegfd, Hspa1b, Kdr, and Stat6;
a gene classified as (e) is at least one cluster selected from a group of Cfb, Mmp9, Calb2, Robo4, Gpr143, Cd44, Pig7, Il1b, C3, Gem, Cd55, Cebpd, Stat3, and Ccnd3; and
a gene classified as (f) is at least one cluster selected from a group of Cxcl1, Timp1, Cxcl2, I16, Igfl, Icam1, Lipc, At1r, Spp1, Ctss, C1qc, Nfkb1, Grem2, Abca4, Apbb1, Chrna7, Cyb5r1, Pdgfb, Isgf3g, Nos3, and Clipl.

**4.** The method according to any of Claims 1~3, wherein the determination formula below is used to evaluate the condition of an eye disease.
determination formula

$$M=(Xli\times\eta 1 / \beta 1+ \cdots + Xki\times\eta k / \beta k) (/\eta 1+ \cdots + \eta k )$$

When M1 max + $\sigma_{m1}$ <M2 min is satisfied, the condition of an evaluation sample cluster is the same as that in each disease model.
[in the formula, "M" represents the Mahalanobis distance to show the distance from the base space, "Xni (n=1~k)" represents a gene expression level or gene expression ratio, "ηn (n=1~k)" represents a signal-to-noise ratio (S/N) in each sample, and "βn (n=1~k) represents the sensitivity of each sample.
"M1" represents a normal sample cluster or a control sample cluster. "M1 max" indicates the maximum value of "M1" and "σ m1" represents the standard deviation from "M1". "M2" represents an evaluation sample cluster, and "M2 min" indicates the minimum value of "M2".

**5.** A microarray to evaluate the condition of an eye disease, wherein the nucleic acids or part of the nucleic acids described in (i), (ii) or (iii) below are mounted.

(i) nucleic acids containing a gene selected from (a)~(c) or (d)~(f) below:

(a) a gene associated with both light damage to the retina and retinal inflammation caused by an inflammation-inducing substance;
(b) a gene associated with light damage to the retina, but excluding genes classified as (a) above;
(c) a gene associated with retinal inflammation caused by an inflammation-inducing substance, but excluding genes classified as (a) above;
(d) regarding retinal pigment epithelial cells and choroid, a gene associated with both light damage and inflammation caused by an inflammation-inducing substance;
(e) a gene associated with light damage to the retinal pigment epithelial cells and choroid, but excluding genes classified as (d) above;
(f) a gene associated with inflammation of the retinal pigment epithelial cells and choroid caused by an inflammation-inducing substance, but excluding genes classified as (d) above.

(ii) nucleic acids composed of a base sequence complementary to the base sequence of the nucleic acids in (i) above; and
(iii) nucleic acids capable of hybridizing under stringent conditions with nucleic acids composed of a base sequence complementary to that in the nucleic acids in (i) or (ii) above.

**6.** A microarray according to Claim 5, wherein a gene classified as (a) is at least one cluster selected from a group of At1r, Jak3, Ccnd3, H2-K1, C1ql1, Id3, Tgfb2, Ckb, Gnat1, Efna5, Crx, Rom1, Arr3, Gsk3a, Adipor1, Hspa1b, Guk1, Abca4, egln3, Gngt2, Clip1, Prnp, Sparc, Elovl4, Gsk3b, Itgav, Vegfa, Vegfb, Vegfc, Vegfd, Sdc2, and Trip1;
a gene classified as (b) is at least one cluster selected from a group of At2r, Pig7, Pgf, Rxrg, Col7a1, Casp9, Pecam1, Rpe65, Cckbr, Cd59a, Opn1mw, Grm6, Pkia, Darc, Apbb1, Prom1, Adam9, Cyb5r1, Gpr143, Atp6ap2, Nr2e3, Pde6a, Nrl, Cnga1, Hif1a, Gnat2, and Mmp2;
a gene classified as (c) is at least one cluster selected from a group of Cxcl1, I16, Selenbp2, Nfkb1, Cldn5, Sox9, Cp, Grm2, Pax6, Prkca, Mark2, Ppara, Gem, Opn1sw, Robo4, Rho, Glut1, and Pex1;
a gene classified as (d) is at least one cluster selected from a group of cxcr4, At2r, Vcam1, Mef2c, Pkia, Scd1, Loxl1, H2-K1, Pxmp3, Erap1, Pgf, Tgfb3, Pdpn, Gsk3a, Fgf7, Ccl2, Cntf, Col8a2, Pecam1, Cd59a, Rxrg, C1s, Ccl7, Osbpl1a, Glut1, Selenbp2, Serpinfl, Gpnmb, Hspa2, Nes, Stat1, Egf, C1qb, Mmp14, Timp2, Lmo1, Lgals3, Mmp8, Flt1, Cldn5, Mmp2, Vegfa, Vegfb, Vegfc, Vegfd, Hspa1b, Kdr, and Stat6;
a gene classified as (e) is at least one cluster selected from a group of Cfb, Mmp9, Calb2, Robo4, Gpr143, Cd44, Pig7, Il1b, C3, Gem, Cd55, Cebpd, Stat3, and Ccnd3; and
a gene classified as (f) is at least one cluster selected from a group of Cxcl1, Timp1, Cxcl2, I16, Igfl, Icam1, Lipc, At1r, Spp1, Ctss, C1qc, Nfkb1, Grem2, Abca4, Apbb1, Chrna7, Cyb5r1, Pdgfb, Isgf3g, Nos3, and Clip1.

**7.** A microarray to evaluate the condition of an eye disease, wherein a probe is equipped to detect at least one type of gene selected from among the clusters of Hspa1b, Gsk3a and H2-K1.

【Fig. 1】

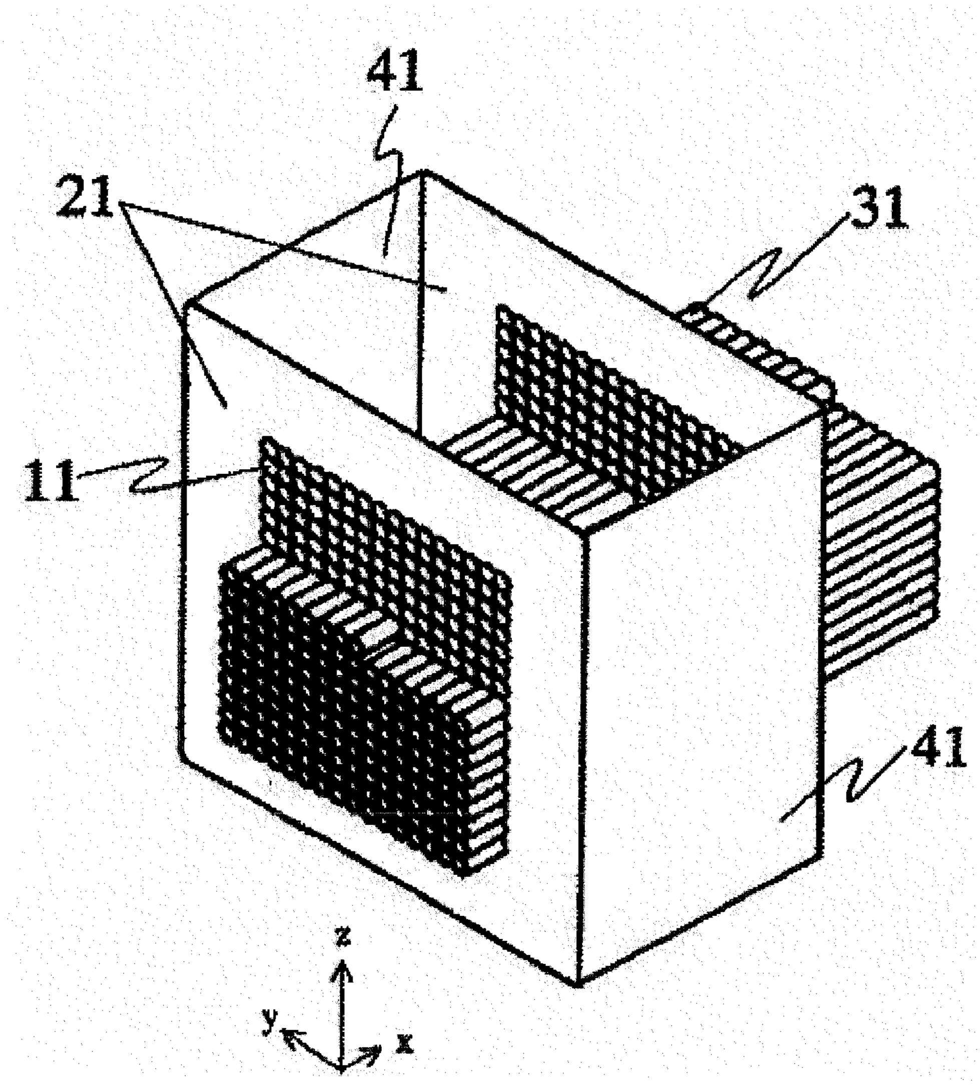

【Fig. 2A-1】

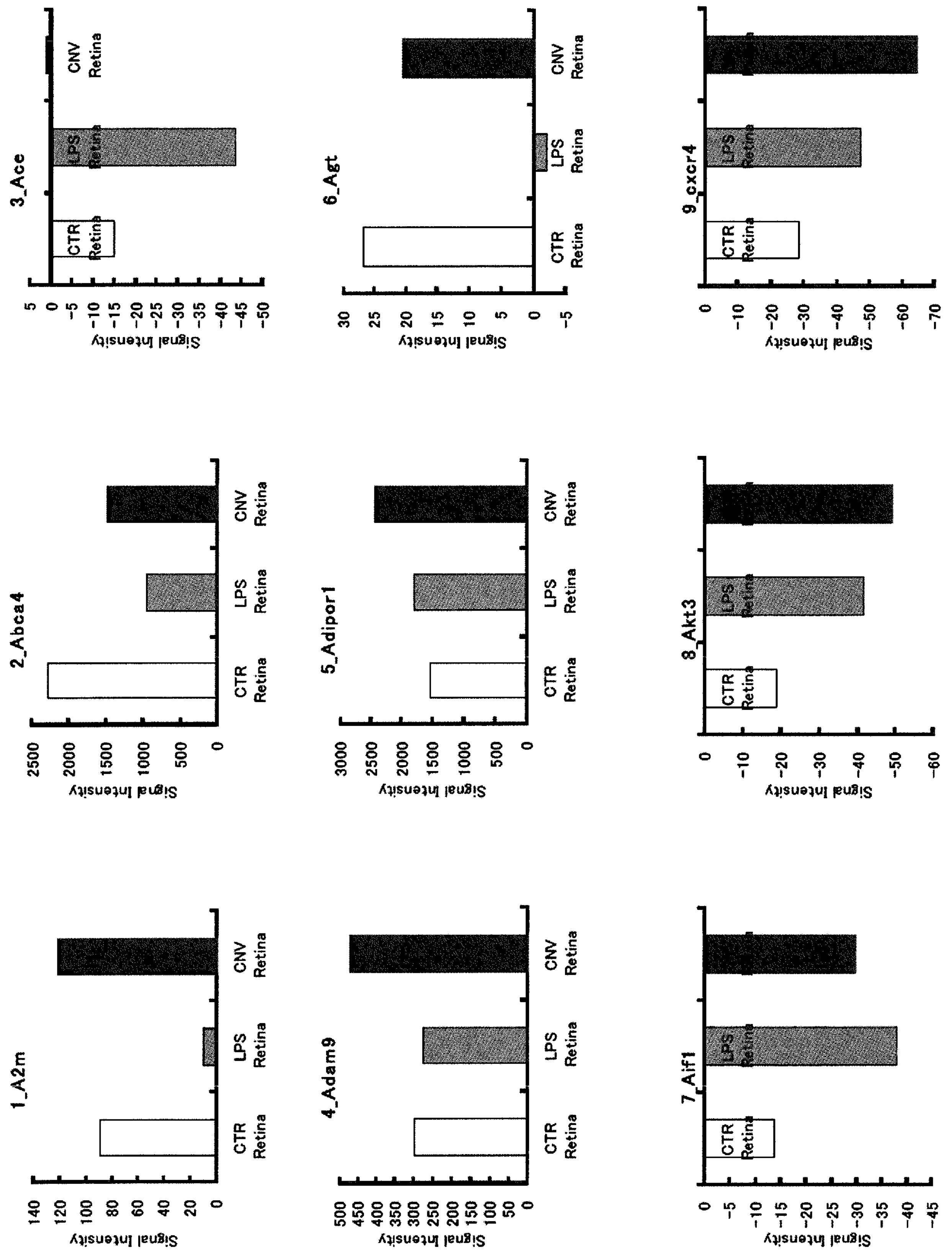
1_A2m
2_Abca4
3_Ace
4_Adam9
5_Adipor1
6_Agt
7_Aif1
8_Akt3
9_cxcr4
Signal Intensity
CTR Retina
LPS Retina
CNV Retina

【Fig. 2A-2】

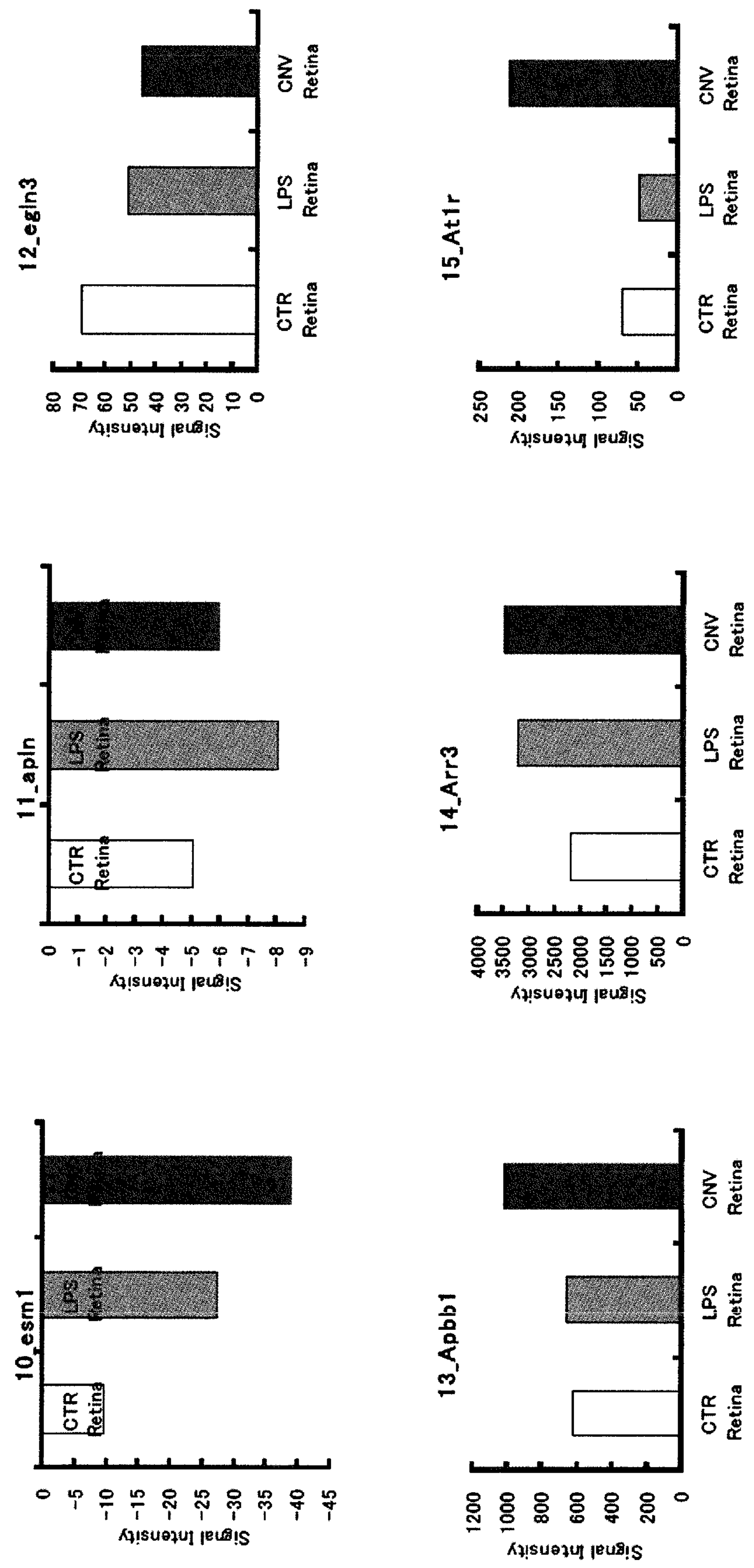
10_esm1
11_apln
12_egln3
13_Apbb1
14_Arr3
15_At1r
Signal Intensity
CTR Retina
LPS Retina
CNV Retina

【Fig. 2B-1】

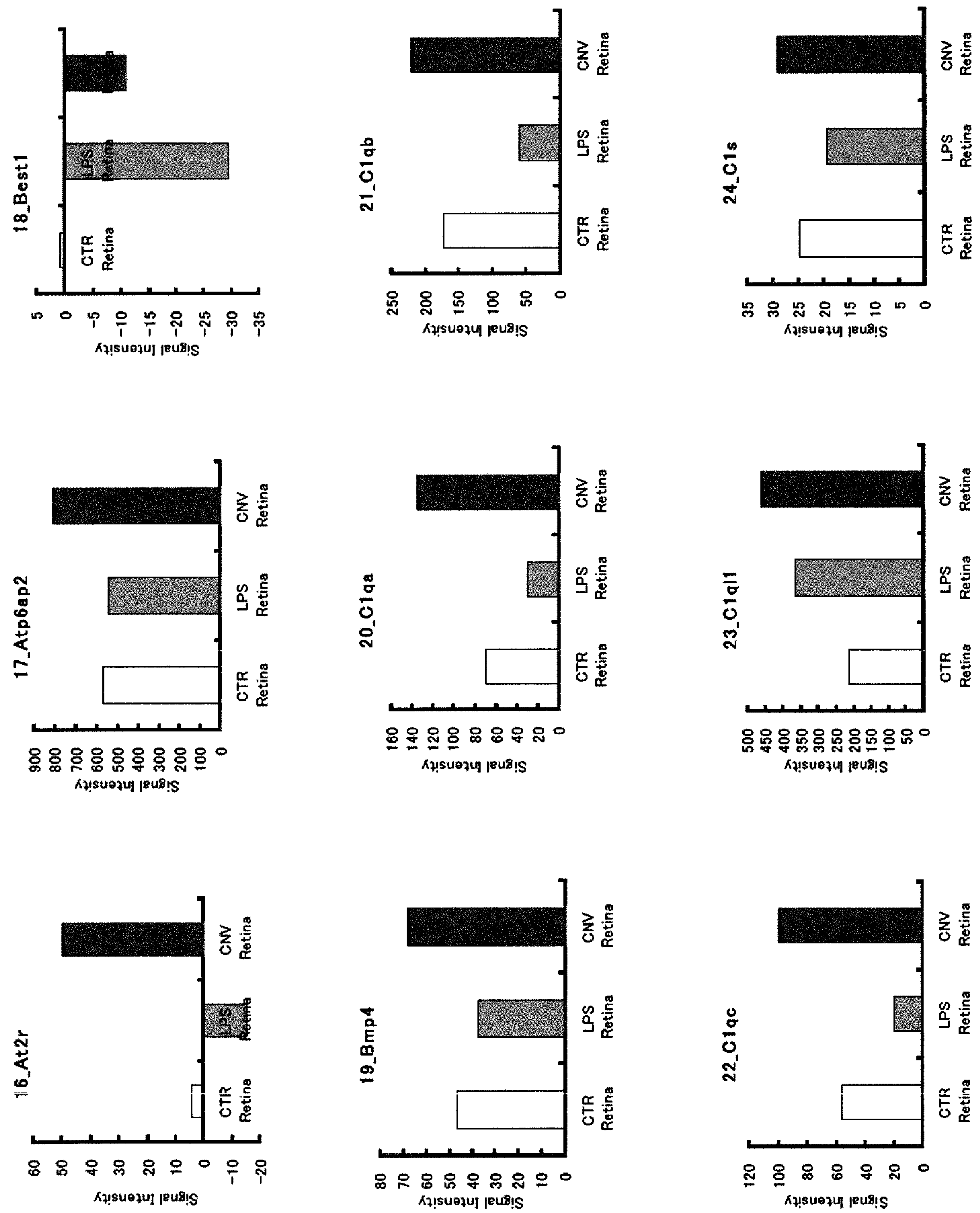
16_At2r
17_Atp6ap2
18_Best1
19_Bmp4
20_C1qa
21_C1qb
22_C1qc
23_C1ql1
24_C1s
Signal Intensity
CTR Retina
LPS Retina
CNV Retina

【Fig. 2B-2】

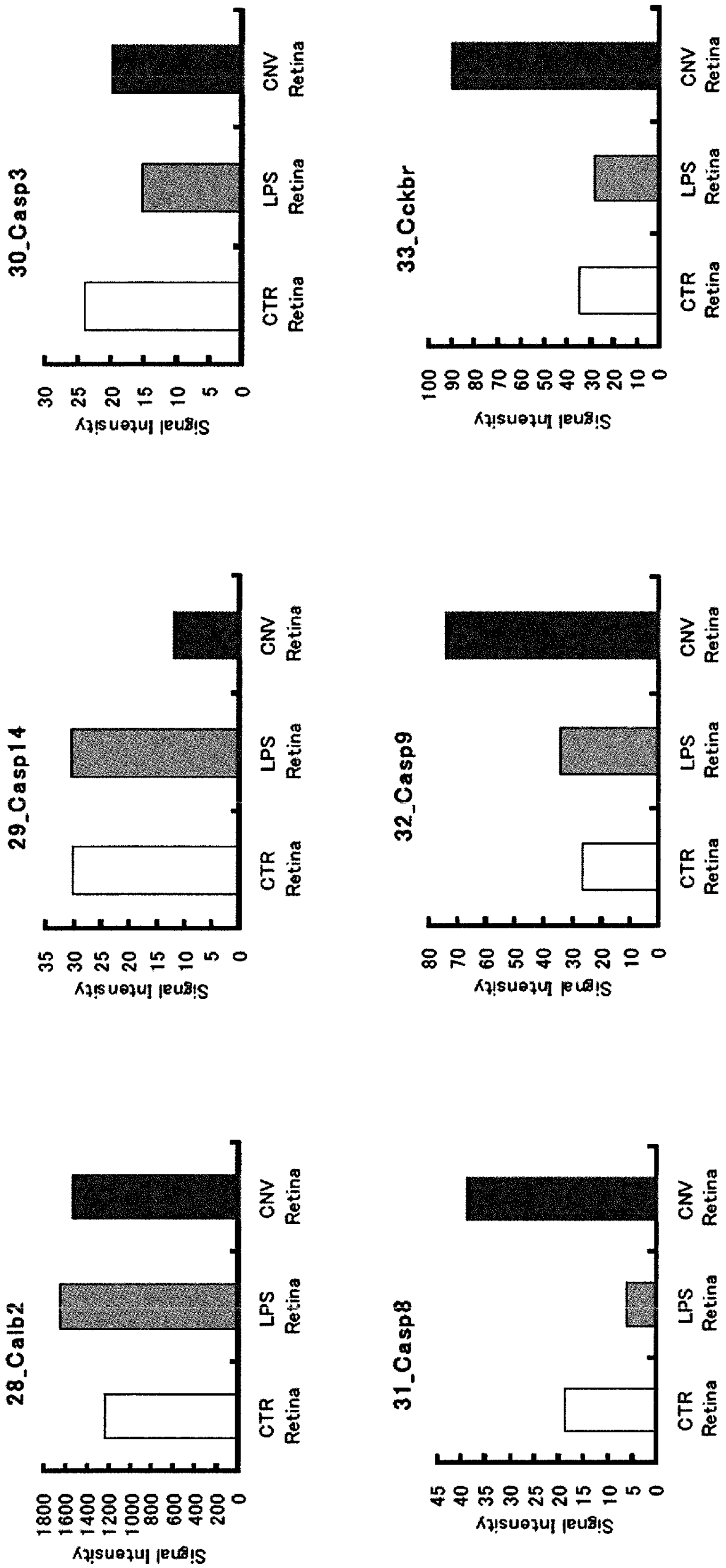

28_Calb2
29_Casp14
30_Casp3
31_Casp8
32_Casp9
33_Cckbr
Signal Intensity
CTR Retina
LPS Retina
CNV Retina

【Fig. 2C-1】

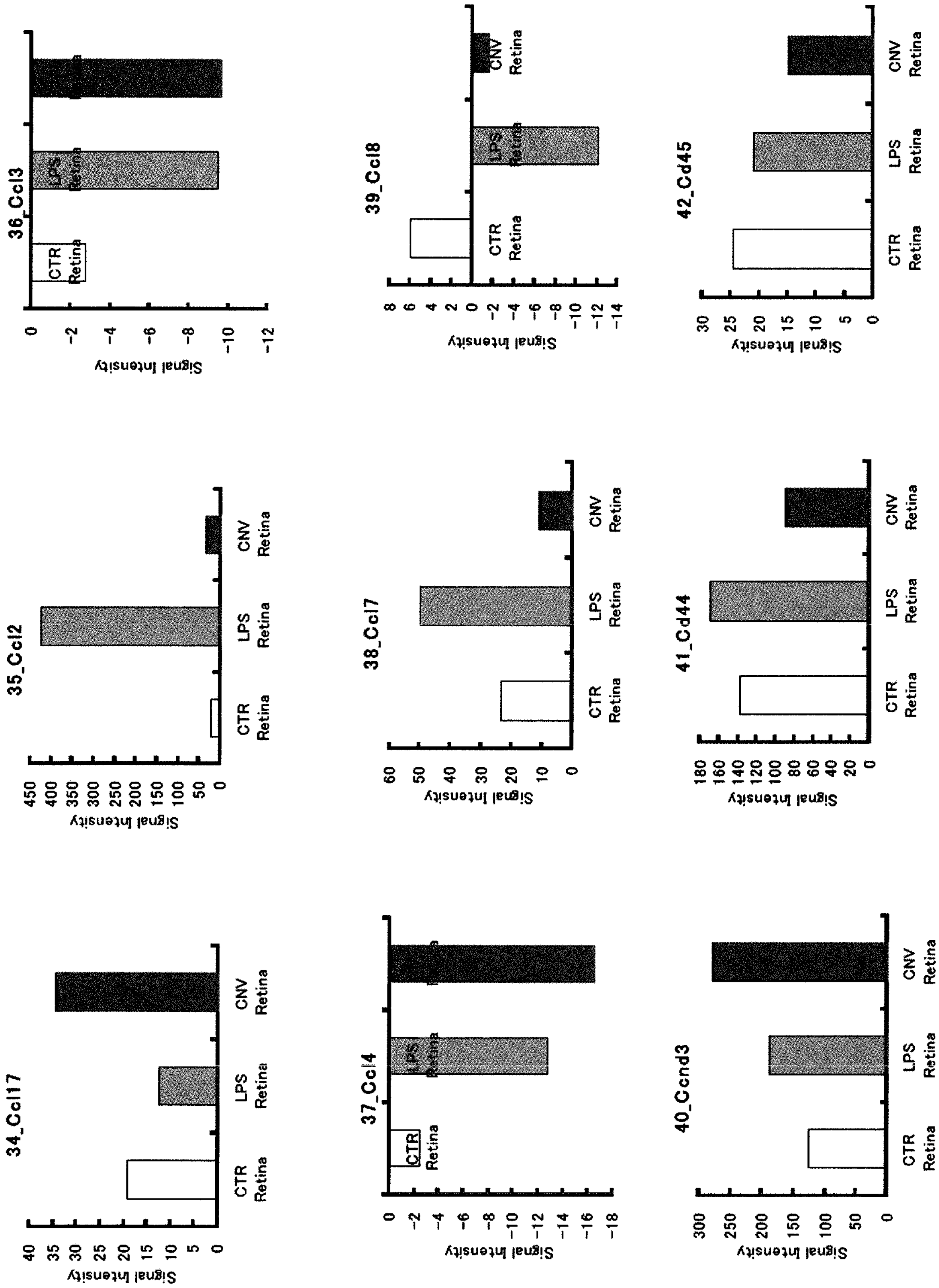
34_Ccl17
35_Ccl2
36_Ccl3
37_Ccl4
38_Ccl7
39_Ccl8
40_Ccnd3
41_Cd44
42_Cd45
Signal Intensity
CTR Retina
LPS Retina
CNV Retina

【Fig. 2C-2】

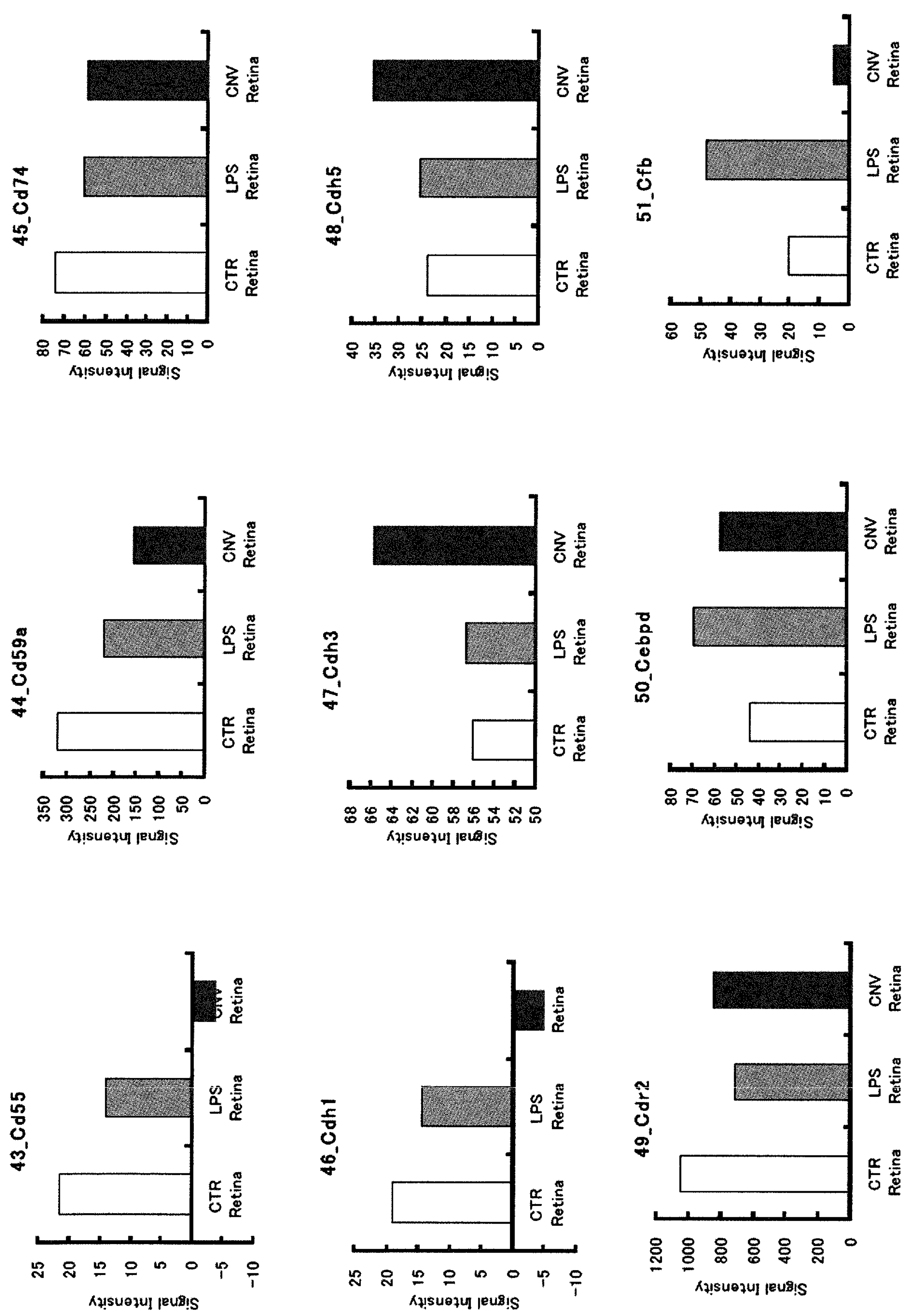
43_Cd55
44_Cd59a
45_Cd74
46_Cdh1
47_Cdh3
48_Cdh5
49_Cdr2
50_Cebpd
51_Cfb
Signal Intensity
CTR Retina
LPS Retina
CNV Retina

【Fig. 2D-1】

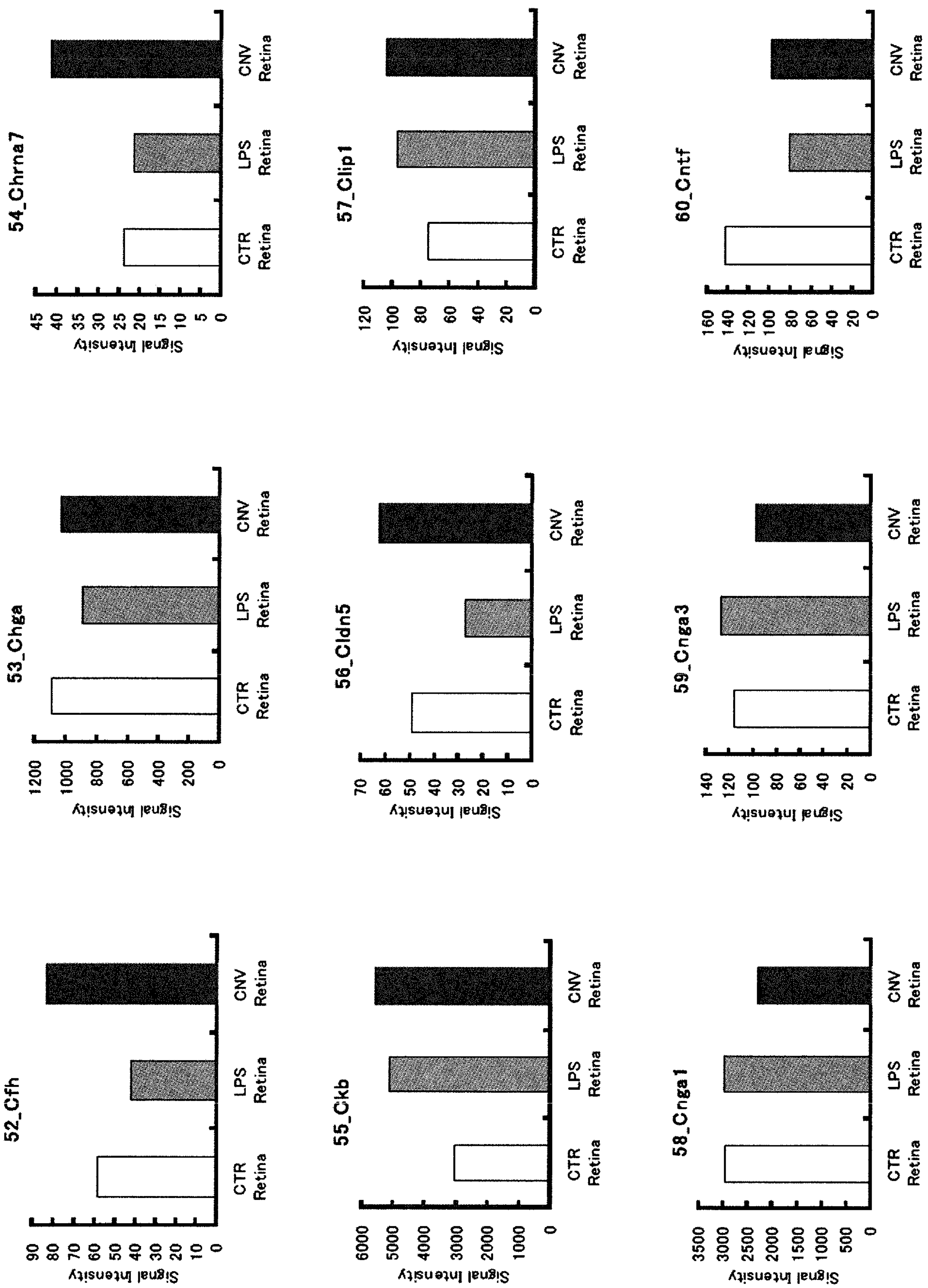
54_Chrna7
53_Chga
52_Cfh
57_Clip1
56_Cldn5
55_Ckb
60_Cntf
59_Cnga3
58_Cnga1
Signal Intensity
CTR Retina
LPS Retina
CNV Retina

【Fig. 2D-2】

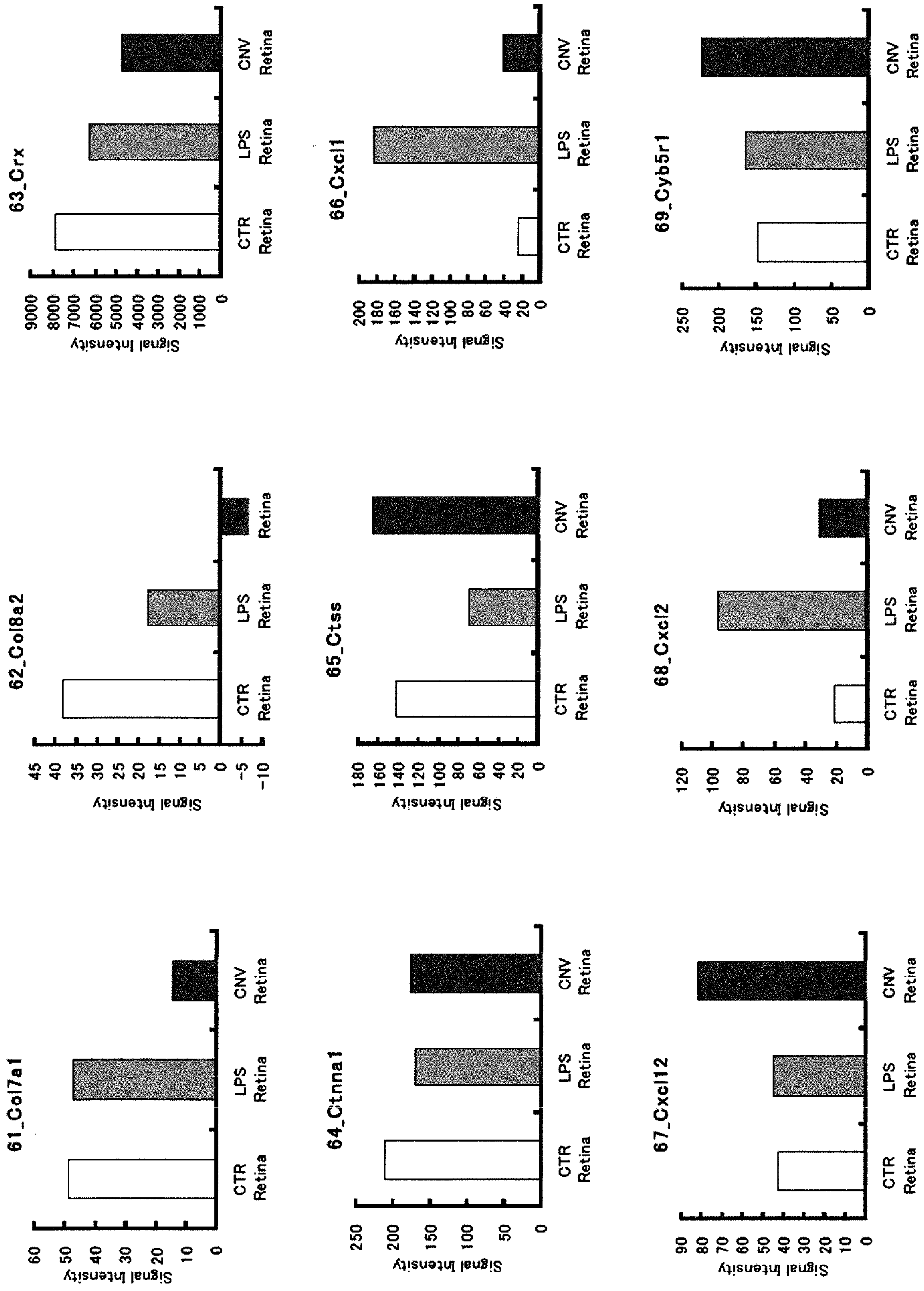
61_Col7a1
62_Col8a2
63_Crx
64_Ctnna1
65_Ctss
66_Cxcl1
67_Cxcl12
68_Cxcl2
69_Cyb5r1
Signal Intensity
CTR Retina
LPS Retina
CNV Retina

【Fig. 2E-1】

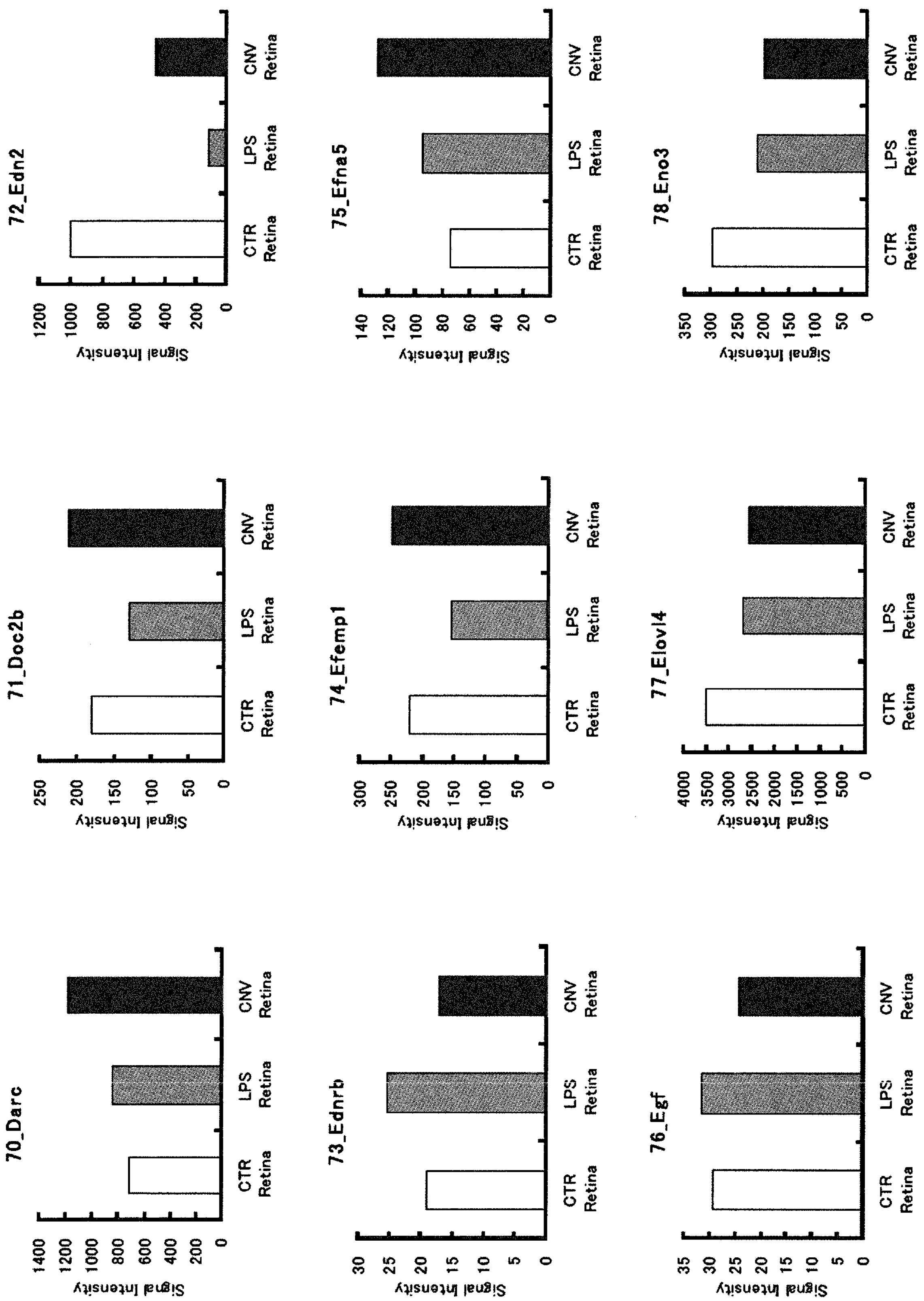
70_Darc
71_Doc2b
72_Edn2
73_Ednrb
74_Efemp1
75_Efna5
76_Egf
77_Elovl4
78_Eno3
Signal Intensity
CTR Retina
LPS Retina
CNV Retina

【Fig. 2E-2】

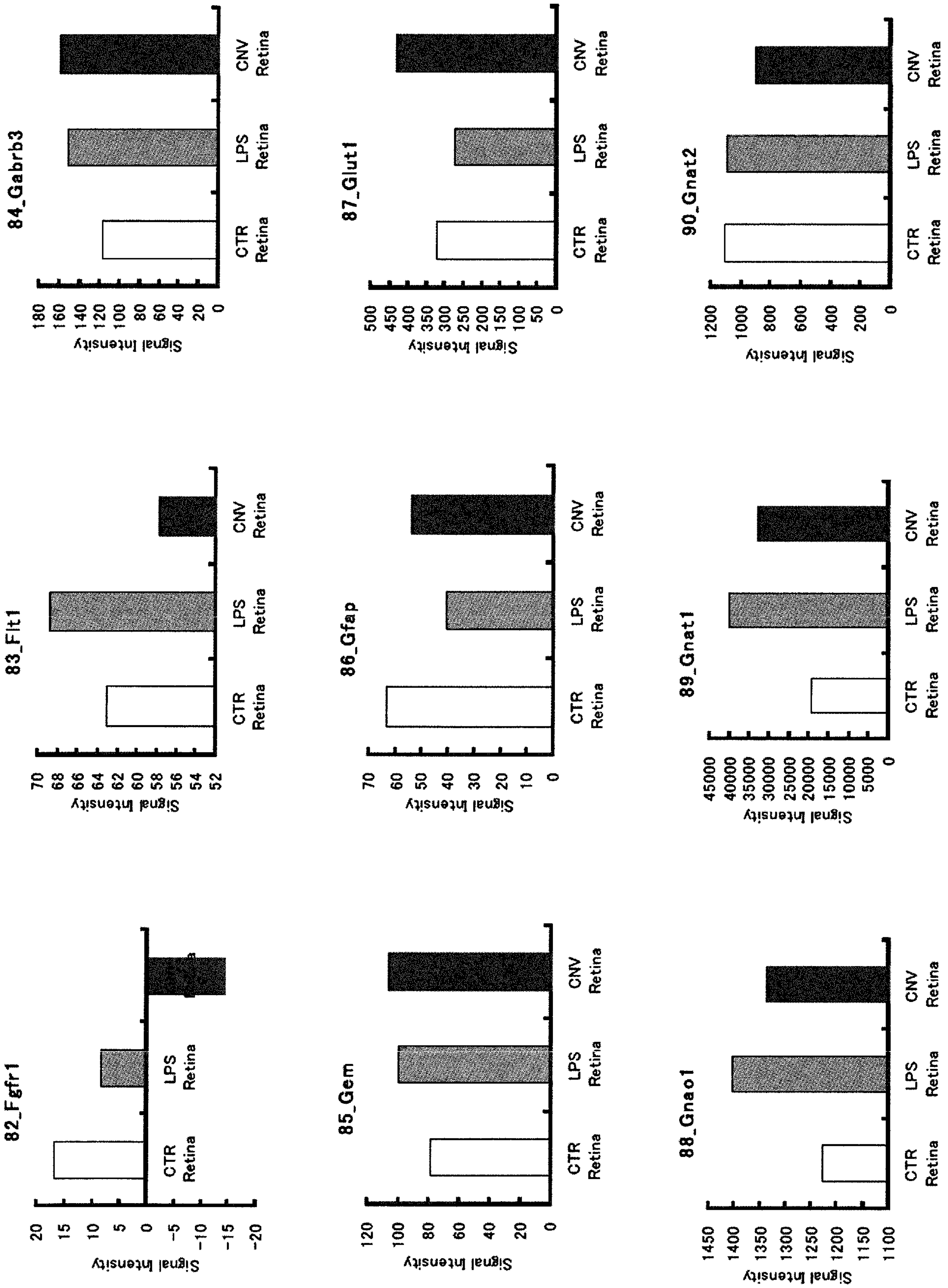
84_Gabrb3
87_Glut1
90_Gnat2
83_Flt1
86_Gfap
89_Gnat1
82_Fgfr1
85_Gem
88_Gnao1
Signal Intensity
CTR Retina
LPS Retina
CNV Retina

【Fig. 2F-1】

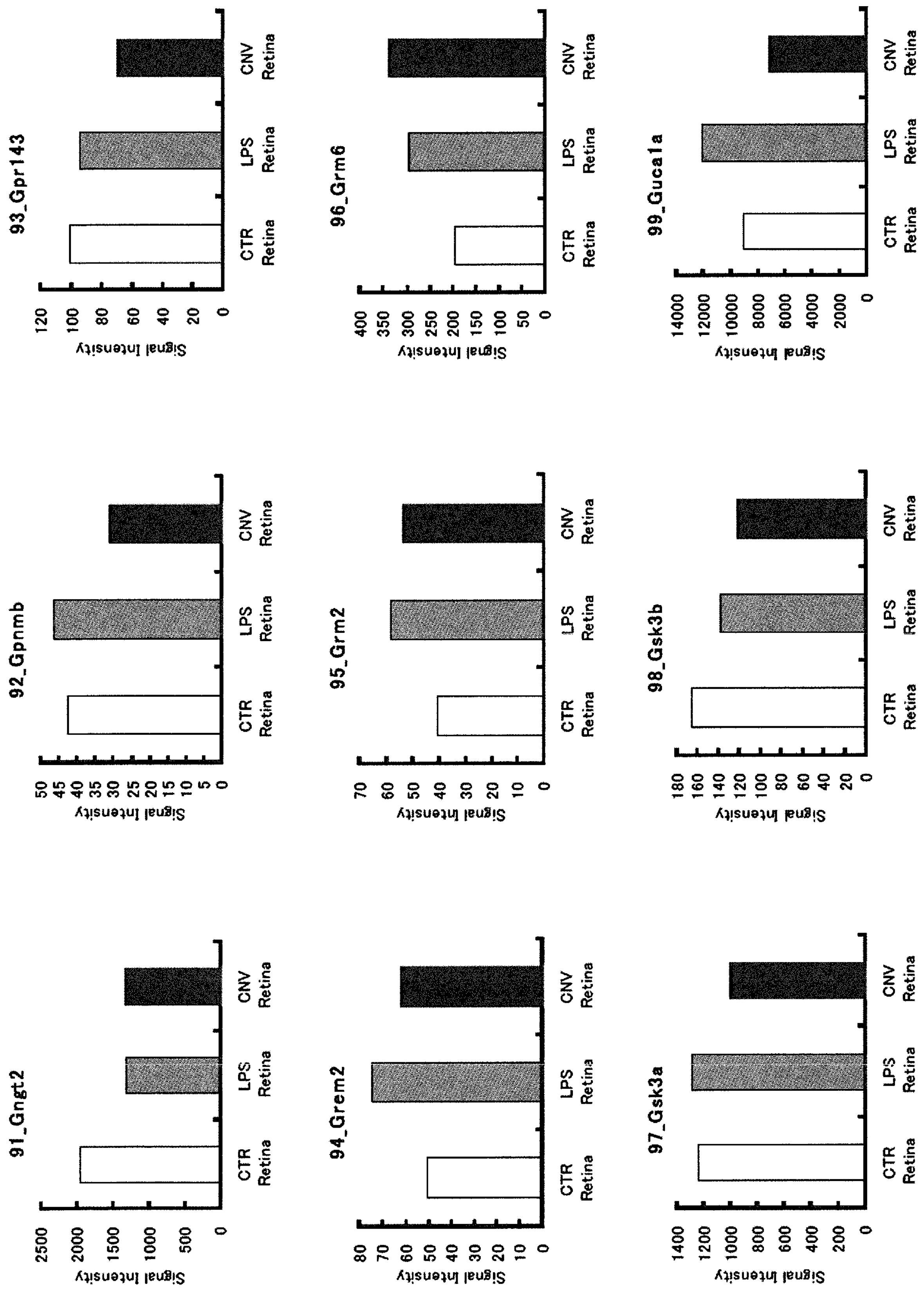
91_Gngt2
92_Gpnmb
93_Gpr143
94_Grem2
95_Grm2
96_Grm6
97_Gsk3a
98_Gsk3b
99_Guca1a
Signal Intensity
CTR Retina
LPS Retina
CNV Retina

【Fig. 2F-2】

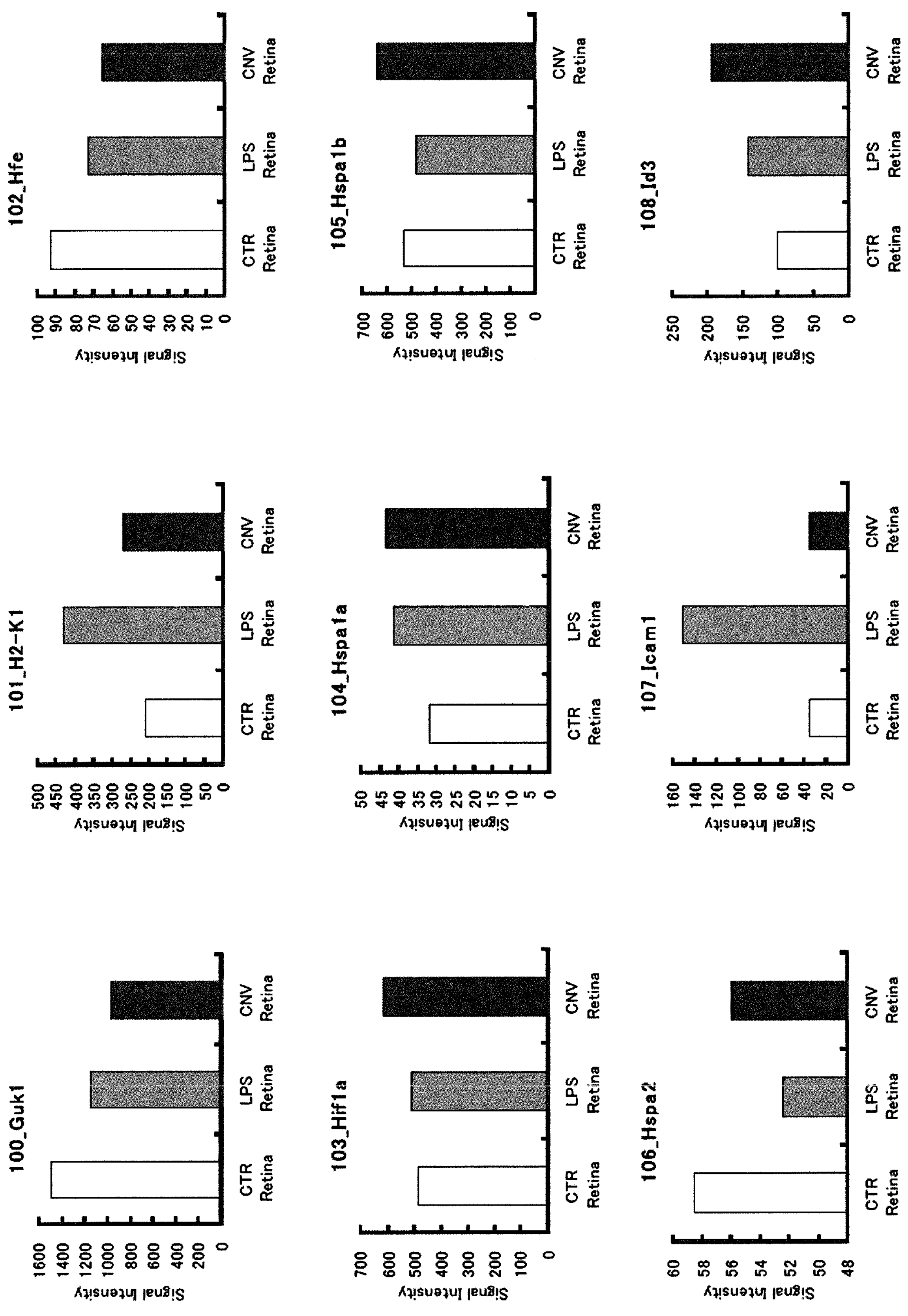
100_Guk1
101_H2-K1
102_Hfe
103_Hif1a
104_Hspa1a
105_Hspa1b
106_Hspa2
107_Icam1
108_Id3
Signal Intensity
CTR Retina
LPS Retina
CNV Retina

【Fig. 2G-1】

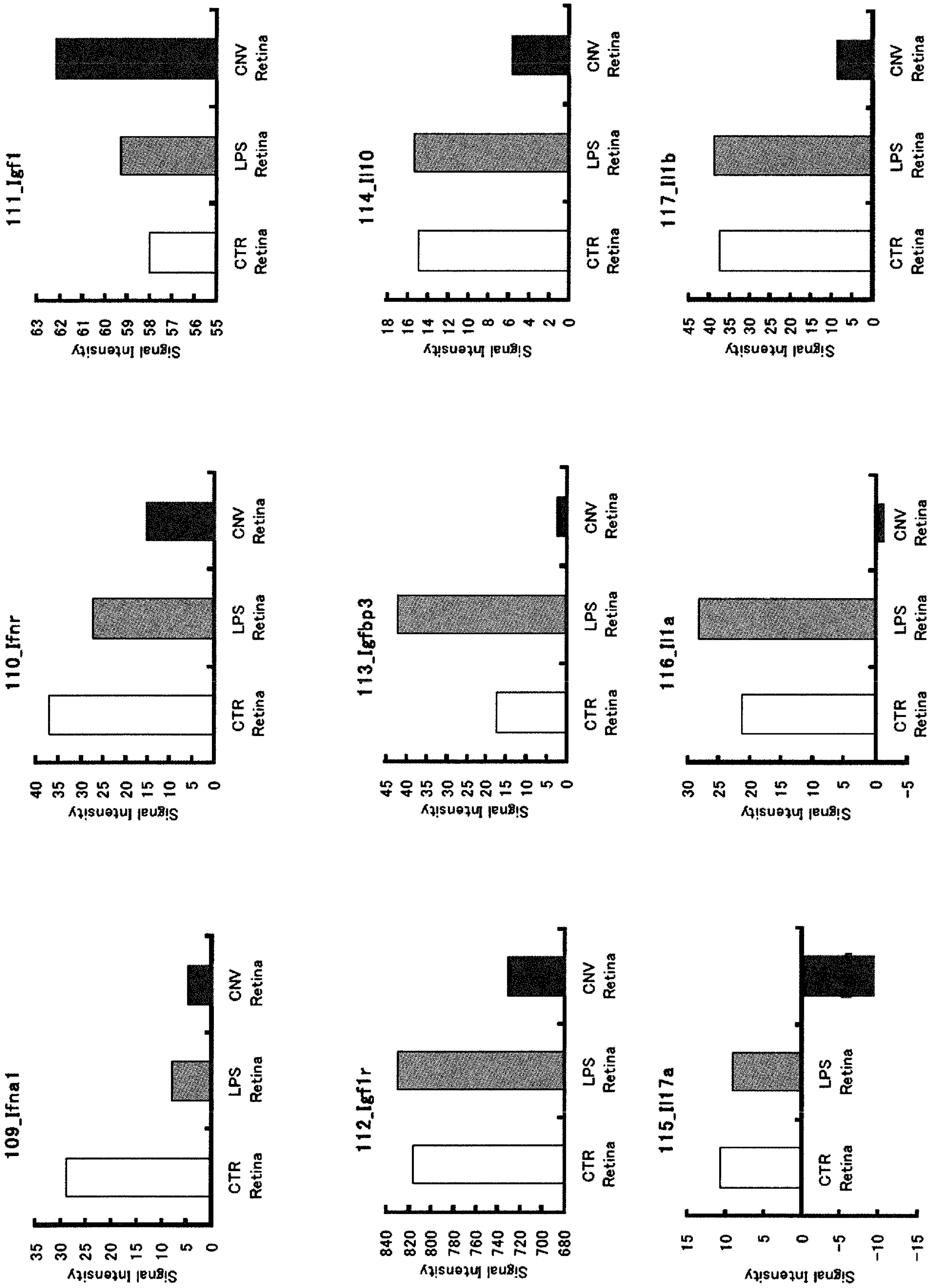
109_Ifna1
110_Ifnr
111_Igf1
112_Igf1r
113_Igfbp3
114_Il10
115_Il17a
116_Il1a
117_Il1b
Signal Intensity
CTR Retina
LPS Retina
CNV Retina

【Fig. 2G-2】

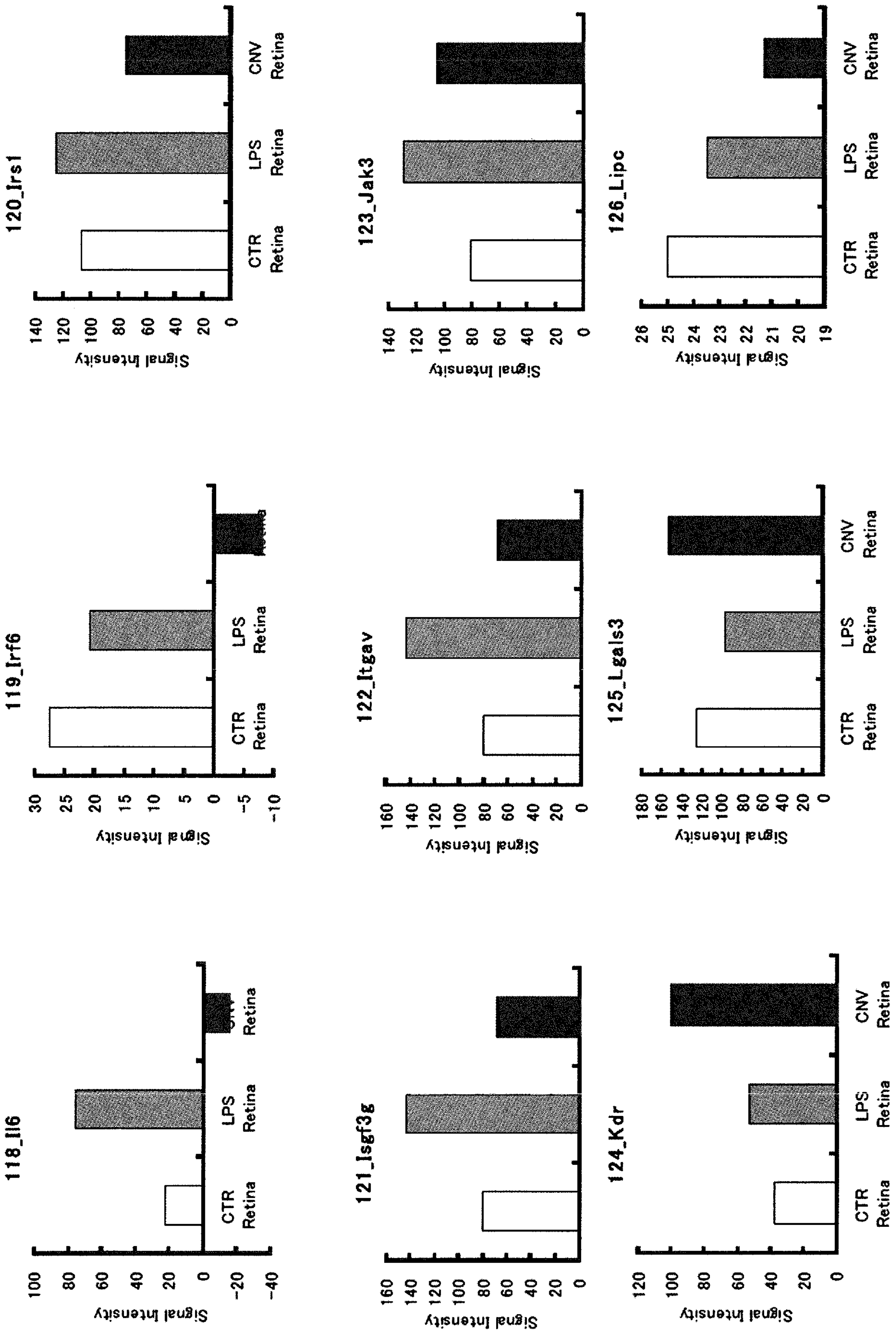
118_Il6
119_Irf6
120_Irs1
121_Isgf3g
122_Itgav
123_Jak3
124_Kdr
125_Lgals3
126_Lipc
Signal Intensity
CTR Retina
LPS Retina
CNV Retina

【Fig. 2H-1】

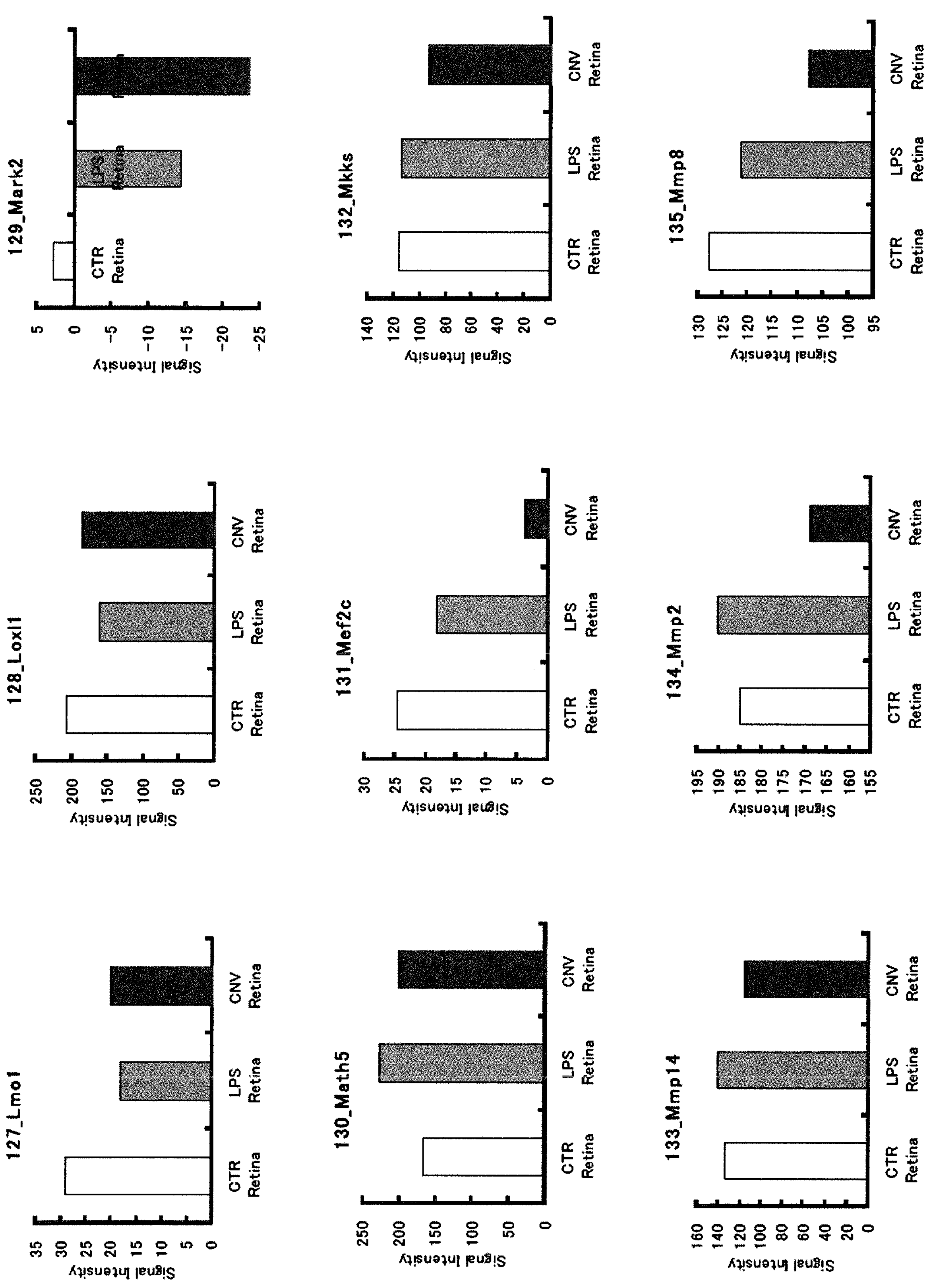
127_Lmo1
128_Loxl1
129_Mark2
130_Math5
131_Mef2c
132_Mkks
133_Mmp14
134_Mmp2
135_Mmp8
Signal Intensity
CTR Retina
LPS Retina
CNV Retina

【Fig. 2H-2】

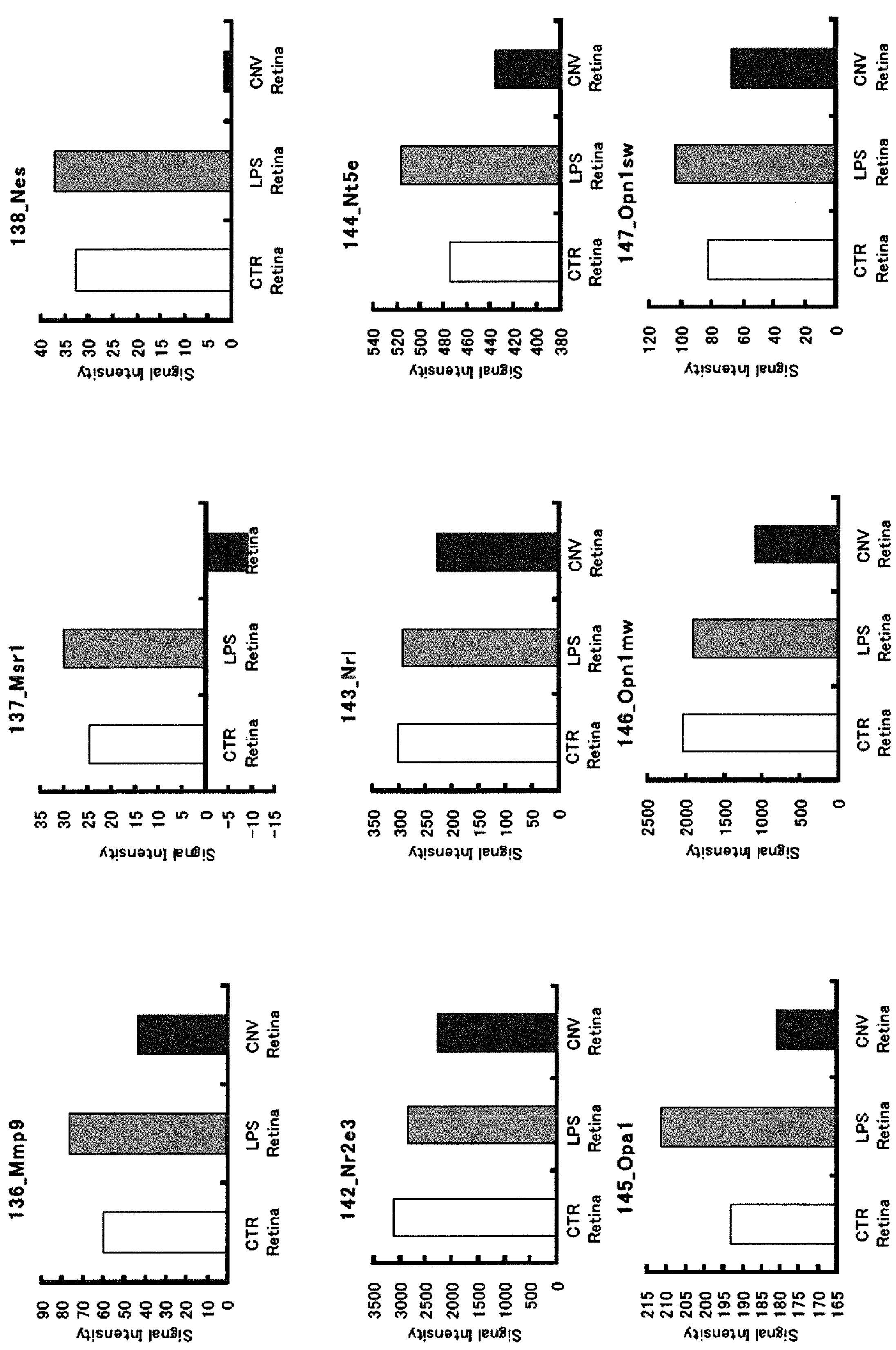
136_Mmp9
137_Msr1
138_Nes
142_Nr2e3
143_Nrl
144_Nt5e
145_Opa1
146_Opn1mw
147_Opn1sw
Signal Intensity
CTR Retina
LPS Retina
CNV Retina

【Fig. 2I-1】

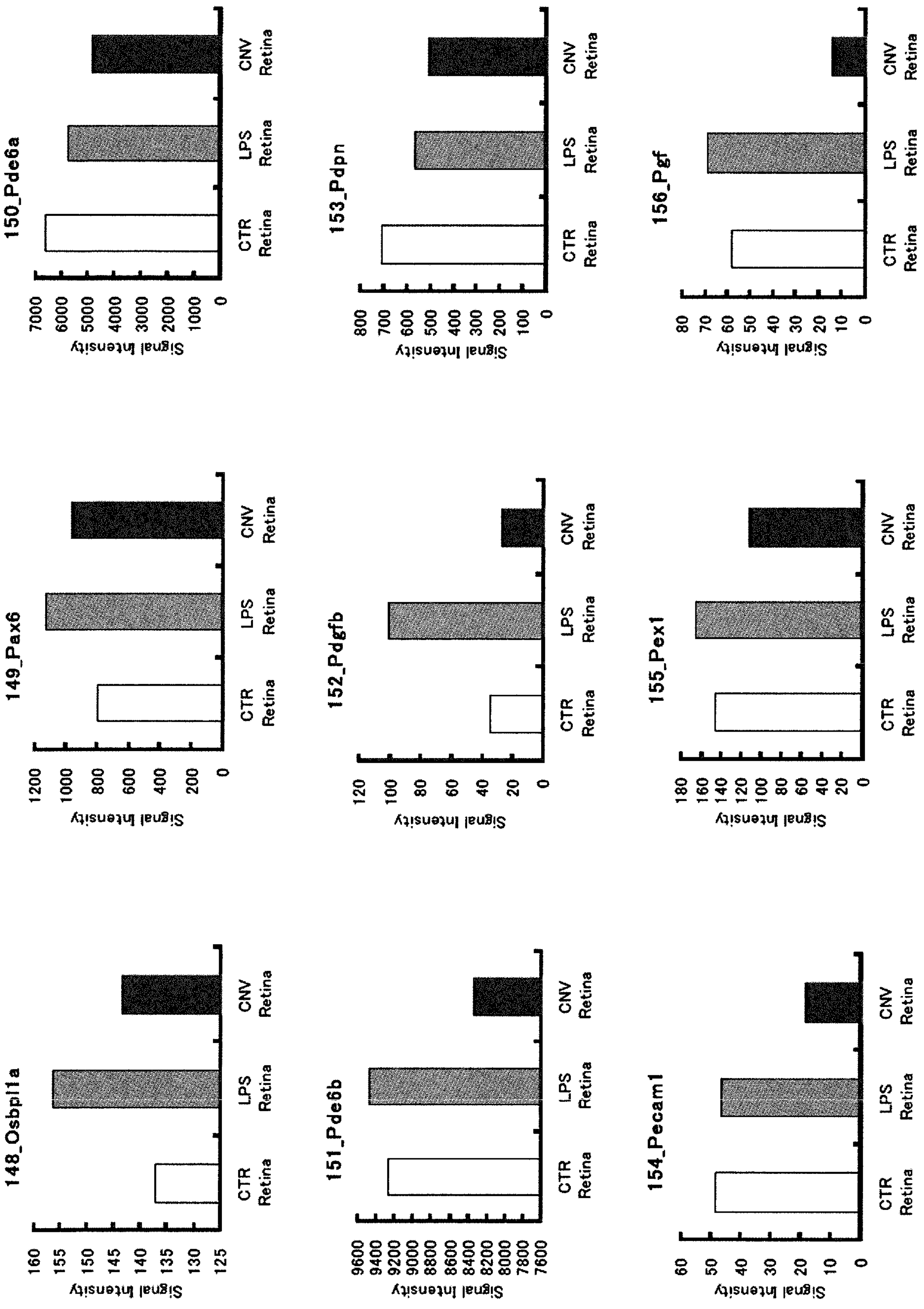
148_Osbpl1a
149_Pax6
150_Pde6a
151_Pde6b
152_Pdgfb
153_Pdpn
154_Pecam1
155_Pex1
156_Pgf
Signal Intensity
CTR Retina
LPS Retina
CNV Retina

【Fig. 2I-2】

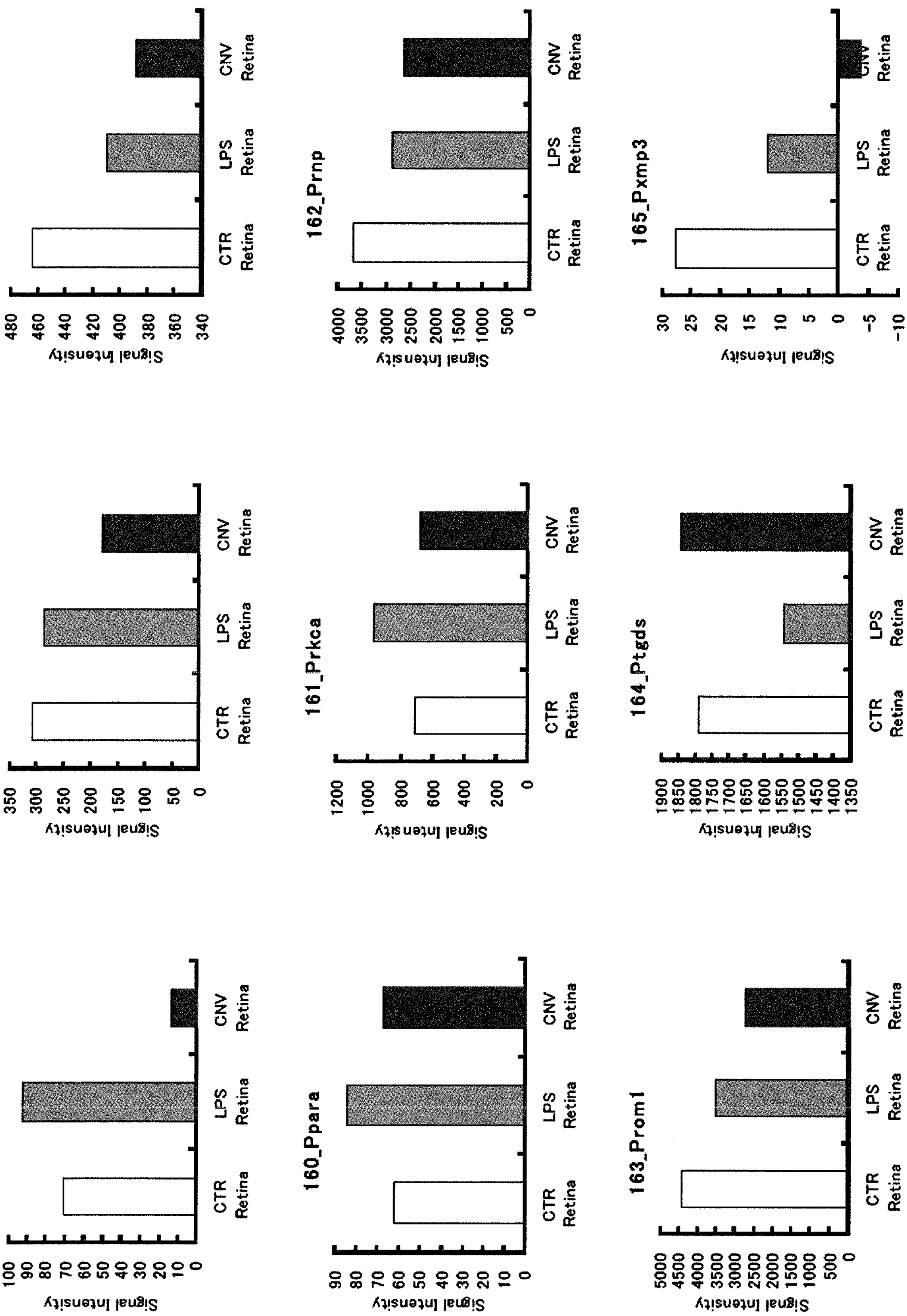
159_Polg2
162_Prnp
165_Pxmp3
158_Pkia
161_Prkca
164_Ptgds
157_Pig7
160_Ppara
163_Prom1
Signal Intensity
CTR Retina
LPS Retina
CNV Retina

【Fig. 2J-1】

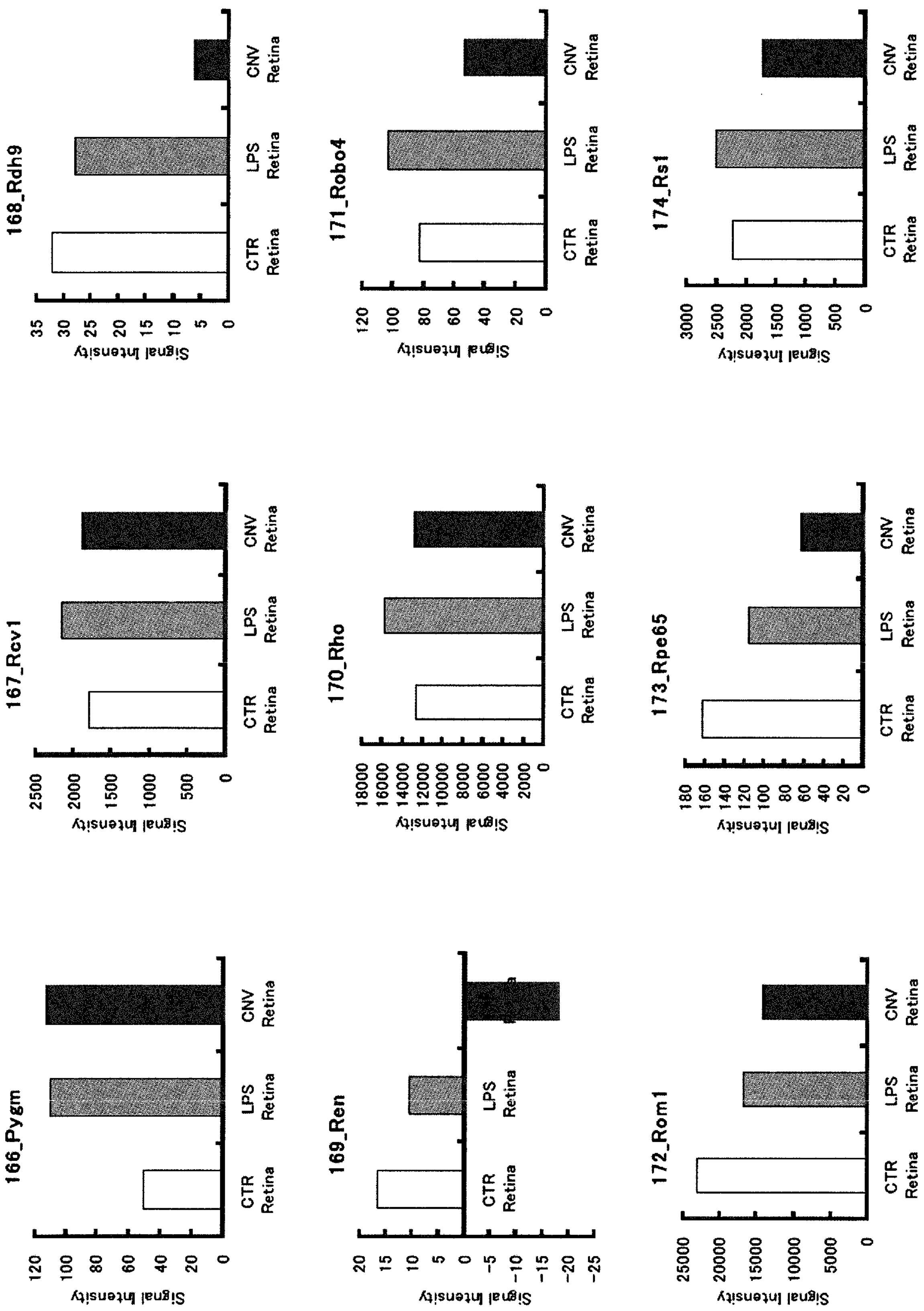
166_Pygm
167_Rcv1
168_Rdh9
169_Ren
170_Rho
171_Robo4
172_Rom1
173_Rpe65
174_Rs1
Signal Intensity
CTR Retina
LPS Retina
CNV Retina

【Fig. 2J-2】

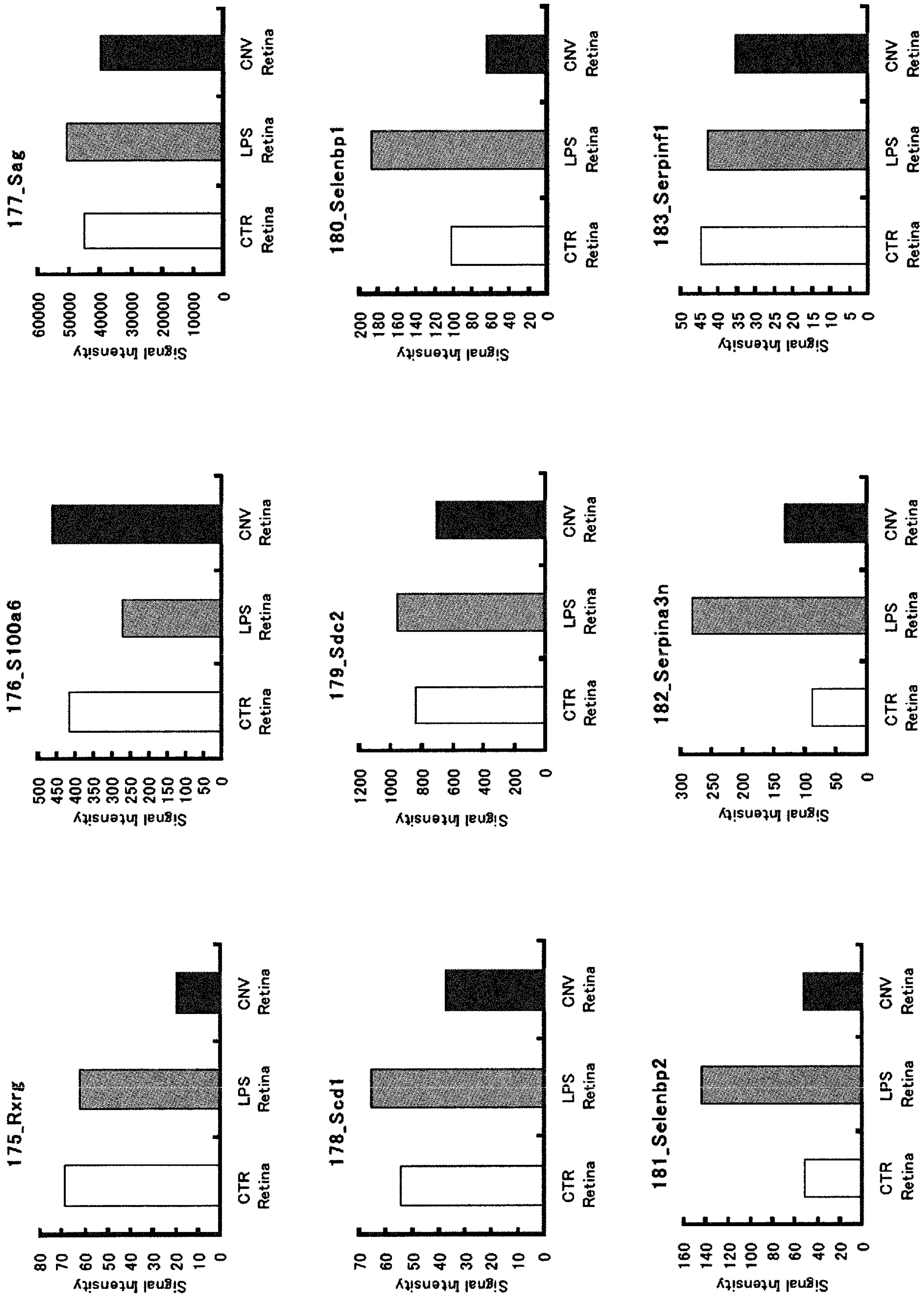
175_Rxrg
176_S100a6
177_Sag
178_Scd1
179_Sdc2
180_Selenbp1
181_Selenbp2
182_Serpina3n
183_Serpinf1
Signal Intensity
CTR Retina
LPS Retina
CNV Retina

【Fig. 2K-1】

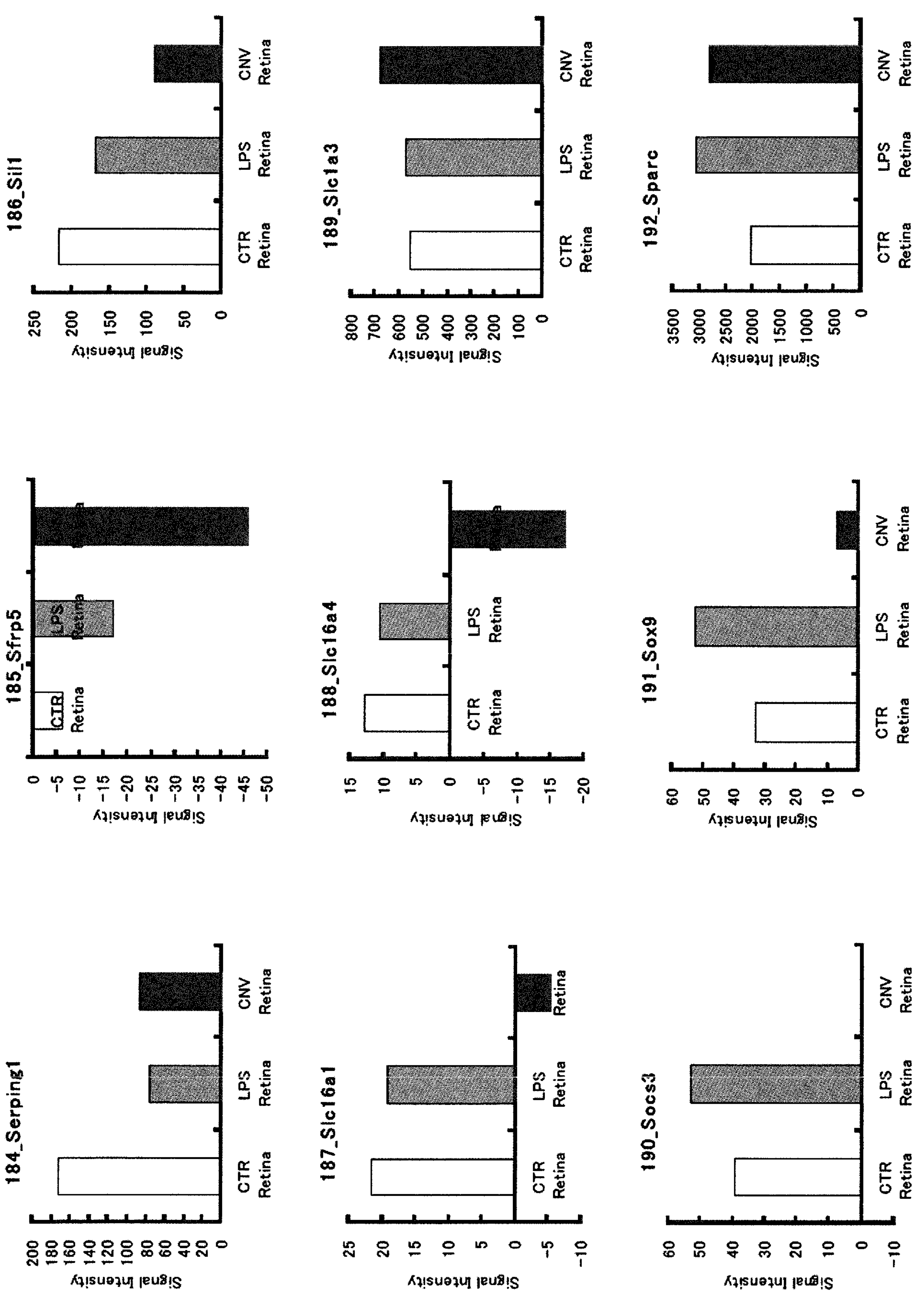
184_Serping1
185_Sfrp5
186_Sil1
187_Slc16a1
188_Slc16a4
189_Slc1a3
190_Socs3
191_Sox9
192_Sparc
Signal Intensity
CTR Retina
LPS Retina
CNV Retina

【Fig. 2K-2】

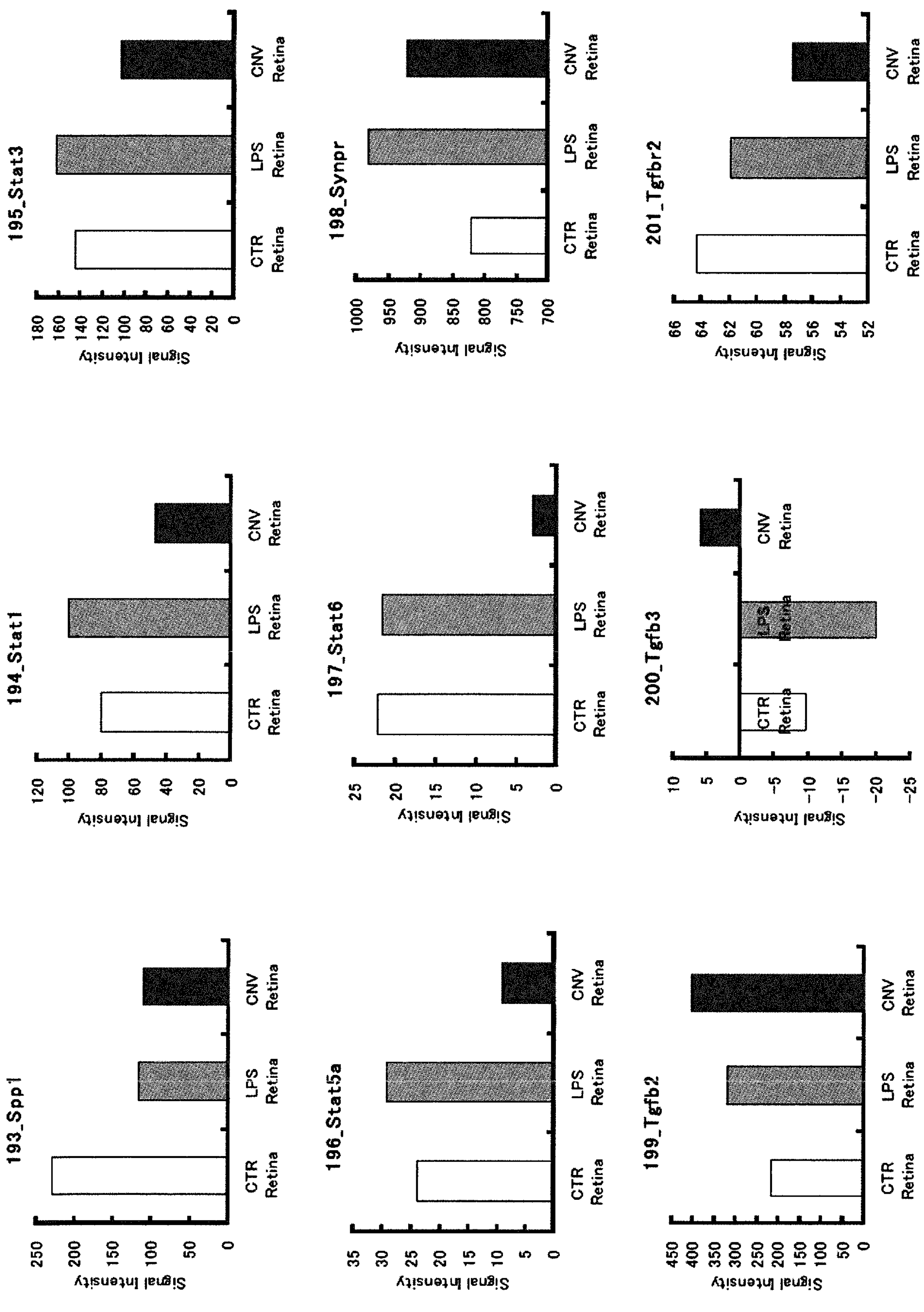
193_Spp1
194_Stat1
195_Stat3
196_Stat5a
197_Stat6
198_Synpr
199_Tgfb2
200_Tgfb3
201_Tgfbr2
Signal Intensity
CTR Retina
LPS Retina
CNV Retina

【Fig. 2L-1】

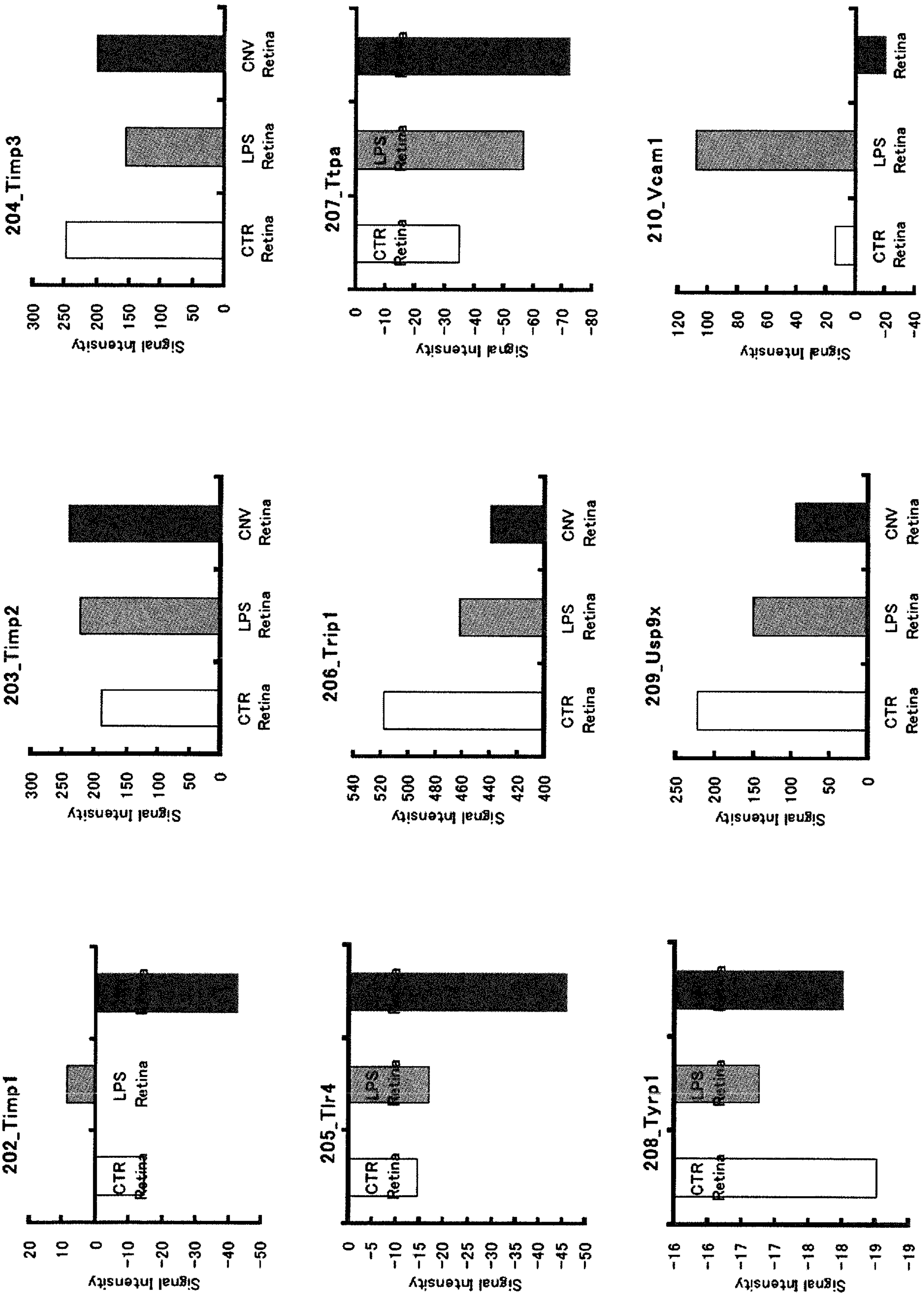
202_Timp1
203_Timp2
204_Timp3
205_Tlr4
206_Trip1
207_Ttpa
208_Tyrp1
209_Usp9x
210_Vcam1
Signal Intensity
CTR Retina
LPS Retina
CNV Retina

【Fig. 2L-2】

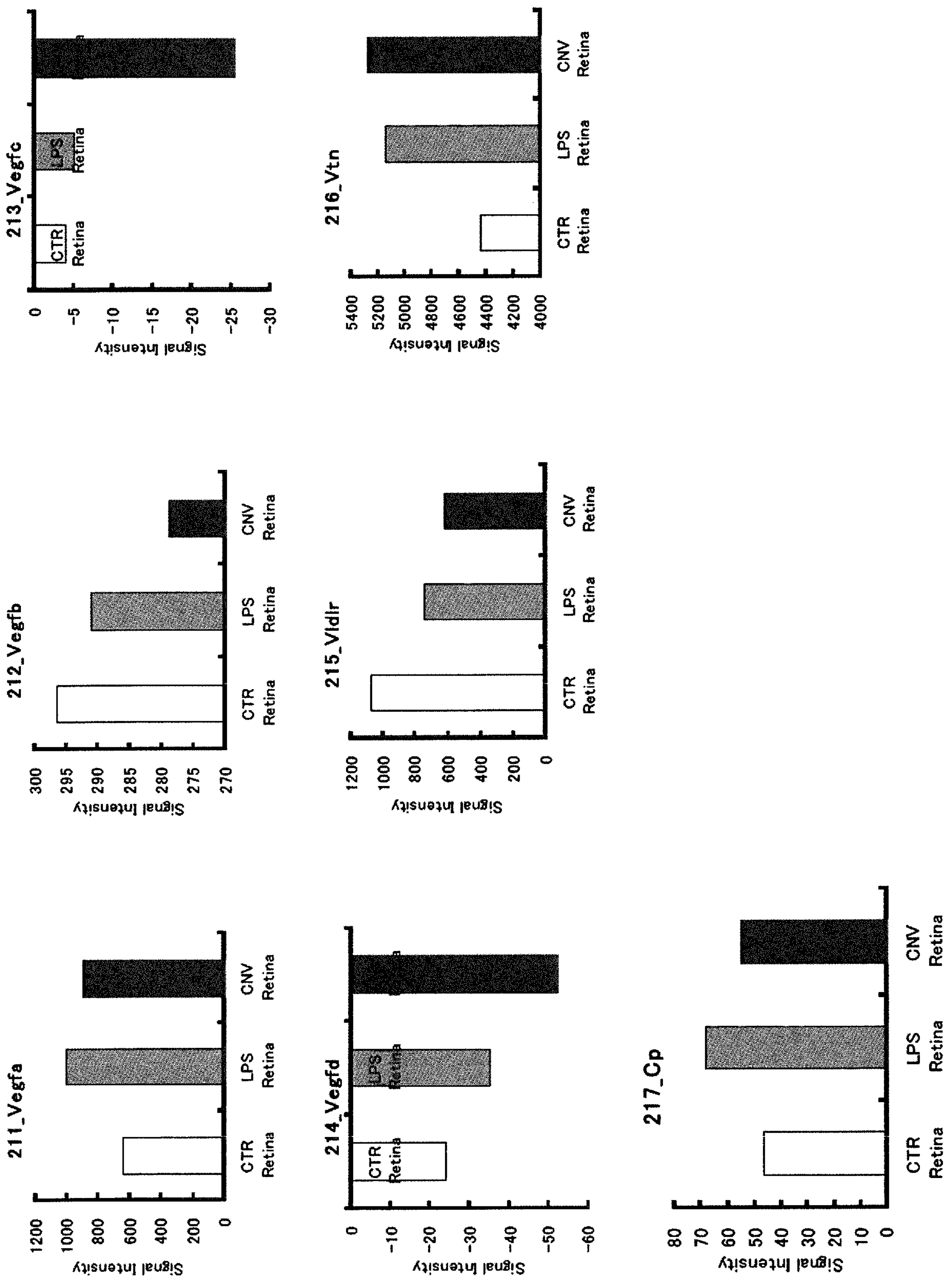
211_Vegfa
212_Vegfb
213_Vegfc
214_Vegfd
215_Vldlr
216_Vtn
217_Cp
Signal Intensity
CTR Retina
LPS Retina
CNV Retina

【Fig. 3A-1】

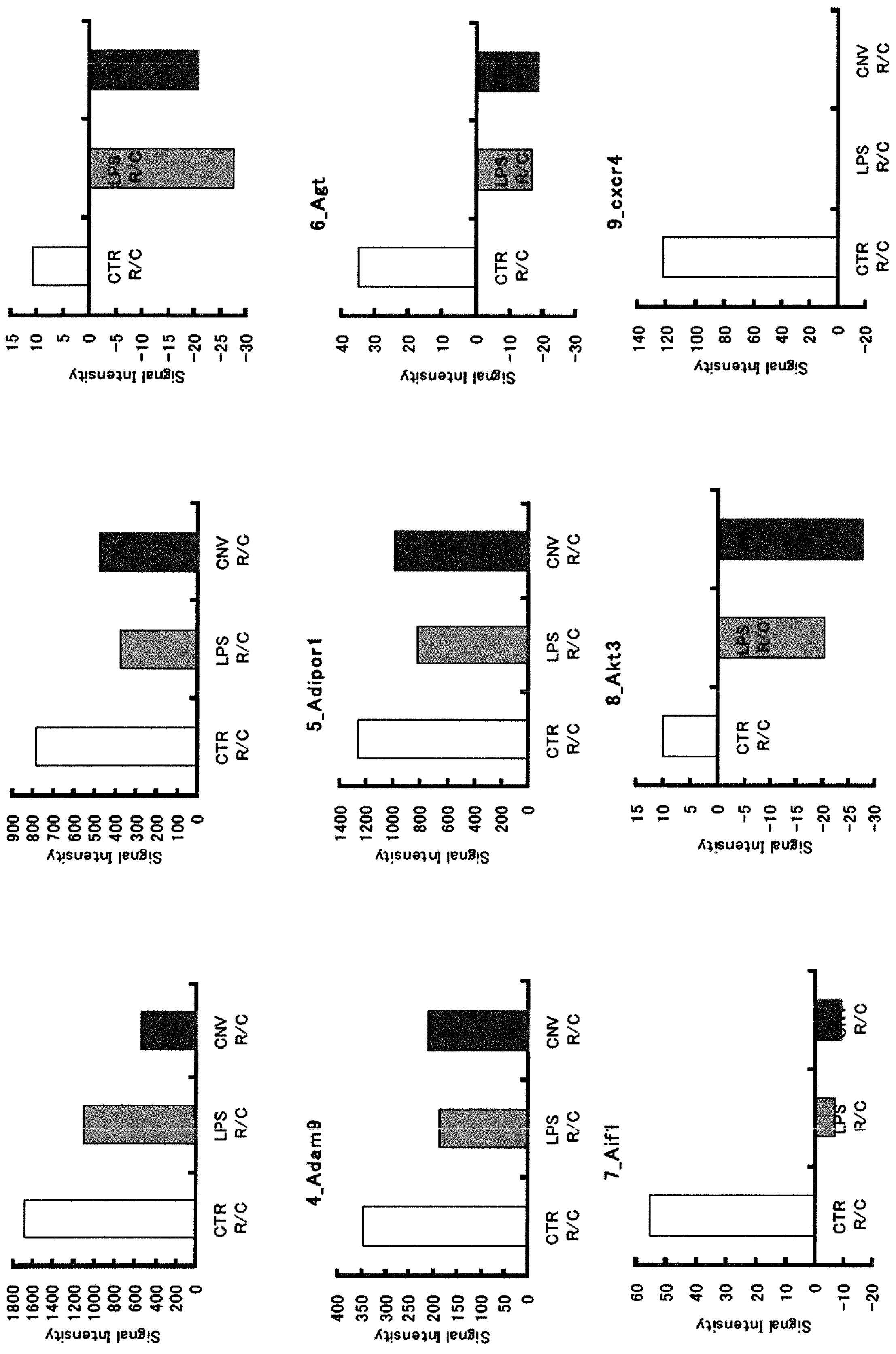
1_A2m
2_Abca4
3_Ace
4_Adam9
5_Adipor1
6_Agt
7_Aif1
8_Akt3
9_cxcr4
Signal Intensity
CTR R/C
LPS R/C
CNV R/C

【Fig. 3A-2】

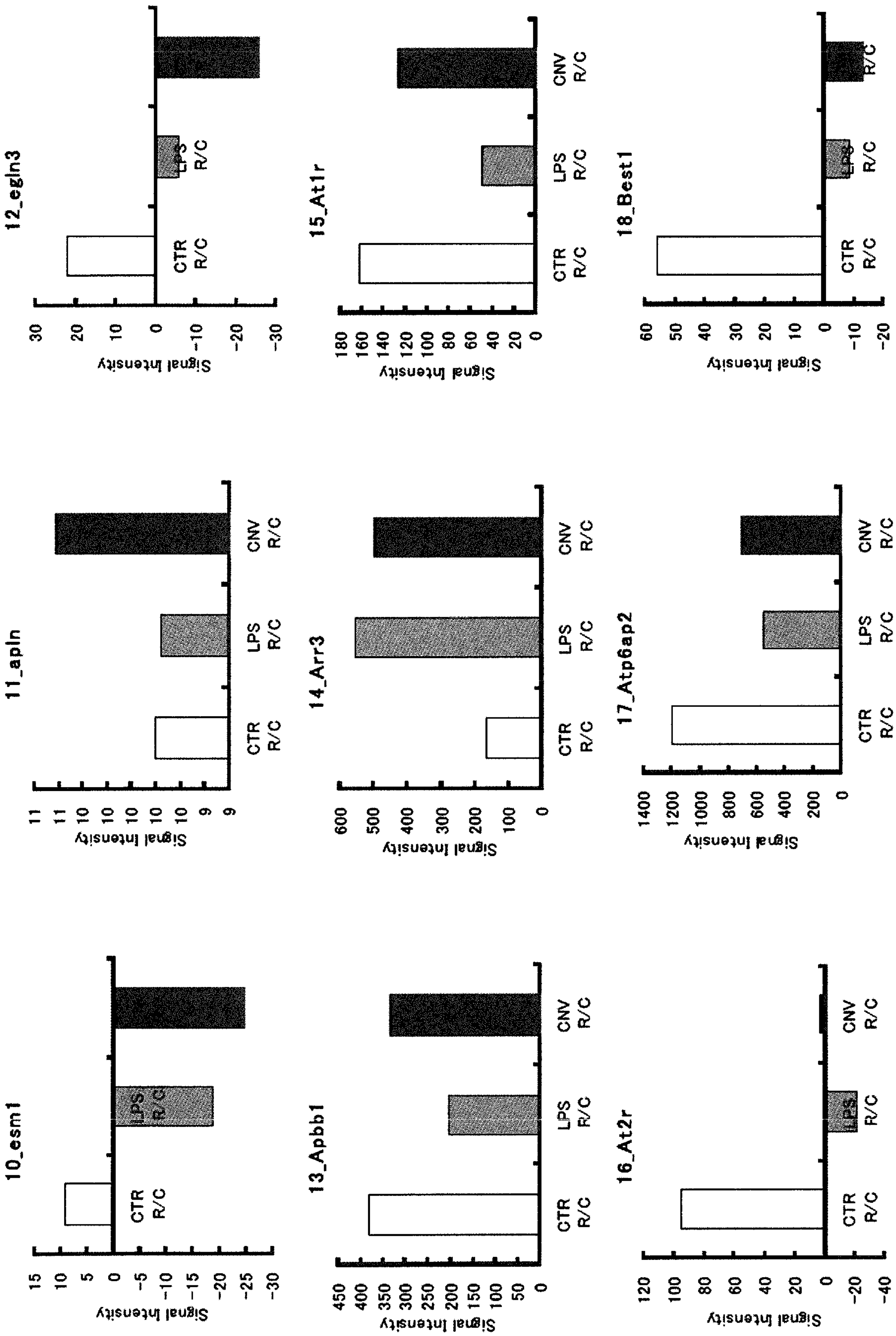
10_esm1
11_apln
12_egln3
13_Apbb1
14_Arr3
15_At1r
16_At2r
17_Atp6ap2
18_Best1
Signal Intensity
CTR R/C
LPS R/C
CNV R/C

【Fig. 3B-1】

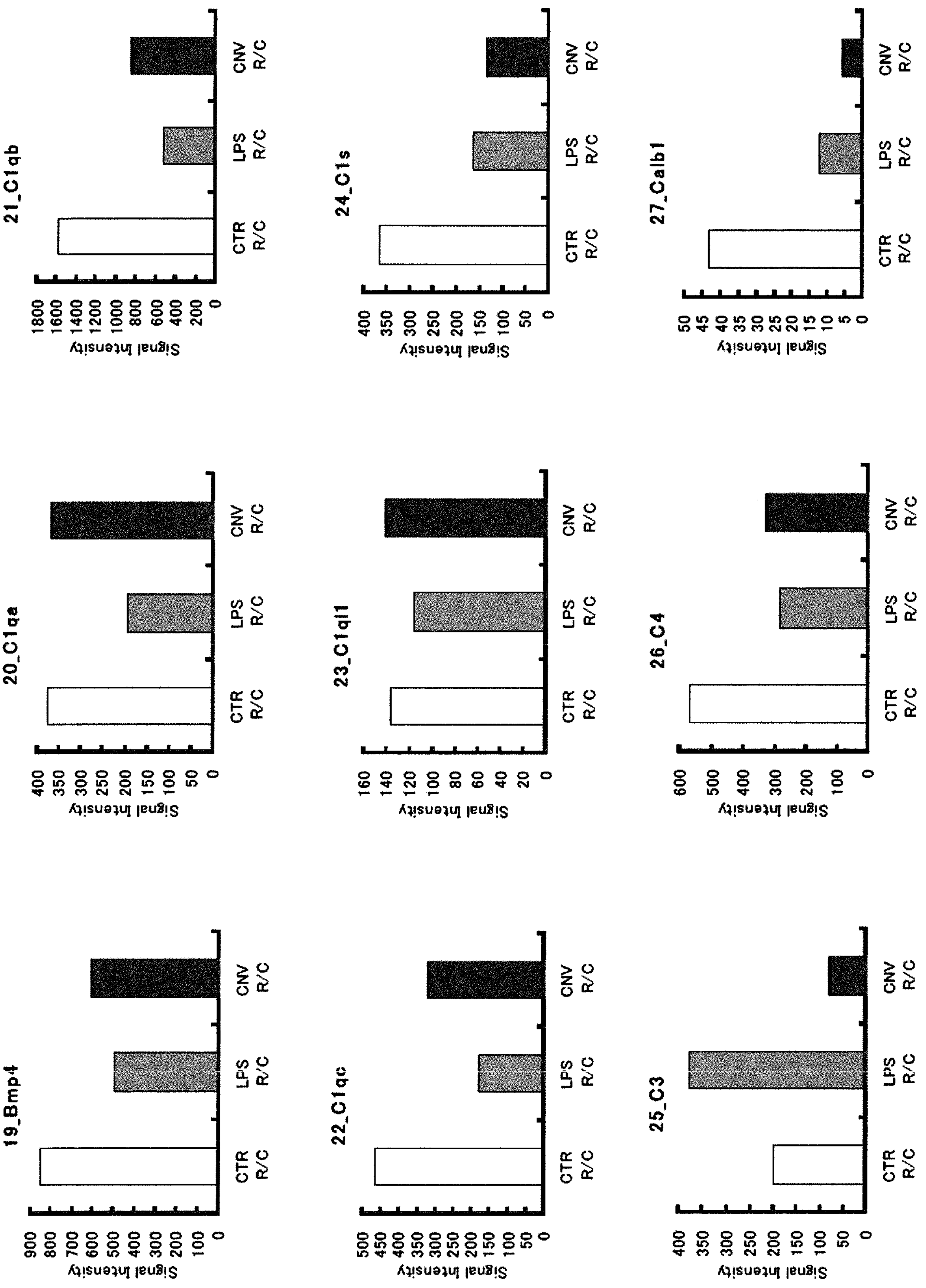
19_Bmp4
20_C1qa
21_C1qb
22_C1qc
23_C1ql1
24_C1s
25_C3
26_C4
27_Calb1
Signal Intensity
CTR R/C
LPS R/C
CNV R/C

【Fig. 3B-2】

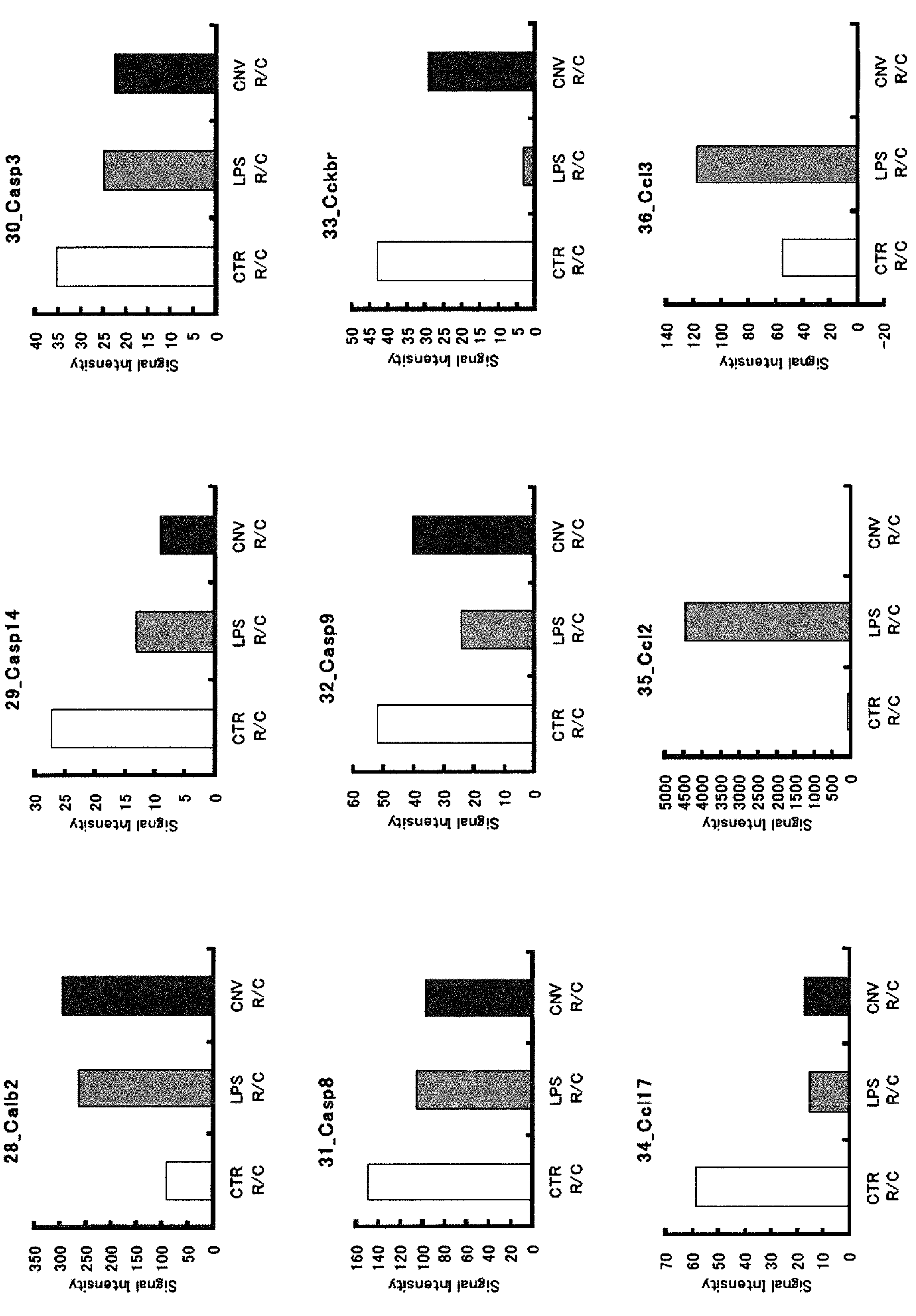
28_Calb2
29_Casp14
30_Casp3
31_Casp8
32_Casp9
33_Cckbr
34_Ccl17
35_Ccl2
36_Ccl3
Signal Intensity
CTR R/C
LPS R/C
CNV R/C

【Fig. 3C-1】

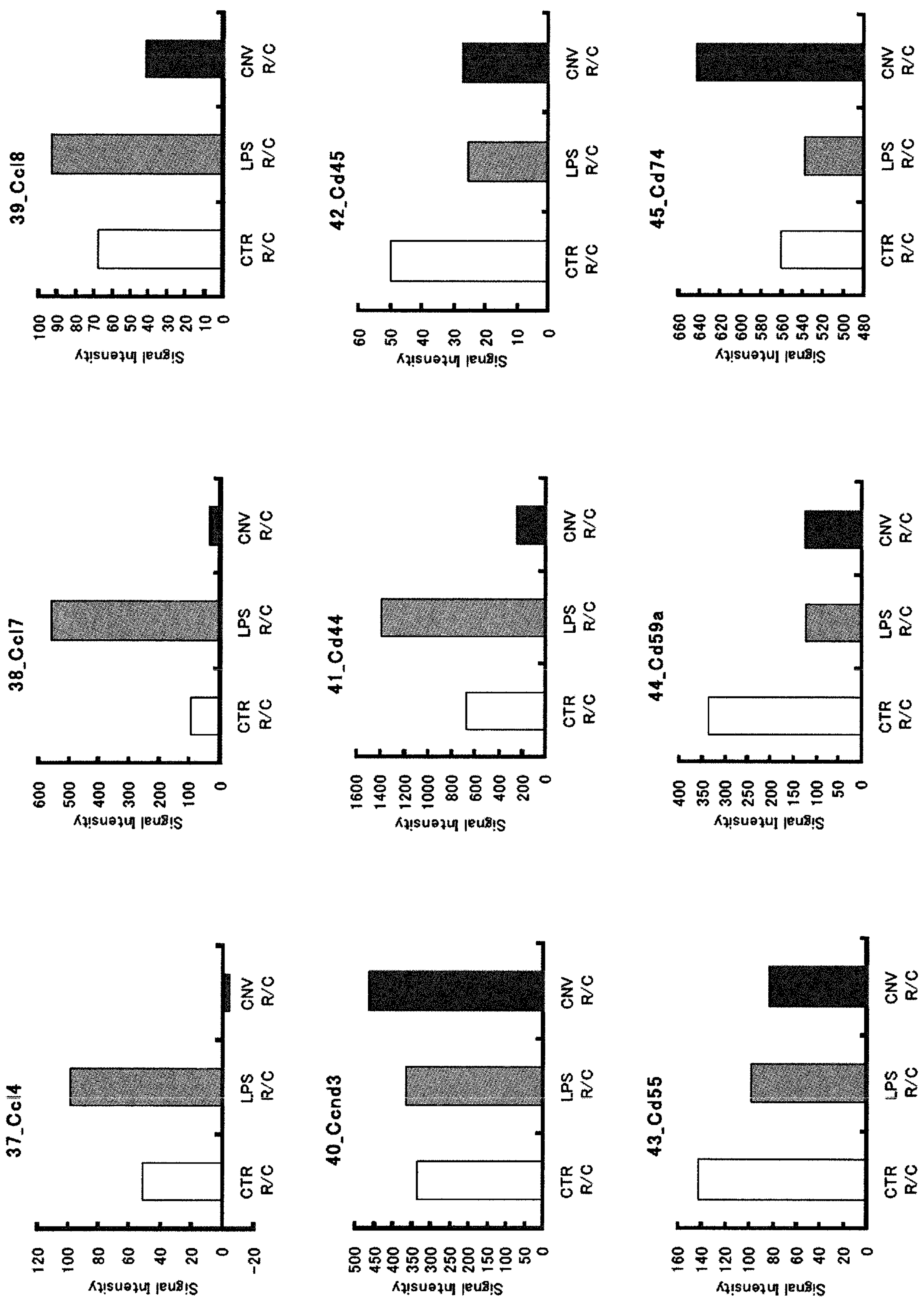
37_Ccl4
38_Ccl7
39_Ccl8
40_Ccnd3
41_Cd44
42_Cd45
43_Cd55
44_Cd59a
45_Cd74
Signal Intensity
CTR R/C
LPS R/C
CNV R/C

【Fig. 3C-2】

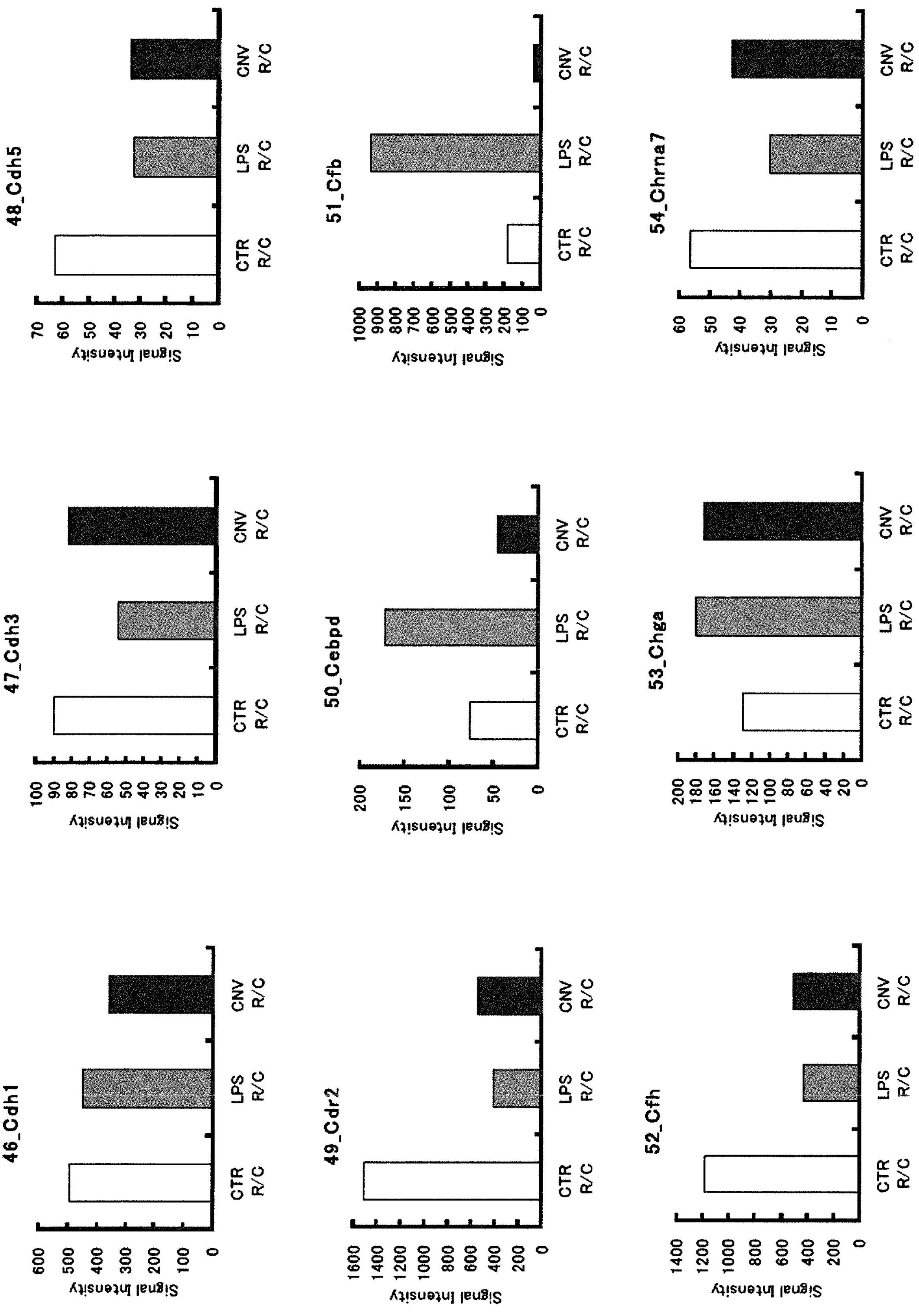
46_Cdh1
47_Cdh3
48_Cdh5
49_Cdr2
50_Cebpd
51_Cfb
52_Cfh
53_Chga
54_Chrna7
Signal Intensity
CTR R/C
LPS R/C
CNV R/C

【Fig. 3D-1】

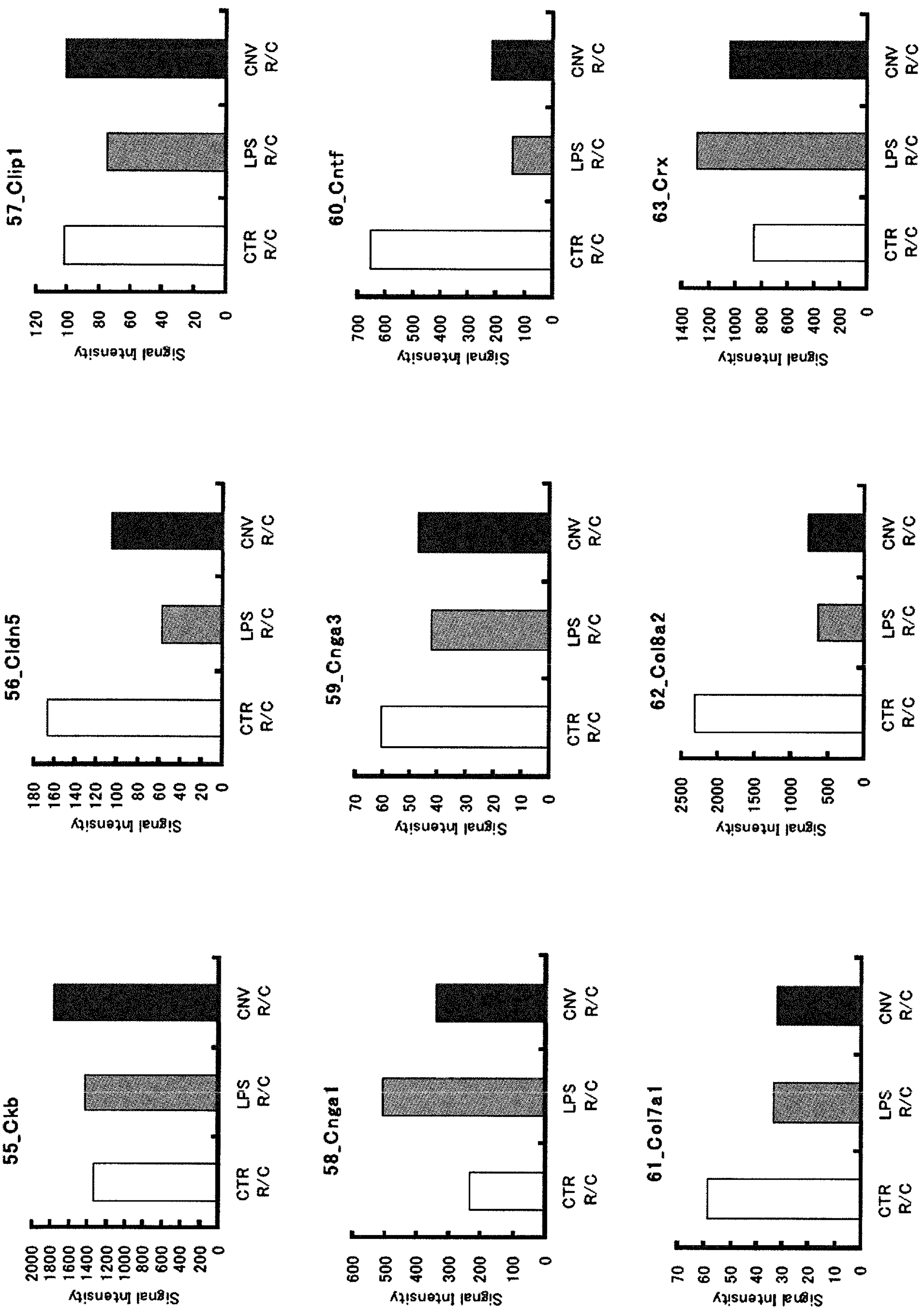
57_Clip1
60_Cntf
63_Crx
56_Cldn5
59_Cnga3
62_Col8a2
55_Ckb
58_Cnga1
61_Col7a1
Signal Intensity
CTR R/C
LPS R/C
CNV R/C

【Fig. 3D-2】

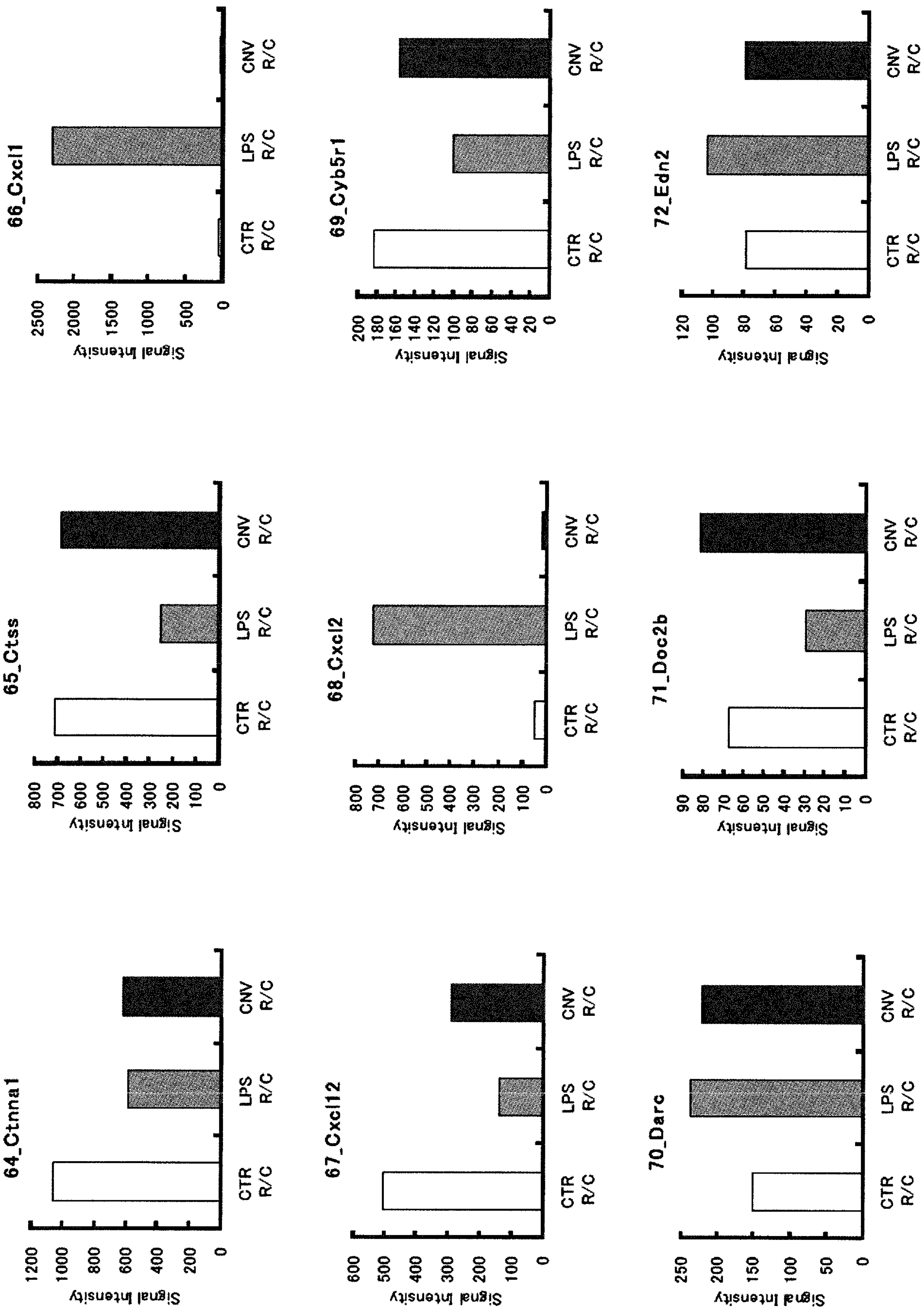
64_Ctnna1
65_Ctss
66_Cxcl1
67_Cxcl12
68_Cxcl2
69_Cyb5r1
70_Darc
71_Doc2b
72_Edn2
Signal Intensity
CTR R/C
LPS R/C
CNV R/C

【Fig. 3E-1】

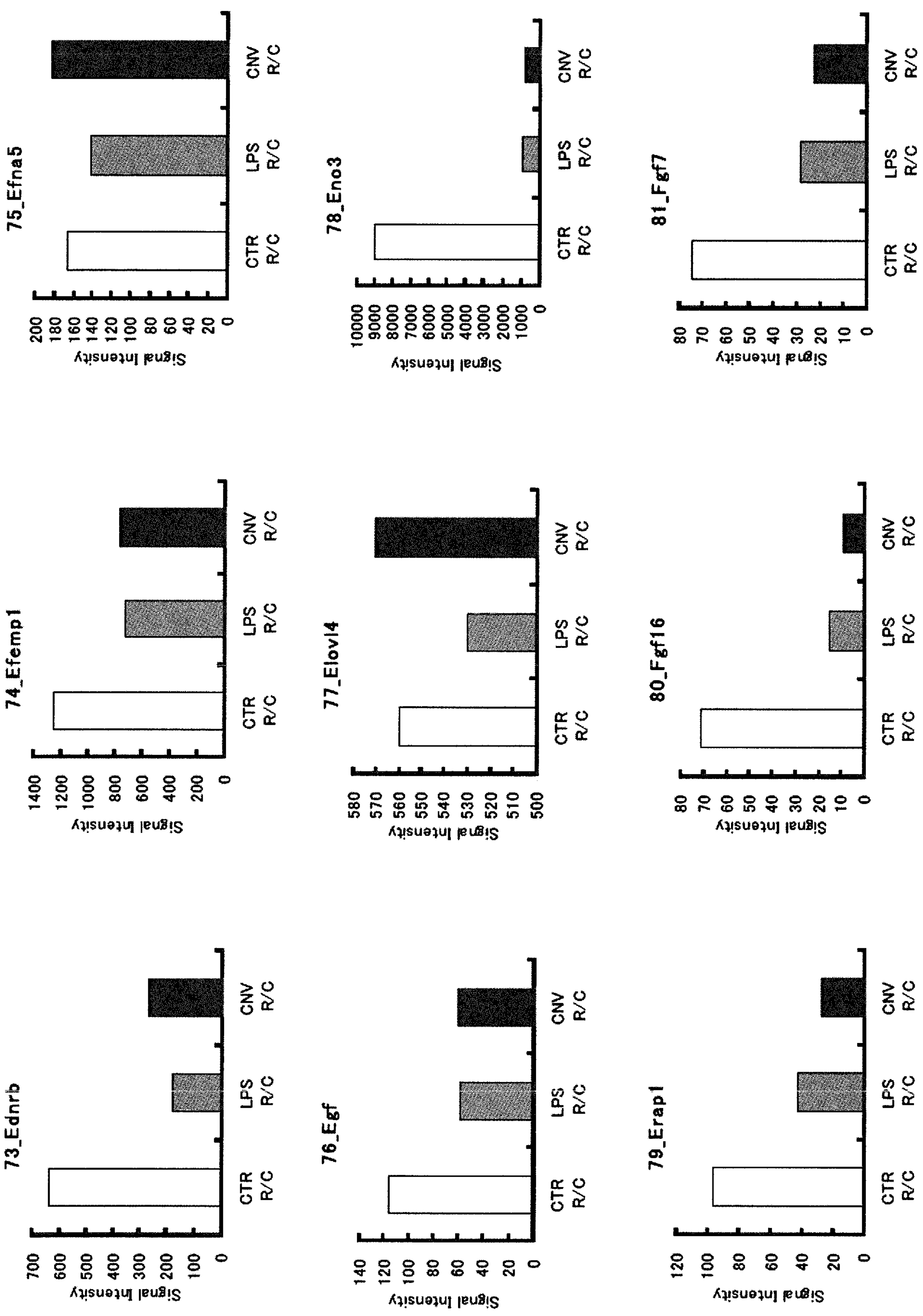
75_Efna5
78_Eno3
81_Fgf7
74_Efemp1
77_Elovl4
80_Fgf16
73_Ednrb
76_Egf
79_Erap1
Signal Intensity
CTR R/C
LPS R/C
CNV R/C

【Fig. 3E-2】

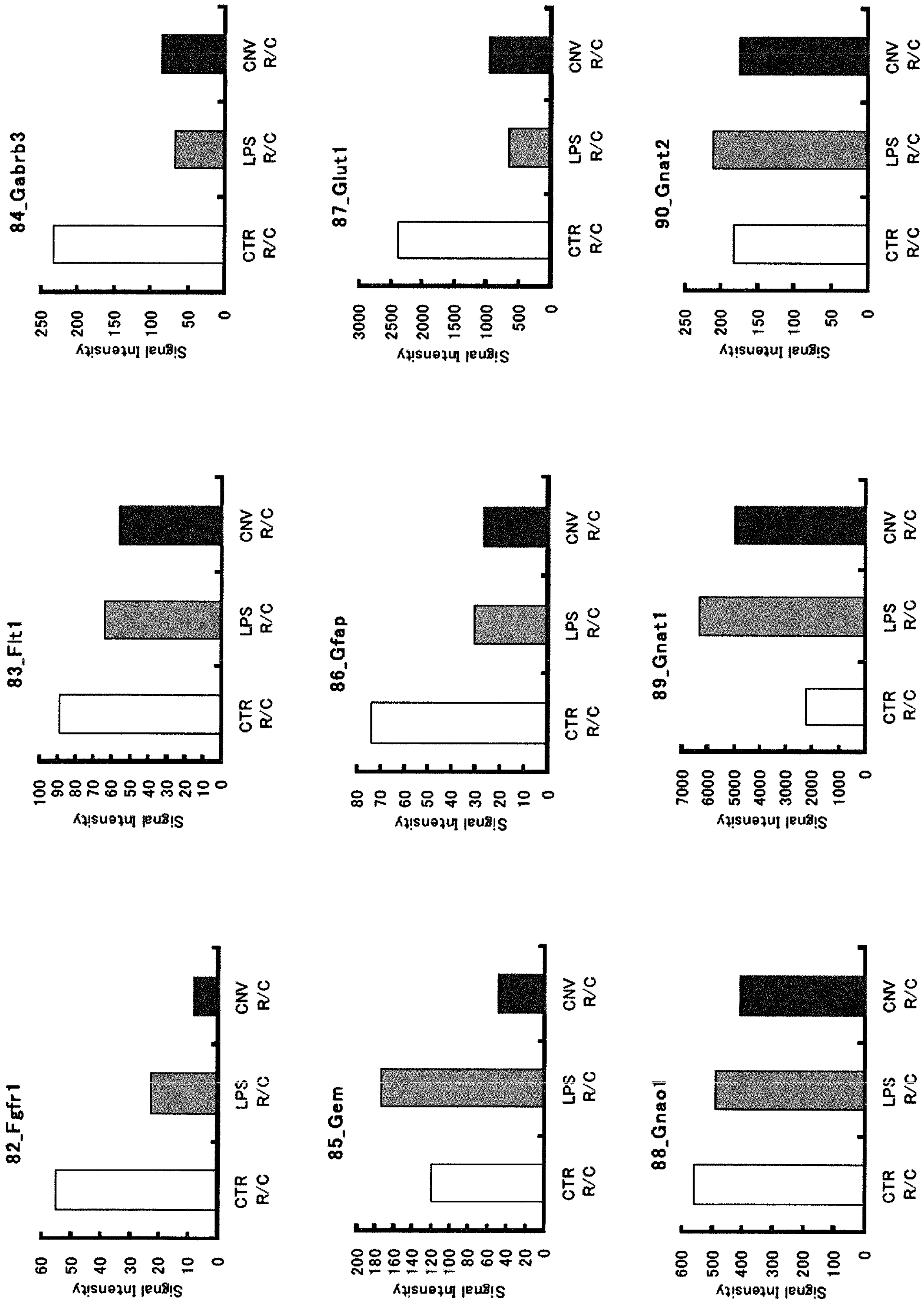
82_Fgfr1
85_Gem
88_Gnao1
83_Flt1
86_Gfap
89_Gnat1
84_Gabrb3
87_Glut1
90_Gnat2
Signal Intensity
CTR R/C
LPS R/C
CNV R/C

【Fig. 3F-1】

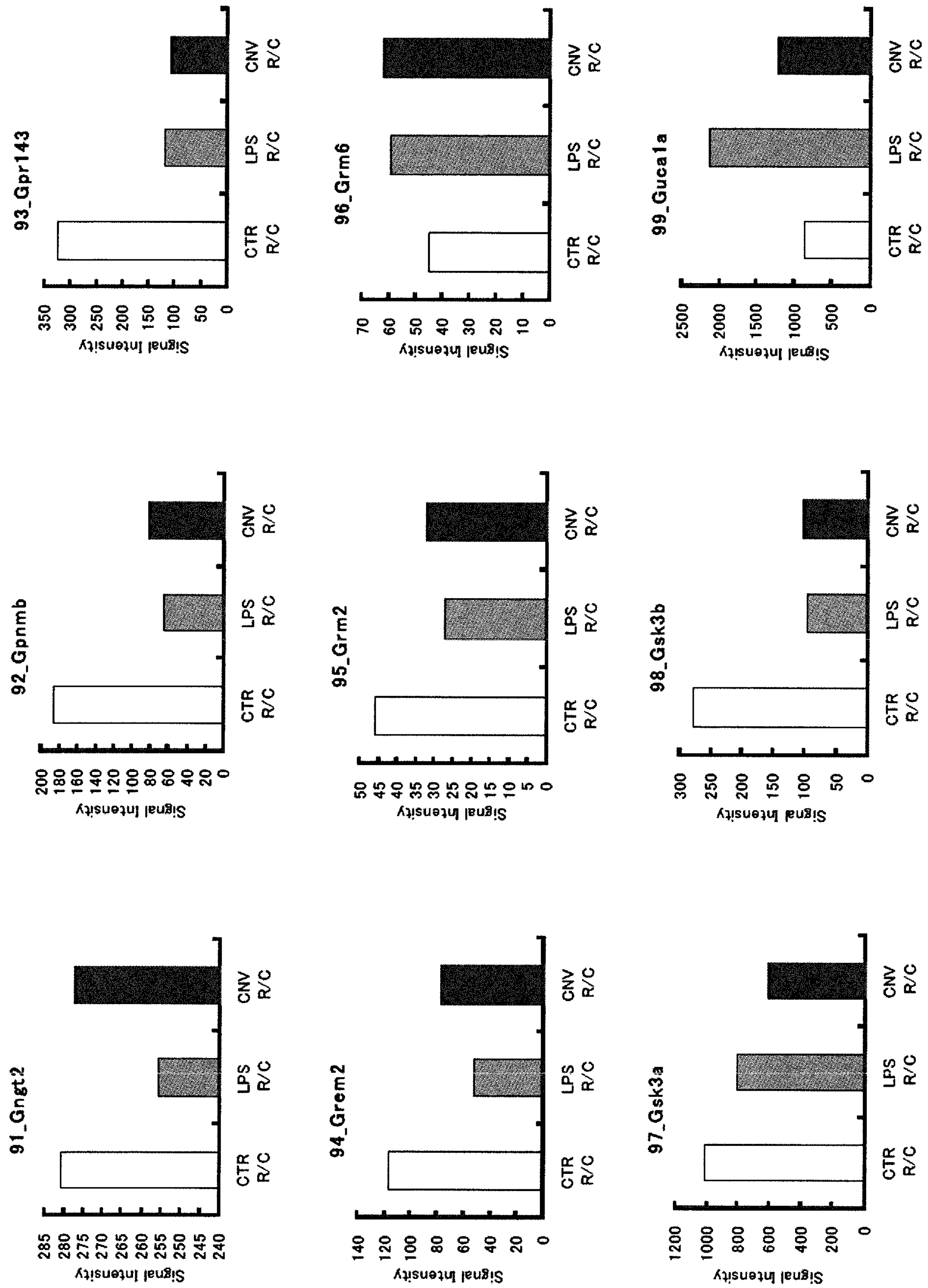
91_Gngt2
92_Gpnmb
93_Gpr143
94_Grem2
95_Grm2
96_Grm6
97_Gsk3a
98_Gsk3b
99_Guca1a
Signal Intensity
CTR R/C
LPS R/C
CNV R/C

【Fig. 3F-2】

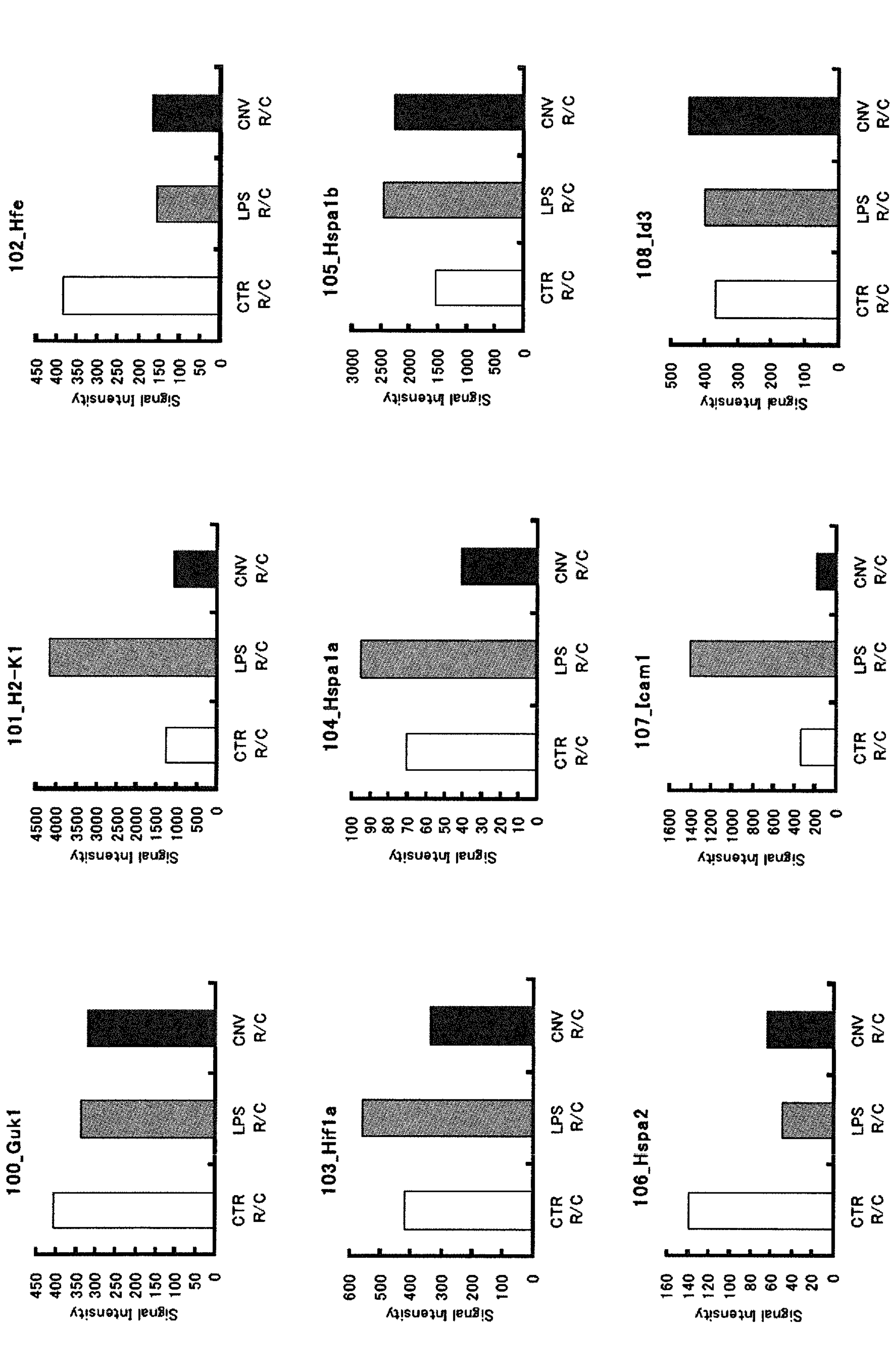
100_Guk1
101_H2-K1
102_Hfe
103_Hif1a
104_Hspa1a
105_Hspa1b
106_Hspa2
107_Icam1
108_Id3
Signal Intensity
CTR R/C
LPS R/C
CNV R/C

【Fig. 3G-1】

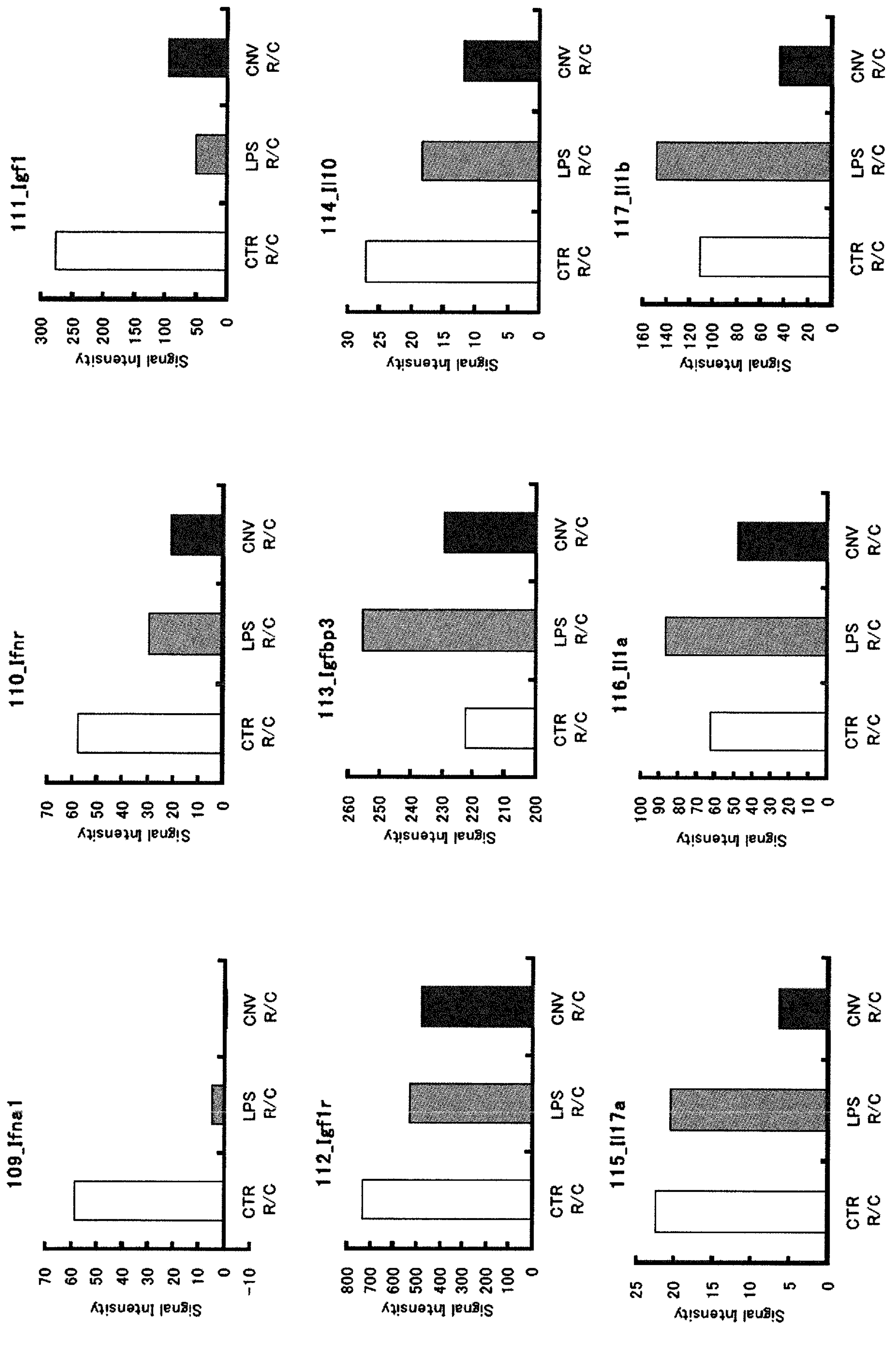
109_Ifna1
110_Ifnr
111_Igf1
112_Igf1r
113_Igfbp3
114_Il10
115_Il17a
116_Il1a
117_Il1b
Signal Intensity
CTR R/C
LPS R/C
CNV R/C

【Fig. 3G-2】

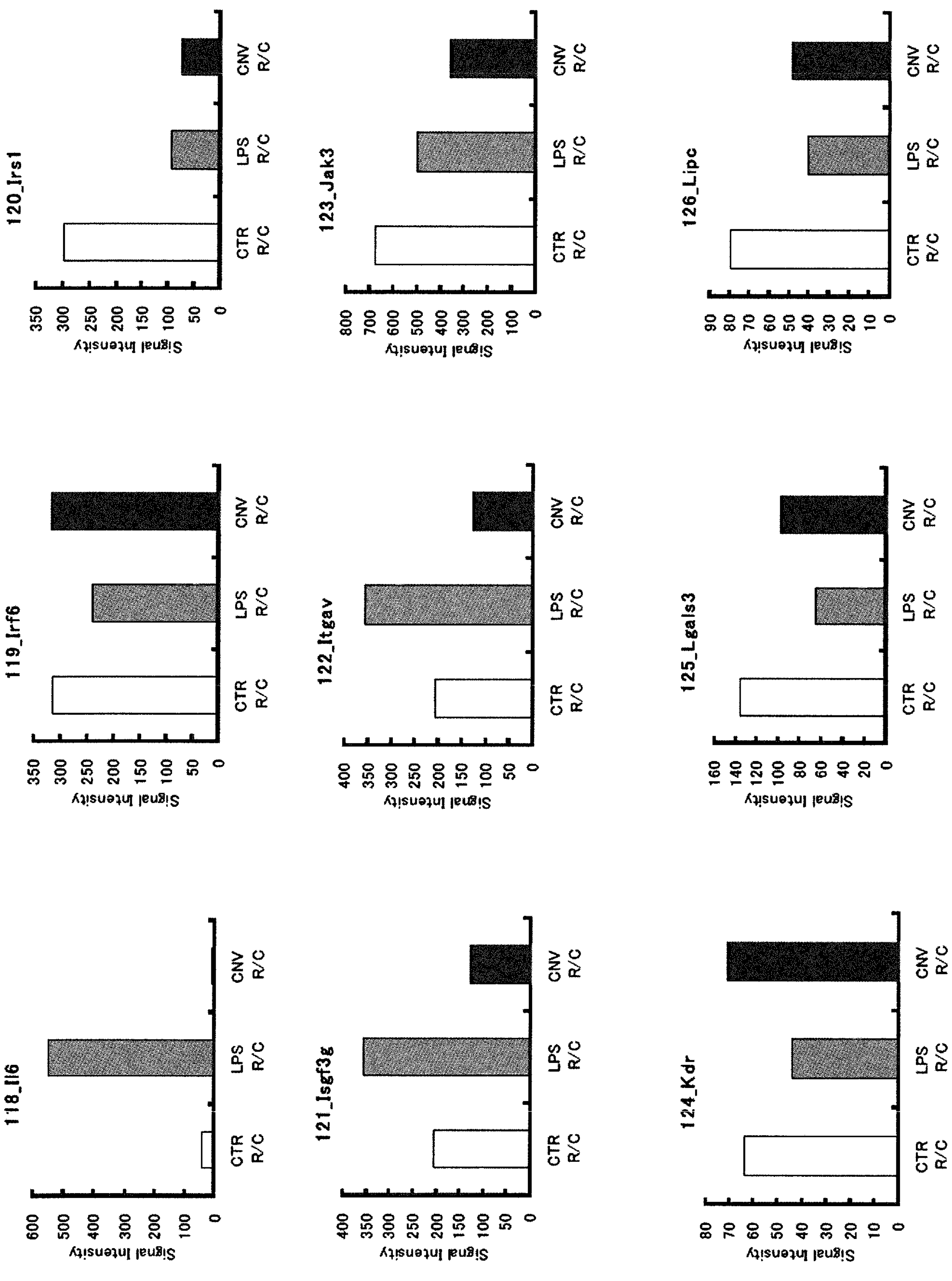
118_Il6
119_Irf6
120_Irs1
121_Isgf3g
122_Itgav
123_Jak3
124_Kdr
125_Lgals3
126_Lipc
Signal Intensity
CTR R/C
LPS R/C
CNV R/C

【Fig. 3H-1】

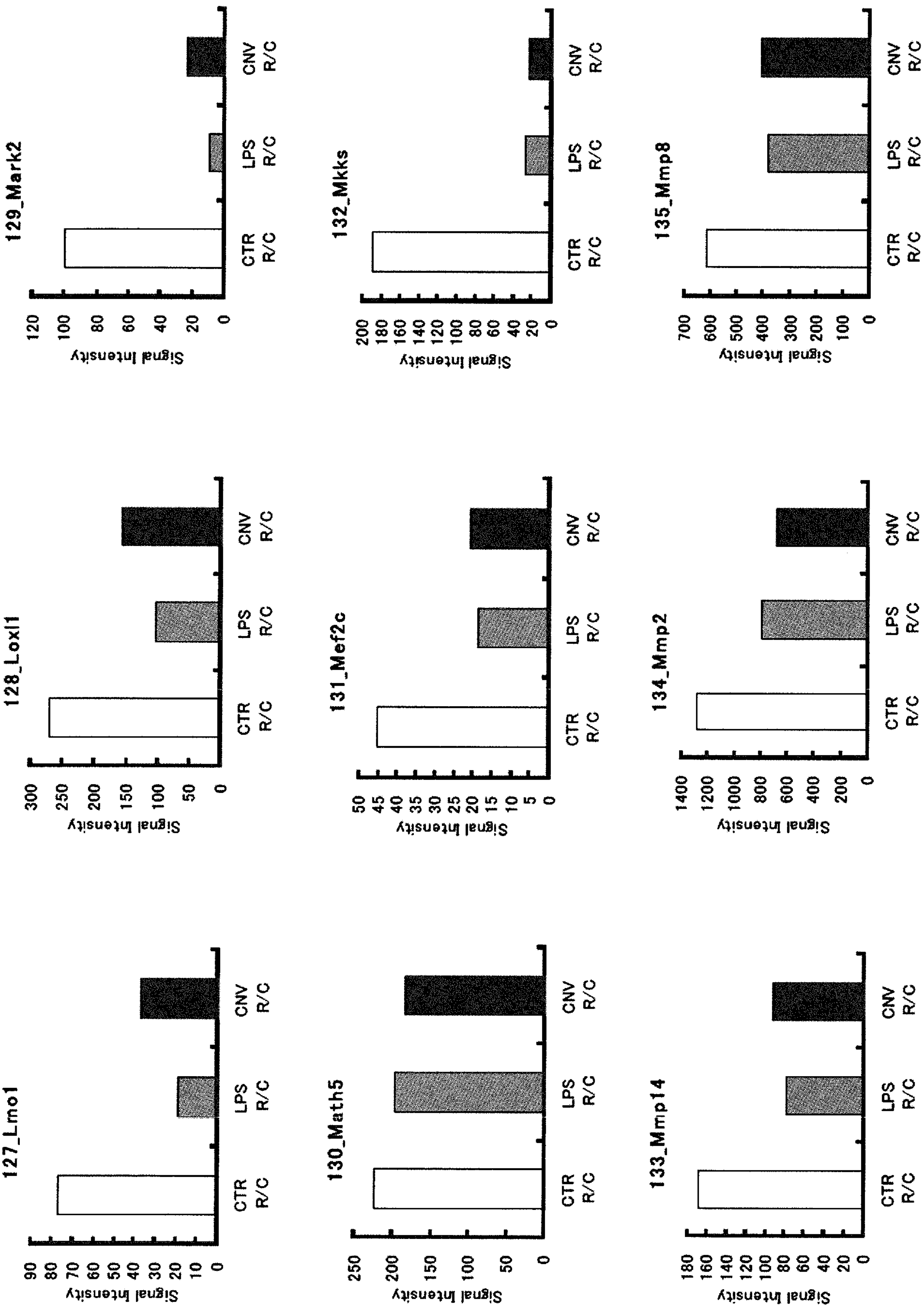
127_Lmo1
128_Loxl1
129_Mark2
130_Math5
131_Mef2c
132_Mkks
133_Mmp14
134_Mmp2
135_Mmp8
Signal Intensity
CTR R/C
LPS R/C
CNV R/C

【Fig. 3H-2】

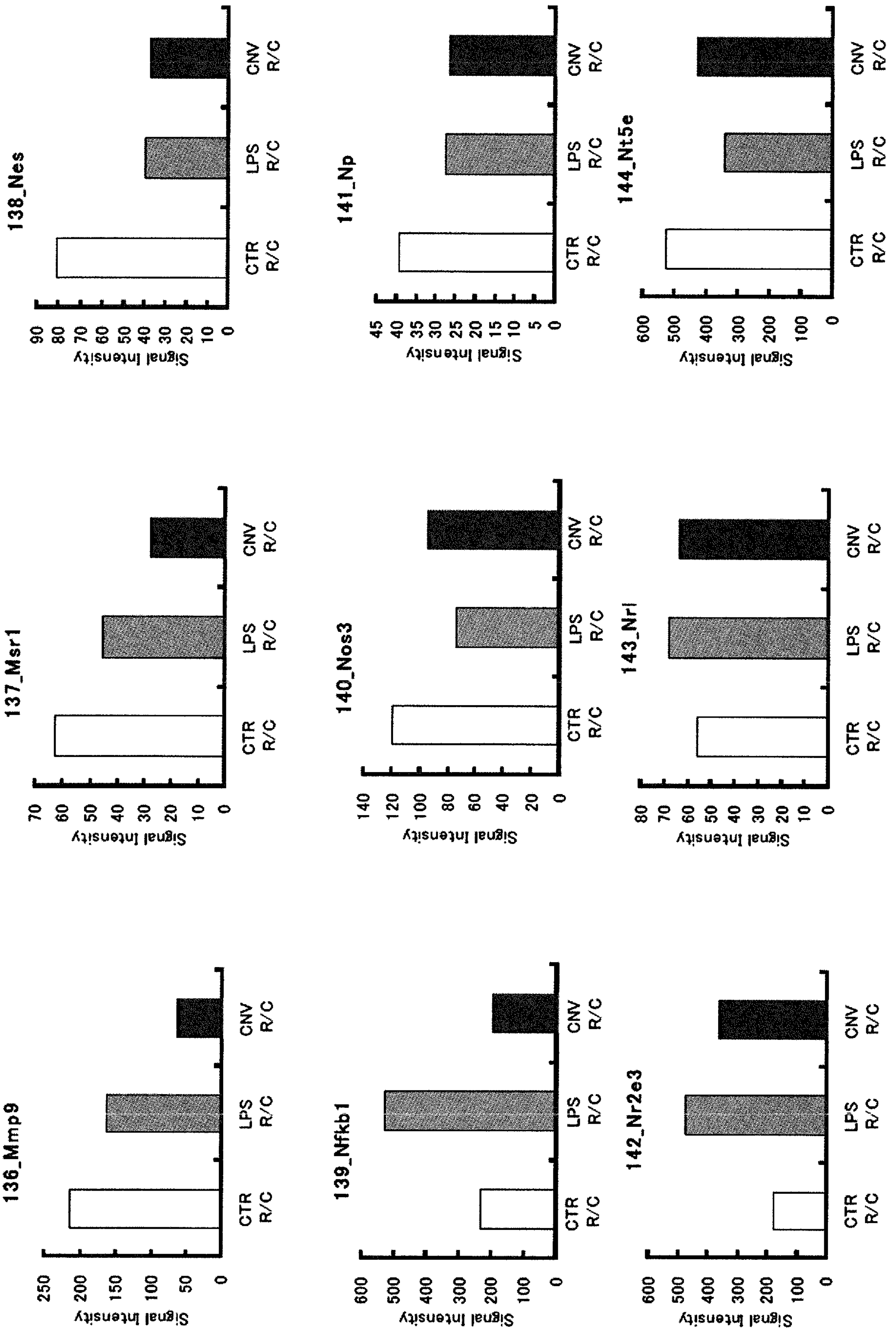
136_Mmp9
137_Msr1
138_Nes
139_Nfkb1
140_Nos3
141_Np
142_Nr2e3
143_Nrl
144_Nt5e
Signal Intensity
CTR R/C
LPS R/C
CNV R/C

【Fig. 3I-1】

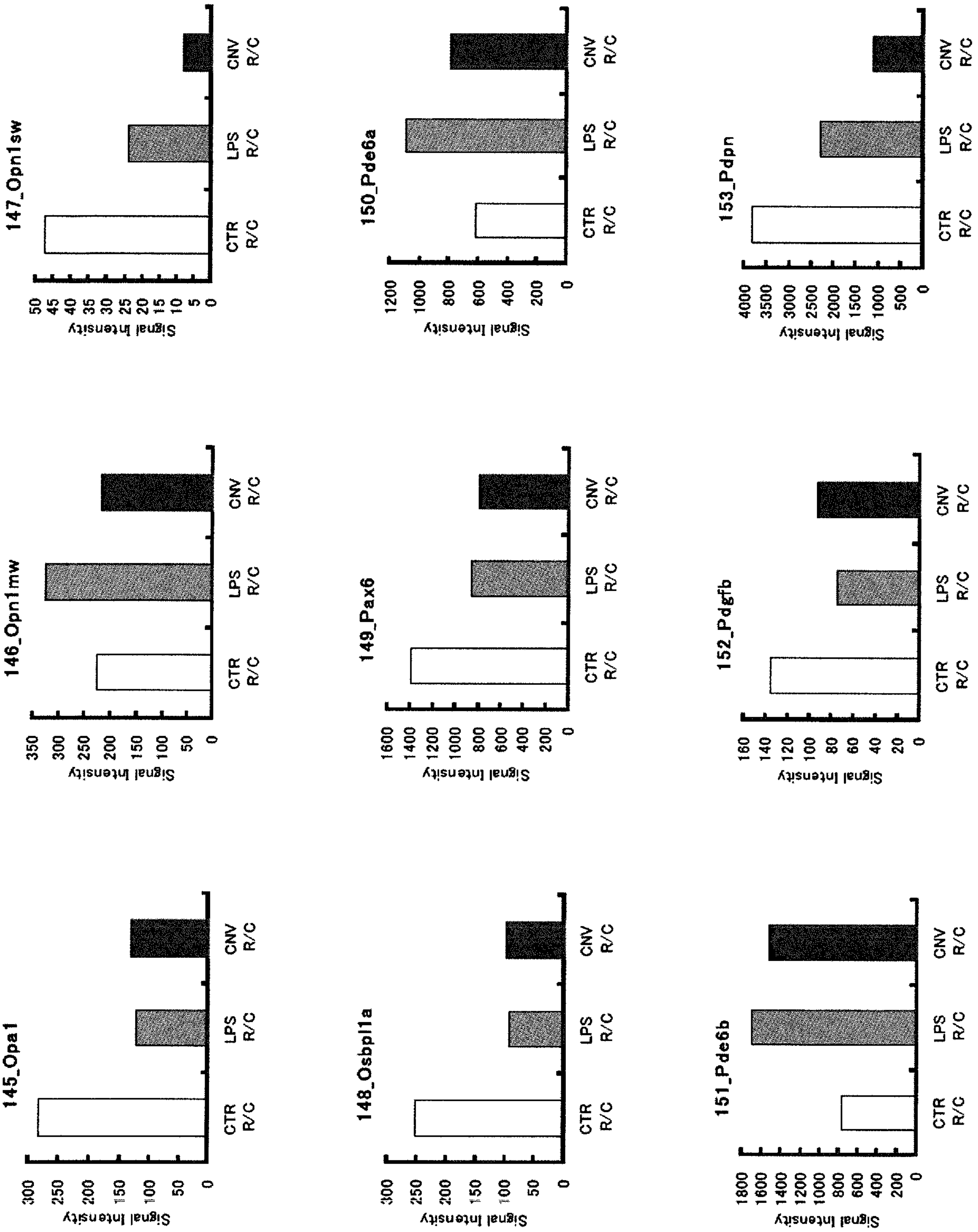
145_Opa1
146_Opn1mw
147_Opn1sw
148_Osbpl1a
149_Pax6
150_Pde6a
151_Pde6b
152_Pdgfb
153_Pdpn
Signal Intensity
CTR R/C
LPS R/C
CNV R/C

【Fig. 3I-2】

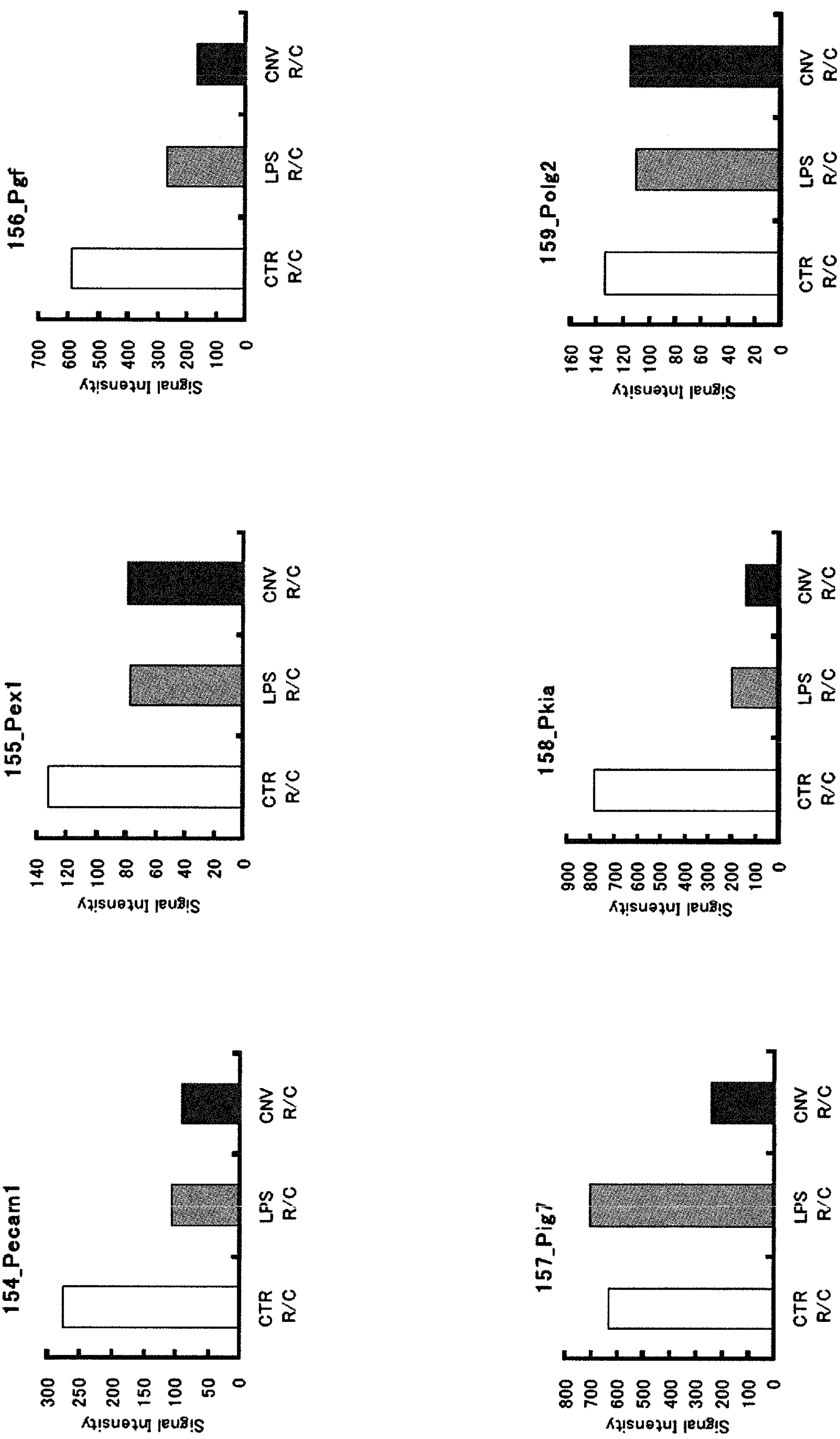
154_Pecam1
155_Pex1
156_Pgf
157_Pig7
158_Pkia
159_Polg2
Signal Intensity
CTR R/C
LPS R/C
CNV R/C

【Fig. 3J-1】

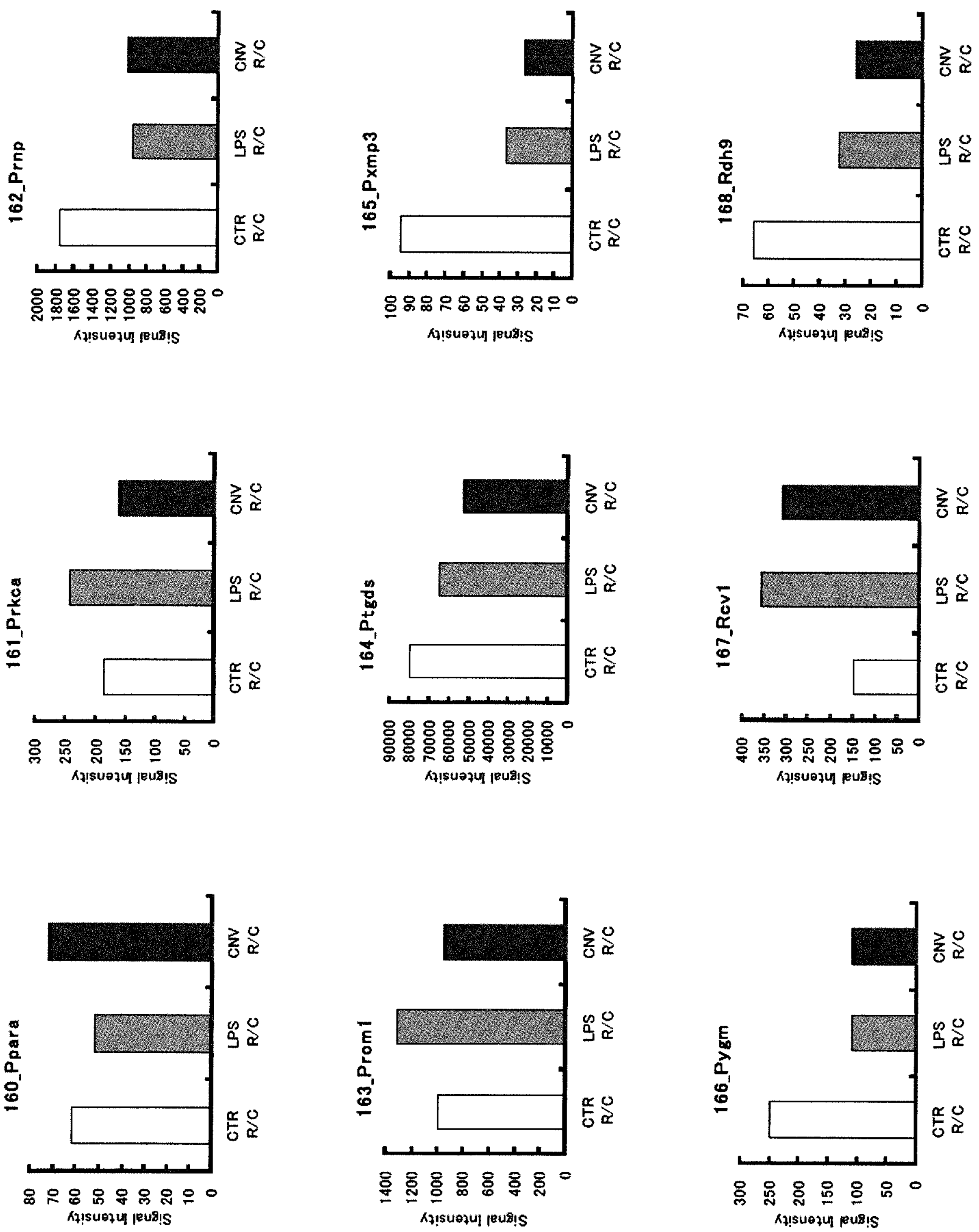
160_Ppara
161_Prkca
162_Prnp
163_Prom1
164_Ptgds
165_Pxmp3
166_Pygm
167_Rcv1
168_Rdh9
Signal Intensity
CTR R/C
LPS R/C
CNV R/C

【Fig. 3J-2】

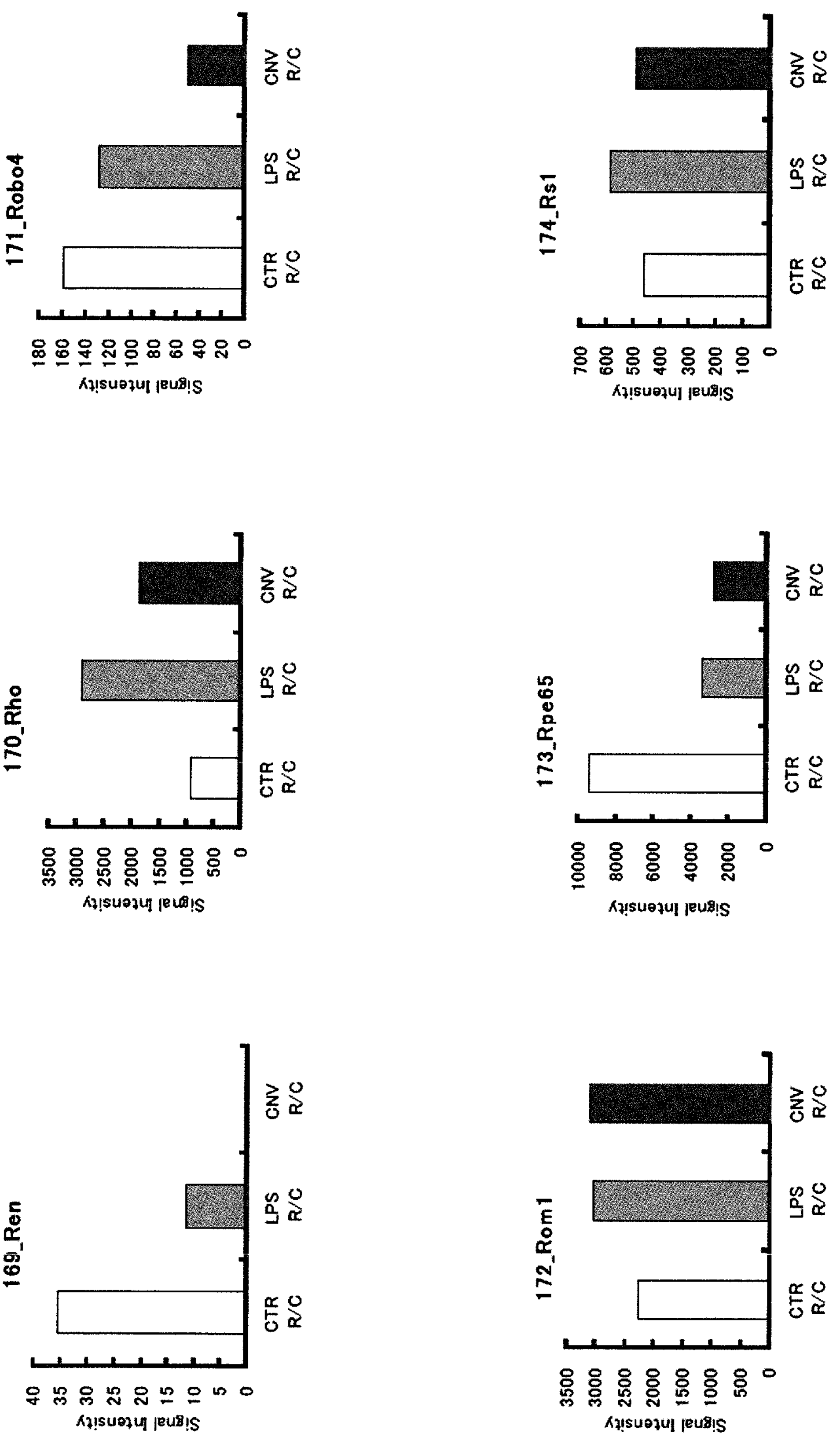
169_Ren
170_Rho
171_Robo4
172_Rom1
173_Rpe65
174_Rs1
Signal Intensity
CTR R/C
LPS R/C
CNV R/C

【Fig. 3K-1】

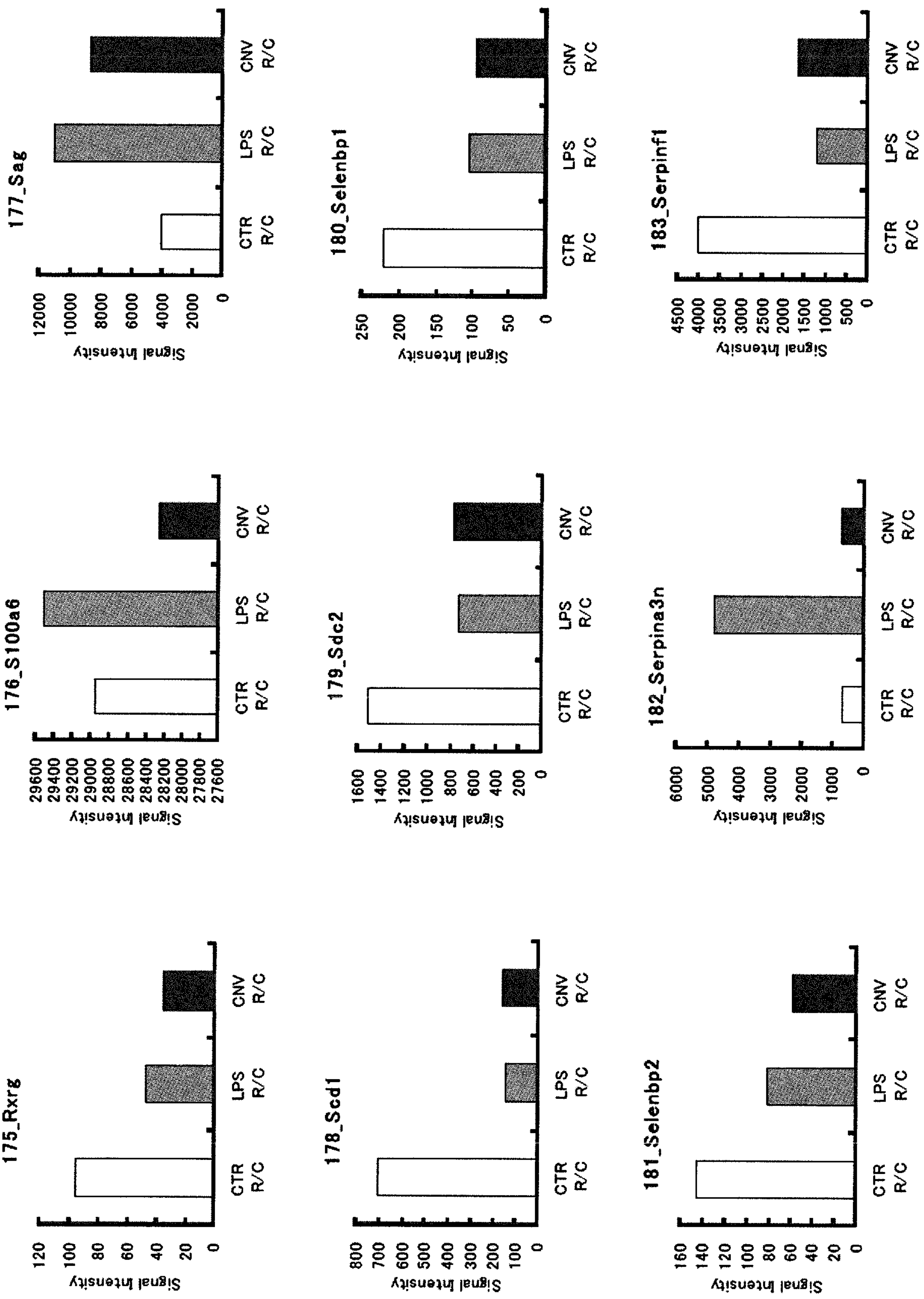
175_Rxrg
176_S100a6
177_Sag
178_Scd1
179_Sdc2
180_Selenbp1
181_Selenbp2
182_Serpina3n
183_Serpinf1
Signal Intensity
CTR R/C
LPS R/C
CNV R/C

【Fig. 3K-2】

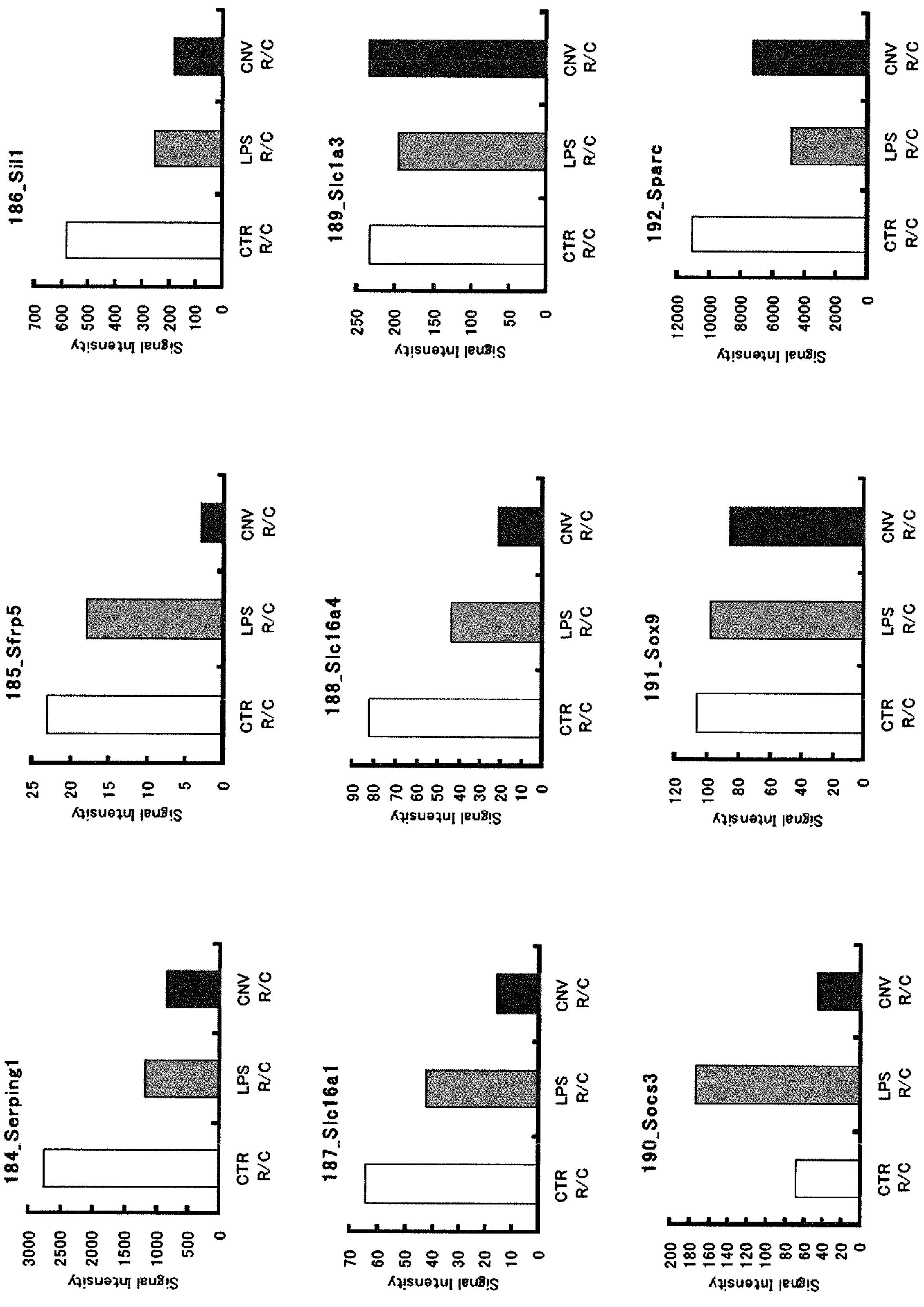
184_Serping1
185_Sfrp5
186_Sil1
187_Slc16a1
188_Slc16a4
189_Slc1a3
190_Socs3
191_Sox9
192_Sparc
Signal Intensity
CTR R/C
LPS R/C
CNV R/C

【Fig. 3L-1】

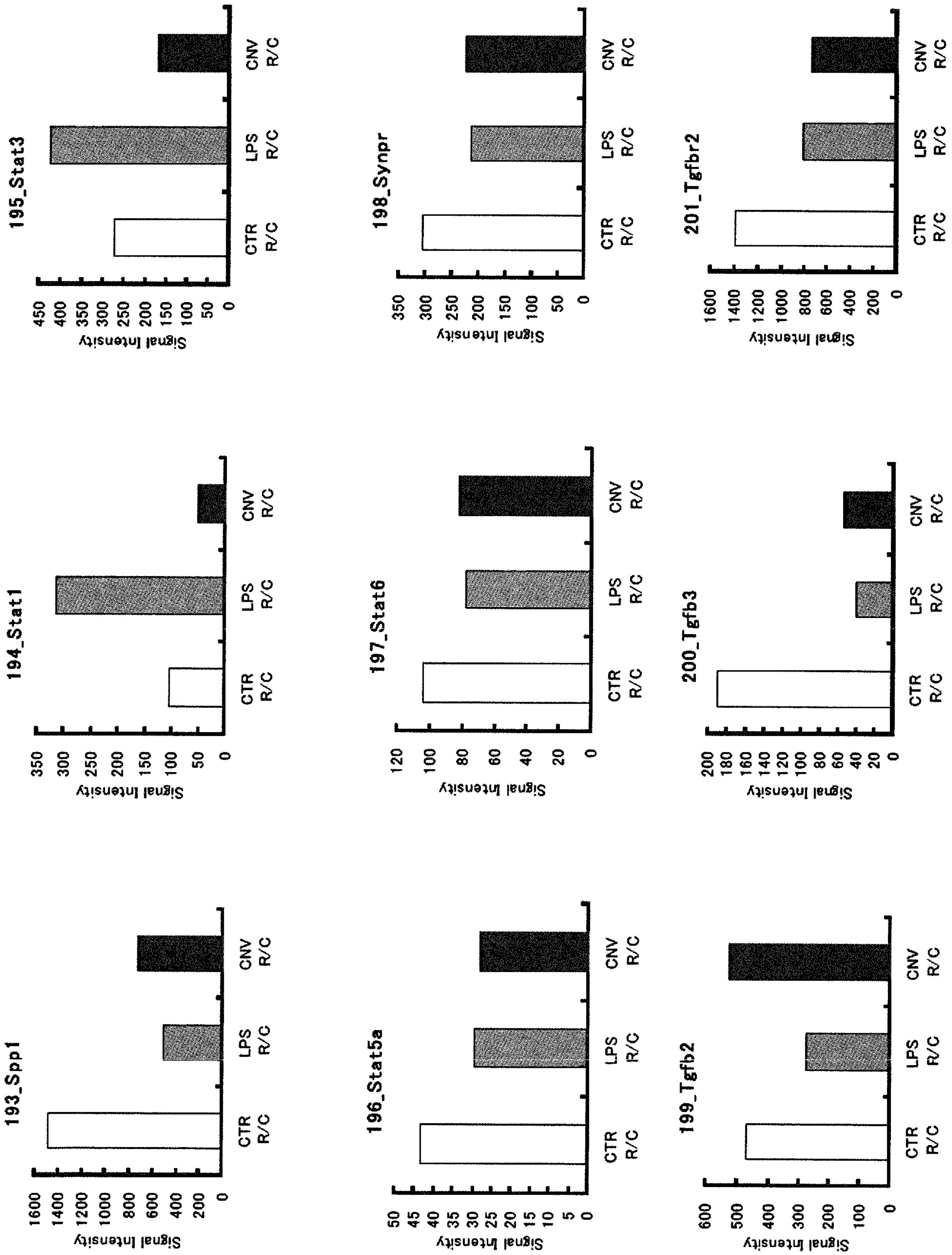
193_Spp1
194_Stat1
195_Stat3
196_Stat5a
197_Stat6
198_Synpr
199_Tgfb2
200_Tgfb3
201_Tgfbr2
Signal Intensity
CTR R/C
LPS R/C
CNV R/C

【Fig. 3L-2】

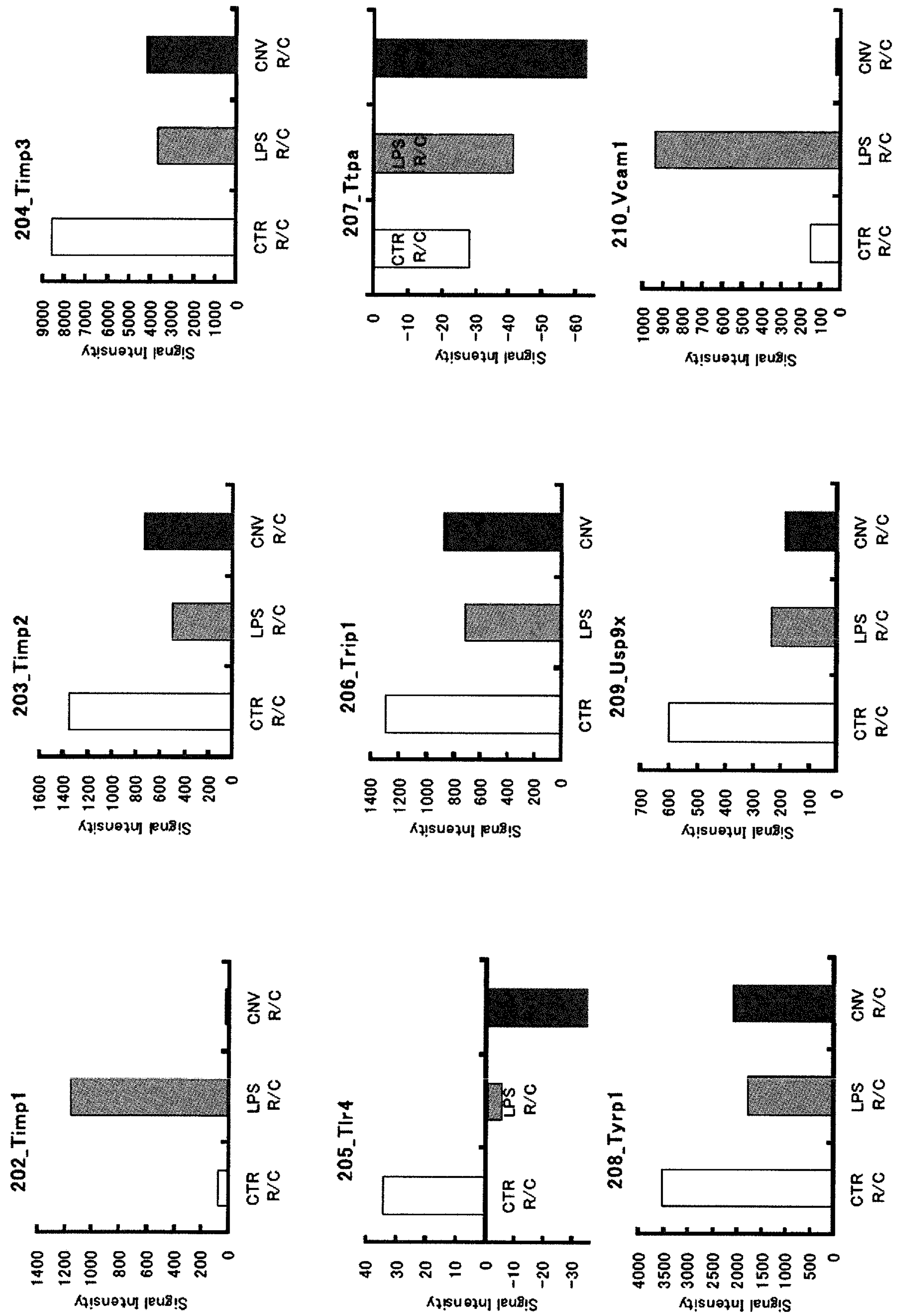
202_Timp1
203_Timp2
204_Timp3
205_Tlr4
206_Trip1
207_Ttpa
208_Tyrp1
209_Usp9x
210_Vcam1
Signal Intensity
CTR R/C
LPS R/C
CNV R/C
CTR
LPS
CNV

【Fig. 3M】

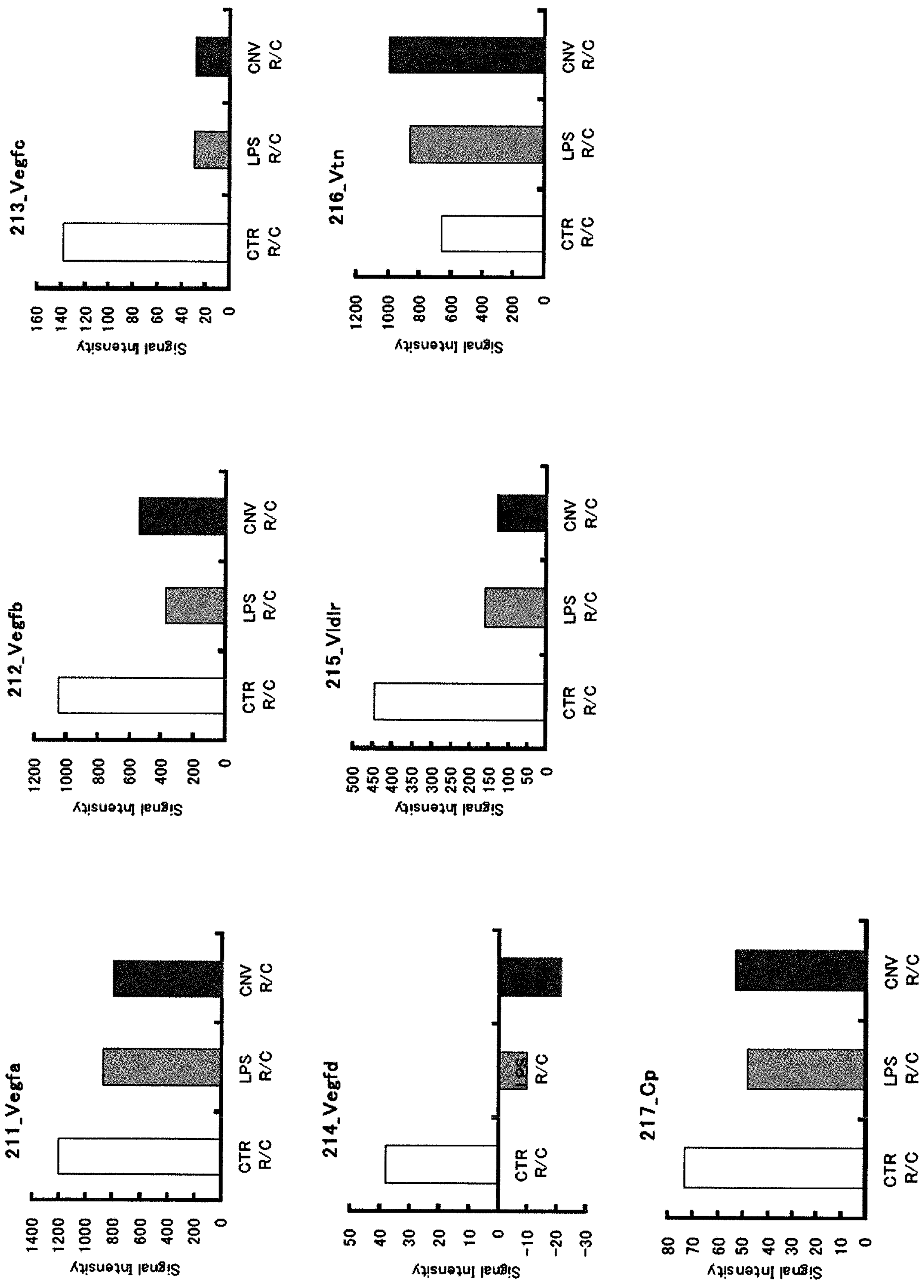
211_Vegfa
212_Vegfb
213_Vegfc
214_Vegfd
215_Vldlr
216_Vtn
217_Cp
Signal Intensity
CTR R/C
LPS R/C
CNV R/C

**INTERNATIONAL SEARCH REPORT**

International application No.
PCT/JP2014/060223

A. CLASSIFICATION OF SUBJECT MATTER
*C12N15/09*(2006.01)i, *C12Q1/68*(2006.01)i, *G01N33/15*(2006.01)i, *G01N33/50*(2006.01)i, *G01N33/53*(2006.01)i, *G01N37/00*(2006.01)i

According to International Patent Classification (IPC) or to both national classification and IPC

B. FIELDS SEARCHED

Minimum documentation searched (classification system followed by classification symbols)
C12N15/09, C12Q1/68, G01N33/15, G01N33/50, G01N33/53, G01N37/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)
CAplus/MEDLINE/BIOSIS(STN), JSTPlus/JMEDPlus/JST7580(JDreamIII), WPI(Thomson Innovation)

C. DOCUMENTS CONSIDERED TO BE RELEVANT

| Category* | Citation of document, with indication, where appropriate, of the relevant passages | Relevant to claim No. |
|---|---|---|
| X/Y | CHEN, L. et al., Light damage induced changes in mouse retinal gene expression, Experimental Eye Research, 2004.08, 79(2), pp. 239-247, Abstract, Introduction, Materials and Methods, Table 2 | 5-7/2,3-4 (Partial), 5-7 |
| X/Y | HADZIAHMETOVIC, M. et al., Microarray Analysis of Murine Retinal Light Damage Reveals Changes in Iron Regulatory, Complement, and Antioxidant Genes in the Neurosensory Retina and Isolated RPE, Investigative Ophthalmology & Visual Science, 2012.08.07, 53(9), pp. 5231-5241, Abstract, Introduction | 5-7/2,3-4 (Partial), 5-7 |

☒ Further documents are listed in the continuation of Box C.　　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 July, 2014 (07.07.14) | 15 July, 2014 (15.07.14) |
| Name and mailing address of the ISA/<br>Japanese Patent Office | Authorized officer |
| Facsimile No. | Telephone No. |

Form PCT/ISA/210 (second sheet) (July 2009)

**INTERNATIONAL SEARCH REPORT**

International application No. PCT/JP2014/060223

C (Continuation). DOCUMENTS CONSIDERED TO BE RELEVANT

| Category* | Citation of document, with indication, where appropriate, of the relevant passages | Relevant to claim No. |
|---|---|---|
| X/Y | YOSHIDA, S. et al., Microarray Analysis of Gene Expression in the Aging Human Retina, Investigative Ophthalmology & Visual Science, 2002.08, 43(8), pp. 2554-2560, Abstract, TABLE 2 | 5-7/2,3-4 (Partial), 5-7 |
| X/Y | JP 2003-504088 A (The University of Iowa Research Foundation), 04 February 2003 (04.02.2003), example 11 & US 2003/0149997 A1 & WO 2001/006262 A1 | 5-7/2,3-4 (Partial), 5-7 |
| Y | LEUNG, K. W. et al., Bacterial endotoxin activates retinal pigment epithelial cells and induces their degeneration through IL-6 and IL-8 autocrine signaling, Molecular Immunology, 2009.04, 46(7), pp. 1374-1386, ABSTRACT, Introduction | 2,3-4 (Partial), 5-7 |
| Y/A | JP 2010-000018 A (Sony Corp.), 07 January 2010 (07.01.2010), paragraph [0103] (Family: none) | 4(Partial)/ 2,3 (Partial), 5-7 |
| A | JP 2008-518610 A (Almac Diagnostics Ltd.), 05 June 2008 (05.06.2008), claims; paragraph [0170] & US 2006/0134663 A1 & WO 2006/048291 A2 | 2,3-4 (Partial), 5-7 |
| A | WO 2007/086490 A1 (Keio University), 02 August 2007 (02.08.2007), paragraphs [0001] to [0010] & US 2010/0034811 A1 & EP 1990060 A1 | 2,3-4 (Partial), 5-7 |

Form PCT/ISA/210 (continuation of second sheet) (July 2009)

**INTERNATIONAL SEARCH REPORT**

International application No.
PCT/JP2014/060223

**Box No. II Observations where certain claims were found unsearchable (Continuation of item 2 of first sheet)**

This international search report has not been established in respect of certain claims under Article 17(2)(a) for the following reasons:

1. [x] Claims Nos.: 1,3-4(a part thereof)
because they relate to subject matter not required to be searched by this Authority, namely:
(See extra sheet)

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying additional fees, this Authority did not invite payment of additional fees.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**
[ ] The additional search fees were accompanied by the applicant's protest and, where applicable, the payment of a protest fee.
[ ] The additional search fees were accompanied by the applicant's protest but the applicable protest fee was not paid within the time limit specified in the invitation.
[ ] No protest accompanied the payment of additional search fees.

Form PCT/ISA/210 (continuation of first sheet (2)) (July 2009)

**INTERNATIONAL SEARCH REPORT**

International application No.
PCT/JP2014/060223

Continuation of Box No.II-1 of continuation of first sheet(2)

Claim 1 and the parts of claims 3 and 4 which refer to claim 1 pertain to [methods for diagnosis of the human body], and thus relate to a subject matter on which this International Searching Authority is not required to carry out an international search under the provisions of PCT Article 17(2)(a)(i) and PCT Rule 39.1(iv).

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2007528371 A
- JP 2013080395 A
- JP 3510882 B
- JP 2004163211 A
- JP H11108928 A
- JP 2001133453 A


### Non-patent literature cited in the description


- Age-Related Macular Degeneration. *Japan Ophthalmological Society,* 06 June 2012, http://www.nichigan.or.jp/public/disease/momaku_karei.jsp
- Molecular Cloning, A Laboratory Manual. Cold Spring Harbor Press, 1989
- Current Protocols in Molecular Biology. John Wiley & Sons, 1987
- *Science,* 1995, vol. 270, 467-470
- *Science,* 1991, vol. 251, 767-773